# EUROPEAN PATENT APPLICATION

(11) **EP 3 900 789 A1**
(43) Date of publication of application: **27.10.2021**
(21) Application number: 20171212.2
(22) Date of filing: 23.04.2020
(51) Int. Cl.: A61P 35/00, A61K 45/00

(54) **PHARMACEUTICAL COMBINATION FOR THE TREATMENT OF MYELOPROLIFERATIVE NEOPLASMS**

(71) Applicant: Universitätsklinikum Jena, 07743 Jena (DE); Max-Planck-Gesellschaft zur Förderung der Wissenschaften e.V., 80539 München (DE)
(72) Inventor: HEIDEL, Florian, 07747 Jena (DE)
(74) Representative: Arth, Hans-Lothar

(57) **Abstract**

The present invention relates to the treatment of myeloproliferative neoplasms through targeted elimination of malignant clones and overcome of diseases persistence. The treatment is based on a combination of inhibitors of mRNA splicing and processing factors together with Jak2 inhibitors.

## Description

The present invention relates to the treatment of myeloproliferative neoplasms through targeted elimination of malignant clones and overcome of diseases persistence. The treatment is based on a combination of inhibitors of mRNA splicing and processing factors together with Jak2 inhibitors.

### Background of the invention

Myeloproliferative neoplasms (MPNs) are a group of diseases of the bone marrow in which excess cells are produced. They are related to, and may evolve into, myelodysplastic syndrome and acute myeloid leukemia, although the myeloproliferative diseases on the whole have a much better prognosis than these conditions. The increased numbers of blood cells may not cause any symptoms, but a number of medical problems or symptoms may occur. The risk of thrombosis is increased in some types of myeloproliferative neoplasms.

Myeloproliferative neoplasms are classified within the hematological neoplasms. There are main myeloproliferative diseases: BCR-ABL1-positive chronic myelogenous leukemia, chronic neutrophilic leukemia, polycythemia vera (PV) with predominant erythrocytosis, primary and idiopathic myelofibrosis (MF) with megakaryocyte expansion and progressive bone marrow fibrosis, essential thrombocythemia (ET) with pronounced thrombocytosis, chronic eosinophilic leukemia, and mastocytosis.

The classic BCR-ABL1 - negative myeloproliferative neoplasms include three different disorders - essential thrombocythemia (ET), polycythemia vera (PV), and primary myelofibrosis (PMF) - and are caused by constitutive activation of the cytokine receptor/JAK2 pathway due to acquired somatic mutations in three major genes.

The only curative therapy for myeloproliferative neoplasms is hematopoietic stem cell transplantation, but as myeloproliferative neoplasms most often affect the elderly, this option is restricted to a subset of patients with limited efficacy and significant toxicity. Thus, effective therapeutic options are critically needed based on new insight into the molecular processes driving myeloproliferative neoplasms.

JAK2V617F is the most prevalent mutation in myeloproliferative neoplasms associated with these three disorders (65-70%) and is present in 95% of polycythemia vera cases. This activating mutation mimics the effects of hematopoietic cytokines by inducing constitutive signaling via the STATs, PI3K, and ERK/MAPK pathways, causing myeloproliferative neoplasms. The identification of JAK2 mutations as disease-initiating in myeloproliferative neoplasms has led to new therapies for these diseases.

Activated Jak2 signalling represents a central feature of all myeloproliferative neoplasms, and Jak2 inhibitors are in clinical use for the treatment of myeloproliferative neoplasms.

The activating JAK2V617F is present in 95% of polycythemia vera (PV) and 50% - 60% of myelofibrosis (MF) and essential thrombocythemia (ET); also identified in myeloproliferative neoplasms are mutations in JAK2 exon 12. Mutations in the thrombopoietin receptor MPL (MPLW515L/K) and in the chaperone protein calreticulin (CALR) were identified and converge on activation of MPL - JAK2 signaling.

While Jak-inhibitors (Jak-i) reduce inflammatory activity and hyperproliferation of myeloid progenitors, Jak2-mutated clones that maintain the disease persist. Therefore, unexpectedly, Jak inhibitors in clinical use have minor effects on overall disease burden or evolution of persistent clones.

**It is the objective of the present invention to provide combinations and/or pharmaceutical compositions which can be used especially for the treatment of myeloproliferative neoplasms, and in particular of myeloproliferative neoplasms which are persistent to treatment with Jak inhibitors.**

**The objective of the present invention is solved by the teaching of the independent claims. Further advantageous features, aspects and details of the invention are evident from the dependent claims, the description, the figures, and the examples of the present application.**

### Brief description of the invention

The present invention relates to a combination for use in the treatment of a myeloproliferative neoplasm, wherein the combination comprises:
at least one inhibitor of a mRNA splicing and processing factor, and
at least one JAK inhibitor.

A preferred embodiment of the invention is directed to a combination for use in the treatment of a myeloproliferative neoplasm, wherein the combination comprises at least one inhibitor of a mRNA splicing and processing factor, and at least one JAK inhibitor, wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor.

Another preferred embodiment of the invention is directed to a combination for use in the treatment of a myeloproliferative neoplasm, wherein the combination comprises at least one inhibitor of a mRNA splicing and processing factor, and at least one JAK inhibitor, wherein the at least one inhibitor of a mRNA splicing and processing factor is a Ybx1 inhibitor.

A more preferred embodiment of the invention is directed to a combination for use in the treatment of a myeloproliferative neoplasm, wherein the combination comprises at least one inhibitor of a mRNA splicing and processing factor, and at least one JAK inhibitor, wherein the at least one inhibitor of a mRNA splicing and processing factor is selected from the group comprising a mRNA expression inhibitor, a protein expression inhibitor, a post-translational modification inhibitor, and a functional inhibitor.

A further preferred embodiment of the invention is directed to a combination for use in the treatment of a myeloproliferative neoplasm, wherein the combination comprises at least one inhibitor of a mRNA splicing and processing factor, and at least one JAK inhibitor, wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

Another embodiment of the invention provides a combination for use in the treatment of a myeloproliferative neoplasm, wherein the combination comprises at least one inhibitor of a mRNA splicing and processing factor, and at least one JAK inhibitor, wherein the myeloproliferative neoplasm is caused by and/or associated with a JAK2V617F mutation.

A further embodiment of the invention provides a combination for use in the treatment of a myeloproliferative neoplasm, wherein the combination comprises at least one inhibitor of a mRNA splicing and processing factor, and at least one JAK inhibitor, wherein the myeloproliferative neoplasm is persistent to JAK-inhibitor.

A preferred embodiment of the invention provides a combination for use in the treatment of a myeloproliferative neoplasm, wherein the combination comprises at least one inhibitor of a mRNA splicing and processing factor, and at least one JAK inhibitor, wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further preferred embodiment of the invention provides a combination comprising at least one inhibitor of a mRNA splicing and processing factor, and at least one JAK inhibitor.

In another aspect, the present invention provides a pharmaceutical composition comprising at least one inhibitor of a mRNA splicing and processing factor, and at least one JAK inhibitor, together with at least one pharmaceutically acceptable vehicle, excipient and/or diluent.

In a particular aspect, the present invention provides a pharmaceutical composition comprising at least one inhibitor of a mRNA splicing and processing factor, and at least one JAK inhibitor, together with at least one pharmaceutically acceptable vehicle, excipient and/or diluent, wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor.

In a preferred aspect, the present invention provides a pharmaceutical composition comprising at least one inhibitor of a mRNA splicing and processing factor, and at least one JAK inhibitor, together with at least one pharmaceutically acceptable vehicle, excipient and/or diluent, wherein the at least one inhibitor of a mRNA splicing and processing factor is a Ybx1 inhibitor.

In a further preferred aspect, the present invention provides a pharmaceutical composition comprising at least one inhibitor of a mRNA splicing and processing factor, and at least one JAK inhibitor, together with at least one pharmaceutically acceptable vehicle, excipient and/or diluent, wherein the at least one inhibitor of a mRNA splicing and processing factor is selected from the group comprising a mRNA expression inhibitor, a protein expression inhibitor, a post-translational modification inhibitor, and a functional inhibitor.

In a more preferred aspect, the present invention provides a pharmaceutical composition comprising at least one inhibitor of a mRNA splicing and processing factor, and at least one JAK inhibitor, together with at least one pharmaceutically acceptable vehicle, excipient and/or diluent, wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

In a still more preferred aspect, the present invention provides a pharmaceutical composition comprising at least one inhibitor of a mRNA splicing and processing factor, and at least one JAK inhibitor, together with at least one pharmaceutically acceptable vehicle, excipient and/or diluent,, for use in the treatment of a myeloproliferative neoplasm.

The present invention also provides a pharmaceutical composition comprising at least one inhibitor of a mRNA splicing and processing factor, and at least one JAK inhibitor, together with at least one pharmaceutically acceptable vehicle, excipient and/or diluent, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

### Detailed Description of the invention

Myeloproliferative neoplasms (MPNs) are a group of diseases of the bone marrow in which excess cells are produced. They are related to, and may evolve into, myelodysplastic syndrome and acute myeloid leukemia, although the myeloproliferative diseases on the whole have a much better prognosis than these conditions.
The identification of JAK2 mutations as disease-initiating in myeloproliferative neoplasms has led to new therapies for these diseases. However, Jak inhibitors in clinical use have minor effects on overall disease burden or evolution of persistent clones.

In order to find new therapeutic treatments for persistent myeloproliferative neoplasms, the inventors aimed to investigate the mechanism underlying evolution of Jak inhibitor persistence. In these experiments, the inventors identified, by in-depth phosphoproteome profiling (**Fig. 2****,** **3**), that proteins involved in the mRNA splicing and processing, (**Fig. 4**), such as Cpsf7, Cstf2, Hnrnpk, Hnrnpu, Pcbp1, Polr2a, Rbm8a, Sf3b1, Snrnp200, Srrm1, Srsf2, Srsf6, Srsf9, Srsf11, and Ybx1, are Jak2 targets, and then assessed their role in resistance to Jak-inhibitors by RNA interference (RNAi)-based functional validation (**Fig. 7a****,b**).

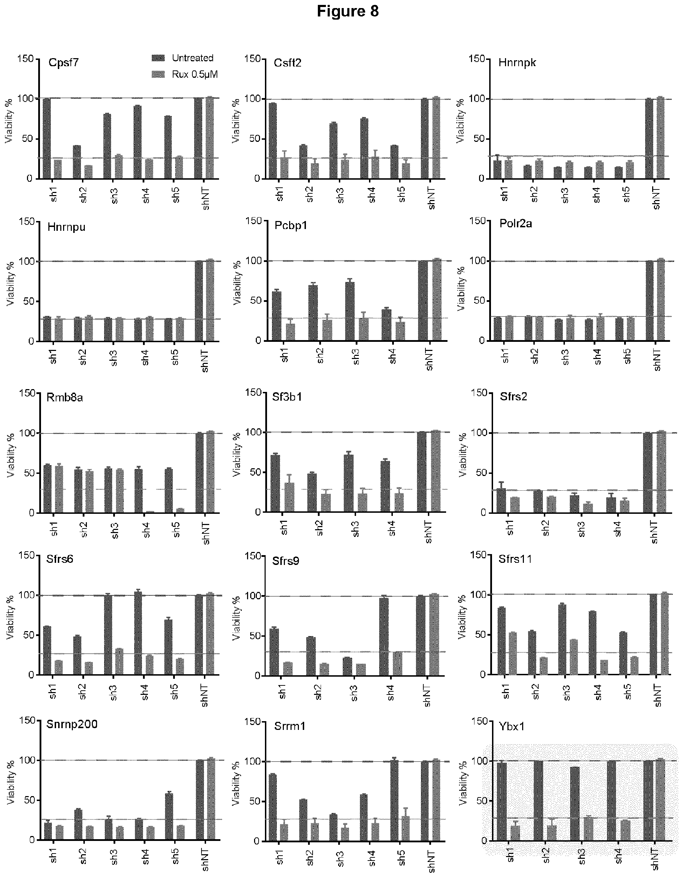

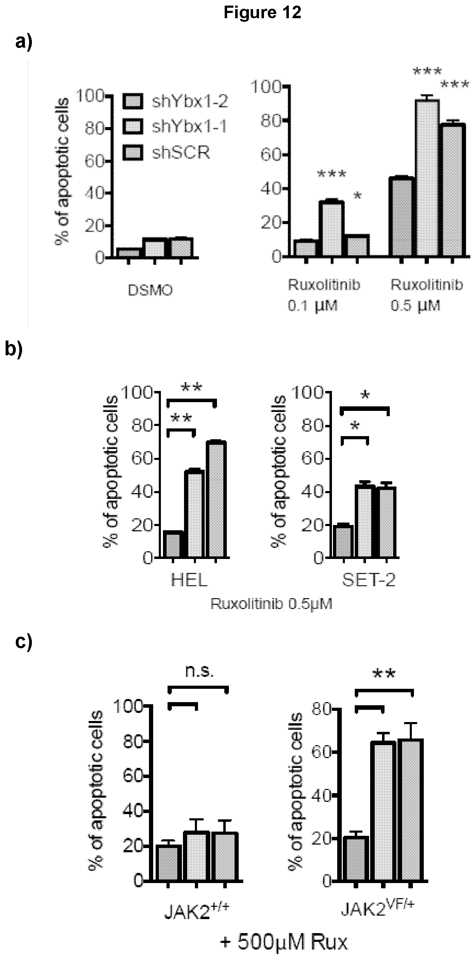

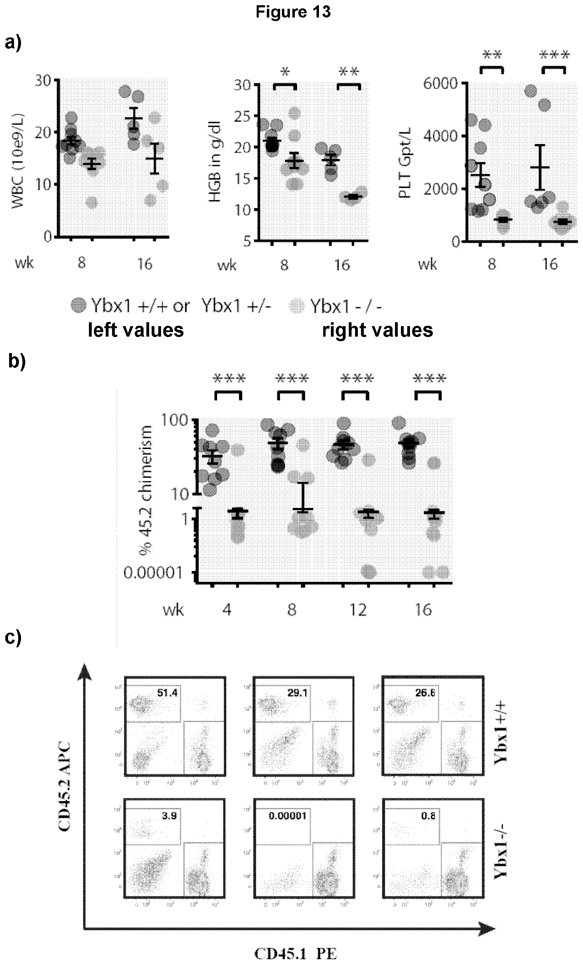

It was found that inactivation of mRNA splicing factors Cpsf7, Cstf2, Pcbp1, Srsf6, and in particular of Ybx1 sensitized JAK2V617F Jak-inhibitor persistent cells to proliferation inhibition (Fig. **8****).** Ybx1 inactivation by RNA interfering combined with pharmacological Jak-inhibition induced apoptosis in JAK2V617F murine and human cells (**Fig. 12a****,b**) and lead to *in vivo* regression of malignant clones (**Fig. 13a****-c**).

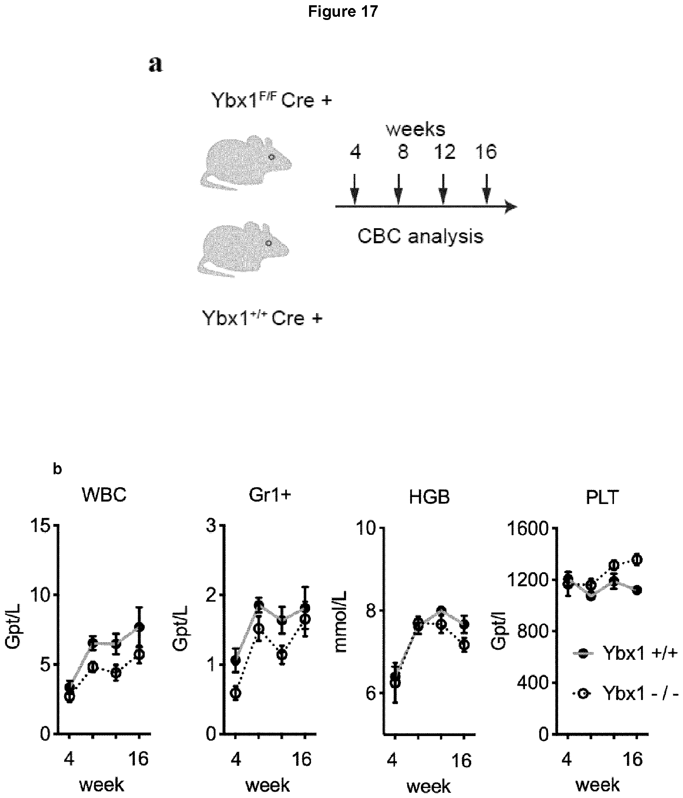

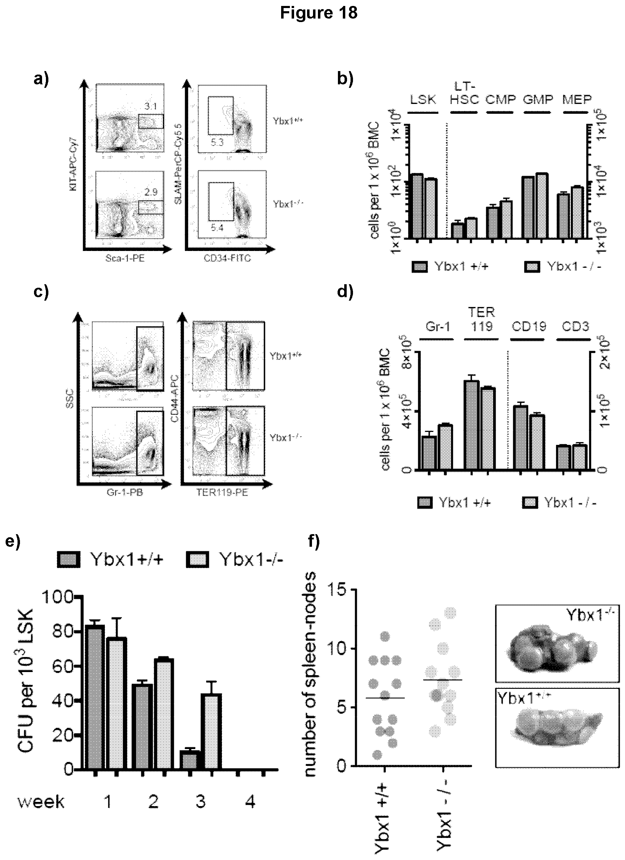

Importantly, it was showed that Ybx1 inactivation does not perturb steady-state haematopoiesis (**Fig. 17****,** **18**).

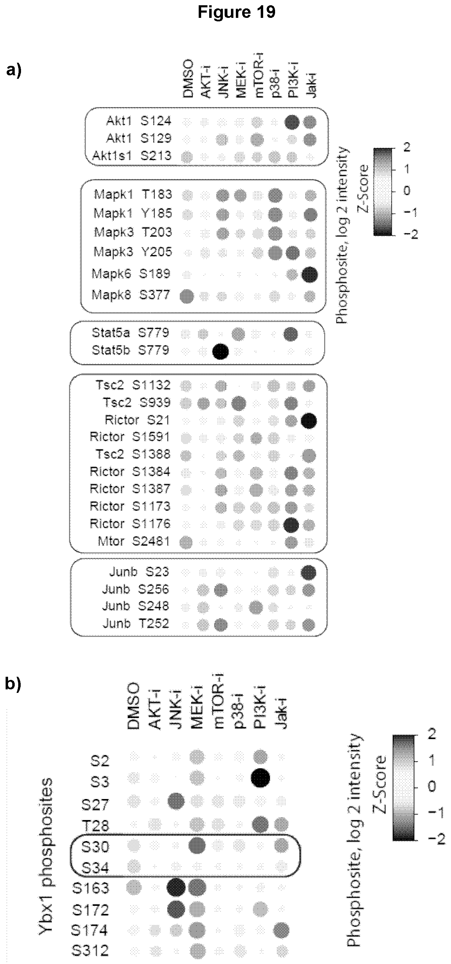

Moreover, it was shown that Jak2 inhibition stimulates Ybx1 phosphorylation at pS2, pS3, pS27, pS30, pS34, pS172, and pS174. Moreover, the MEK/ERK inhibitor PD0325901 was able to inhibit phosphorylation at pS30 and pS34, which are conserved across species (**Fig. 19b**).

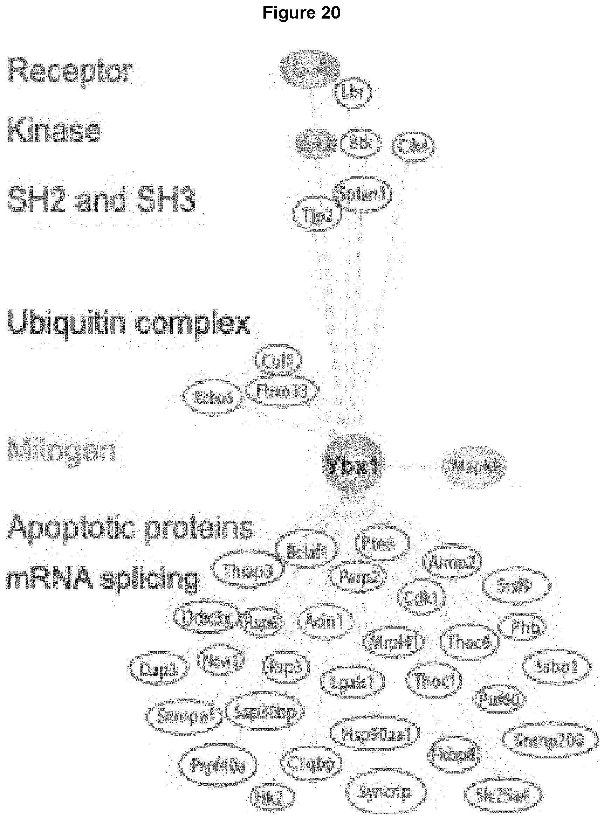

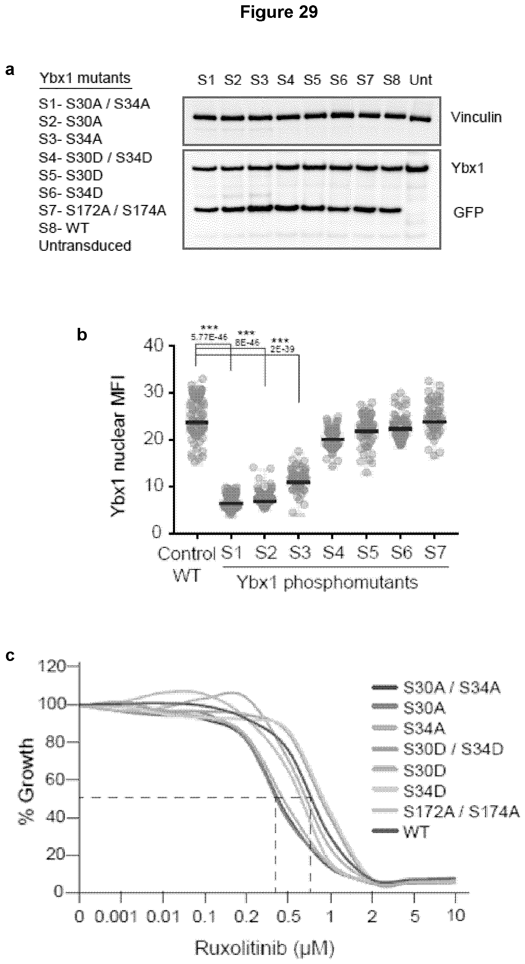

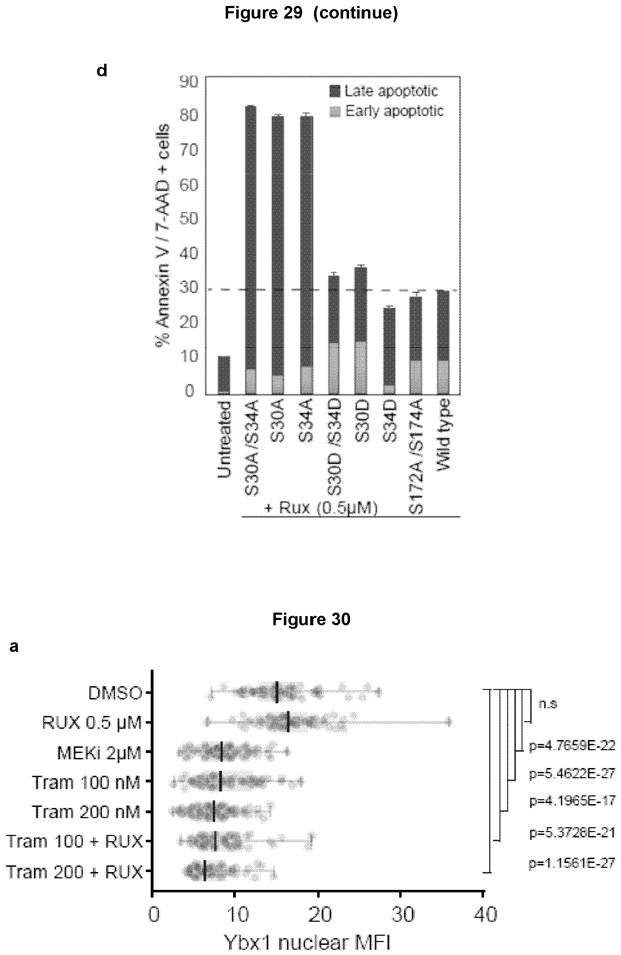

Further investigations showed that in Jak-2 mutated cells Ybx-1 interact with several bona fide members of mRNA splicing complexes (**Fig.20**) that are known to interact during spliceosome formation and activation. Moreover, they showed that Ybx1 phosphorylation depend on Jak2V617F-Mapk1 interaction, and that Ybx1-Mapk1 interaction is crucial for Ybx1 nuclear translocation and persists despite Jak-i treatment (**Fig. 30a****-d**). Indeed Ybx1 phosphorylation at pS30 and pS34 was shown to be essential for nuclear Ybx1 nuclear translocation (**Fig. 29b**). The therapeutic efficacy of inhibitors of Ybx1 phosphorylation at pS30 and pS34 combined with Jak2 inhibitors was confirmed as these modifications sensitized Jak-mutated cells to Jak-i mediated cell death (**Fig. 29c****, d**), recapitulating the phenotype of Ybx1 genetic inactivation.

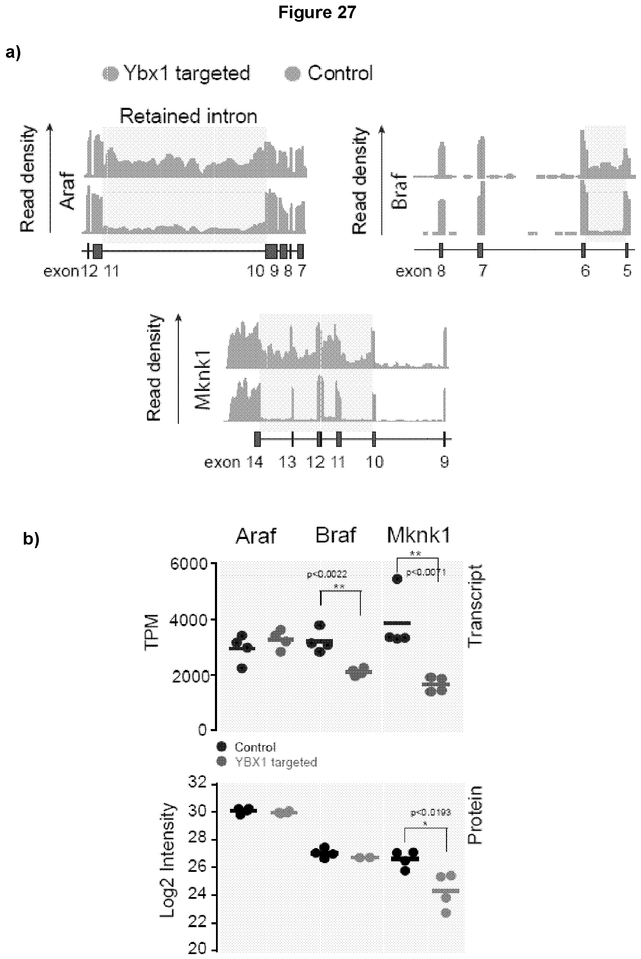

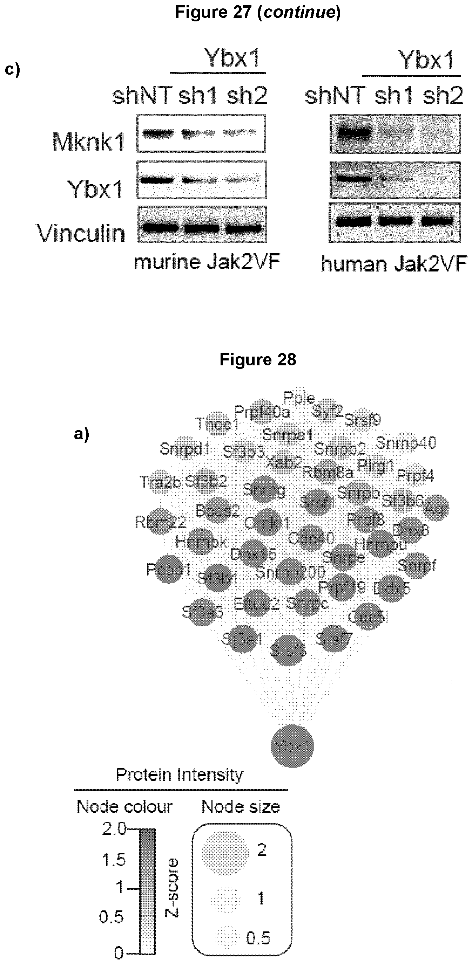

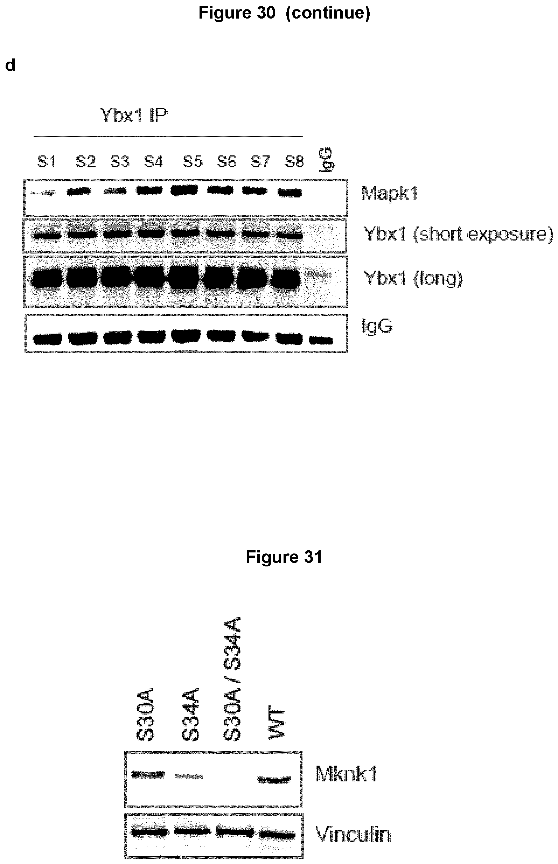

Importantly, RNA sequencing analysis of murine and human Jak2-mutated cells after YB1 inactivation, revealed that Ybx1 transcriptionally controls pathway related to inflammation, chemotaxis, and cytokine production, but also MAPK and ERK signalling and programmed cell death. Moreover, Ybx1 inactivation caused a significant decrease of ERK-signalling molecules Braf (mRNA) and Mknk1 in Jak2-mutated cells (mRNA and protein) in Jak-mutated cells (**Fig. 27b****, c**), due to intron retention. Importantly, Ybx1 phosphomutants (S30A, S34A, and S30A/S34A) resulted in reduction or abrogation of Mknk1 (**Fig. 31**), **confirming that pS30/pS34 phosphorylation of Ybx1 in Jak2V617F cells is a critical requirement for efficient mRNA splicing and transcriptional regulation of Mknk1 transcripts and a mechanistic link to the Ybx1 dependent disruption of ERK-signaling.**

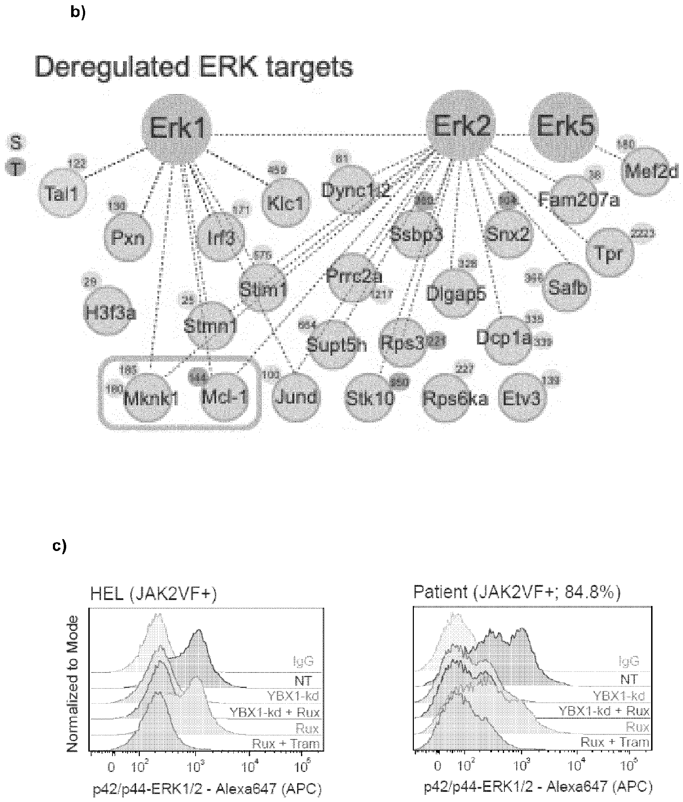

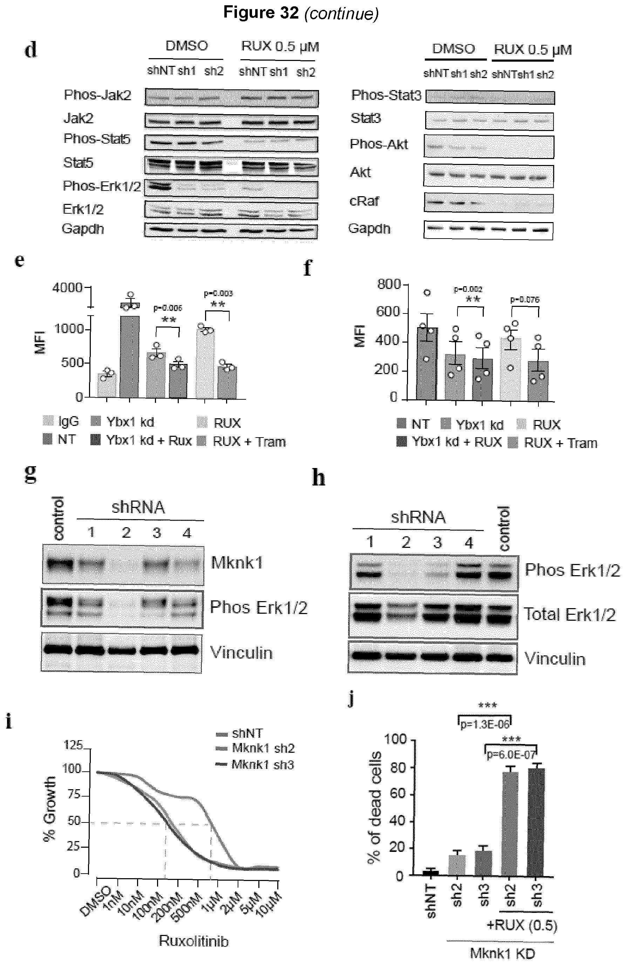

Moreover, it was shown that Ybx1 inactivation in JAK2V617F cells led to a considerable reduction of ERK phosphorylation in murine and human cells (**Fig. 22c** **and** **Fig. 32d****-f**) while leaving STAT signaling largely unaffected. Interestingly, concomitant pharmacological Jak inhibition resulted in abrogation of ERK-signaling. These findings indicate that Ybx1 is required for maintenance of ERK-signaling downstream of Jak2V617F.

Likewise, genetic inactivation of **Mknk1** by RNAi abrogated ERK signaling and sensitized the cells to Jak-i induced cell death (**Fig. 32g****-j**), which links Ybx1 dependent Mknk1 expression to maintenance of ERK signaling in Jak2V617F cells.

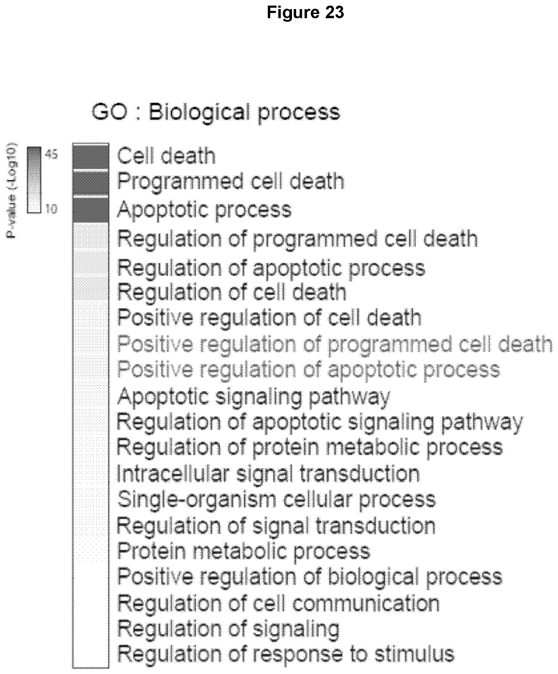

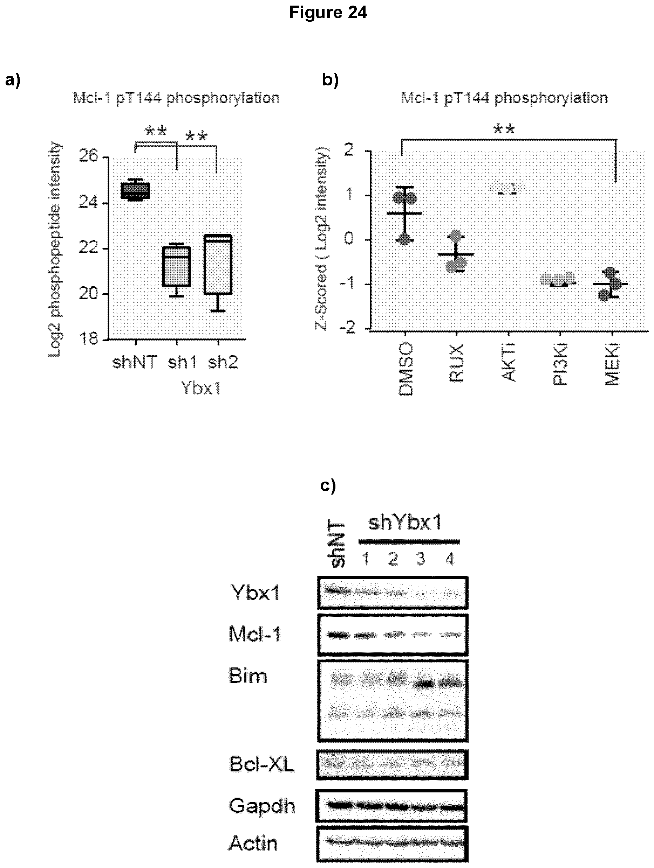

In the proteome of Ybx1 depleted Jak2V617F cells, affected cellular functions besides RNA splicing and processing included positive regulation of programmed cell death and apoptosis (**Fig. 23**). Accordingly, inactivation of Ybx1 in Jak2V617F cells resulted in reduction of pro-apoptotic Mcl-1 phosphorylation (**Fig. 24a****,b**), concomitant decrease of Mcl-1 protein abundance and induction of Bim (**Fig. 24c**).

These data show that a combination of an inhibitor of mRNA splicing and processing, such as Ybx1, together with a JAK2 inhibitor represents a novel therapeutic strategy to contrast disease persistence in myeloproliferative neoplasms. Ybx1 inhibitor may be directed to mRNA expression, post-translational modification such as phosphorylation or protein function.

Recent studies have identified mutations in components of the RNA spliceosome machinery, such as SRSF2, SF3B1, and U2AF1, in patients with myelofibrosis as well as a number of other myeloproliferative neoplasms. Thus, spliceosome inhibitors are being evaluated to treat myeloproliferative neoplasms characterized by mutations in spliceosome members.
Differently from this prior art, the inventive combination for use in the therapy of myeloproliferative neoplasms comprises a Jax inhibitor and an inhibitor of not mutated mRNA splicing and processing factor. Indeed, the inventive combination functions by targeting the MEK/ERK pathway, whose maintenance depend on the activity of Ybx1 and other mRNA splicing and processing factor. In other words, the present invention relates to a combination for use in the treatment of a myeloproliferative neoplasm, comprising one JAK inhibitor, and one inhibitor of a mRNA splicing and processing factor which is relevant for MEK signaling and/or ERK signaling.

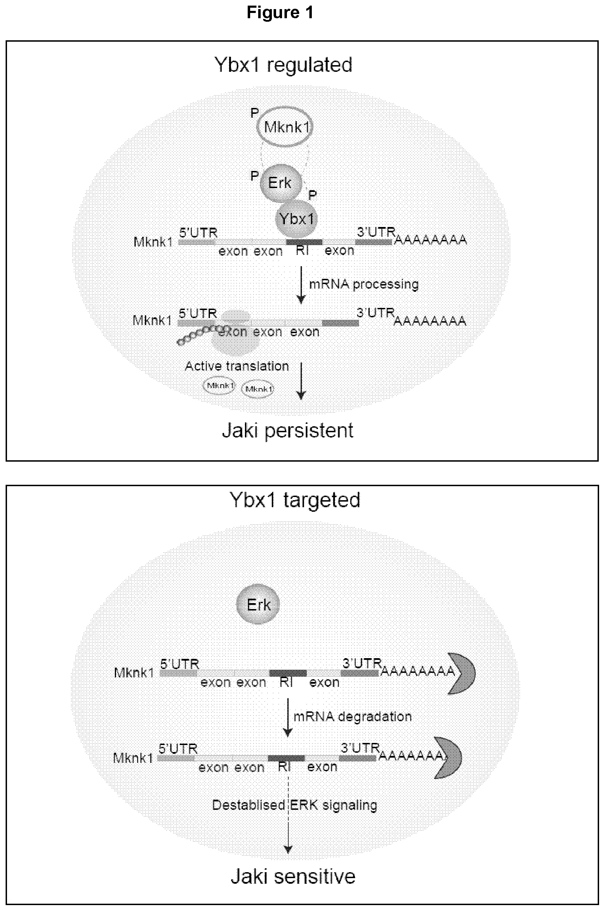

Moreover, the inventor identified post translational modification of Ybx1 by mutated Jak2 as a critical event required for mRNA splicing of Mknk1, which is an essential component of ERK-signaling and required for maintenance of Jak2-mutated cells during Jak-i treatment (**Fig. 1**).Thus Mknk1 represents a major regulatory factor of cancer cell persistence.

Thus, while recent studies have highlighted the role of ERK-signaling in Jak2-mutated cells, the inventor show here for the first time that mechanisms of differential protein phosphorylation results in splicing-dependent alterations of Mknk1-dependent ERK signaling. Confirmation of dual Jak2-ERK targeting using primary cells in vivo establishes a therapeutic principle for Jak-mutated neoplasms and a strong experimental rationale for incorporating the **Jak2-Ybx1 mediated regulation** of Mknk1-dependent ERK signaling into therapies directed at the elimination of persistent Jak-mutated cells.

Thus, the present invention is directed to a combination for use in the treatment of a myeloproliferative neoplasm, wherein the combination comprises:
at least one inhibitor of a mRNA splicing and processing factor, and
at least one JAK inhibitor.

According to one embodiment, the present invention is directed to a combination for use in the treatment of a myeloproliferative neoplasm, wherein the combination comprises:
at least one inhibitor of a mRNA splicing and processing factor, and
at least one JAK inhibitor, and
wherein the mRNA splicing factor is relevant for MEK signaling and/or ERK signaling.

According to one particular embodiment, the present invention is directed to a combination for use in the treatment of a myeloproliferative neoplasm, wherein the combination comprises:
at least one inhibitor of a mRNA splicing and processing factor, and
at least one JAK inhibitor, and
wherein the mRNA splicing and processing factor is relevant for Mknk1 activity.

According to a further embodiment, the present invention is directed to a combination for use in the treatment of a myeloproliferative neoplasm, wherein the combination comprises:
at least one inhibitor of a **not mutated** mRNA splicing and processing factor, and
at least one JAK inhibitor.

### Definitions

As used herein, the term "**cancer**" refers to any of those diseases characterized by an uncontrolled division and growth of abnormal cells in the body. Examples of cancers are **myeloproliferative neoplasms** which are a group of diseases of the bone marrow in which excess cells are produced.

The **MAPK signalling pathway**, here also referred to as the **MEK/ERK pathway**, engages 3 tiers of kinases, RAF, MEK1/2, and ERK1/2, and is typically activated by the GTPase RAS. MAP kinases undergo sequential phosphorylation events and promote cell proliferation, differentiation, and survival via a multitude of cytoplasmic and nuclear effectors, including transcription factors, cell cycle regulators, kinases, and phosphatases. MEK1/2 represent the intermediate kinases in the MAPK pathway, while ERK1/2 are distal in the cascade. MEK1/2 are dual-specificity kinases phosphorylating tyrosine and threonine residues of ERK1/2, which represent their exclusive substrates.

The core RNA splicing machinery, the spliceosome, removes introns and joins exons together to generate a mature mRNA molecule. This machinery assembles on the pre-mRNA molecule on specific sequences located at the exon-intron boundaries and that define the 3' and 5' splice sites (SSs) and the branch point site (BPS). The core human spliceosome, together with associated regulatory factors, comprise more than 300 proteins and five small nuclear RNAs (snRNAs) and catalyze both constitutive and regulated alternative splicing. The architecture of the spliceosome undergoes dynamic remodeling in preparation for, during, and after the splicing reaction. In addition to the core spliceosome, regulatory proteins are involved in modulating the splicing reaction, and act as splicing activators or repressors by binding to exonic or intronic enhancer or silencer elements.

The term "**mRNA processing**" defines, according to the definition of Gene Ontology on geneontology.org, any process involved in the conversion of a primary mRNA transcript into one or more mature mRNA(s) prior to translation into polypeptide. The term "mRNA processing" has the same meaning as "mRNA maturation". The "**mRNA processing**" term comprises 1332 proteins in human.

The term "**mRNA splicing**" represents a subgroup of the "mRNA processing", and is defined, according to the definition of Gene Ontology on geneontology.org, as the joining together of exons from one or more primary transcripts of messenger RNA (mRNA) and the excision of intron sequences, via a spliceosomal mechanism, so that mRNA consisting only of the joined exons is produced. The term "**mRNA splicing**" has the same meaning as "**nuclear mRNA splicing, via spliceosome**". The "**mRNA splicing**" comprises 918 proteins in human.

The term "**mRNA splicing and processing factors**" refers to proteins involved in mRNA splicing and processing. Preferred mRNA splicing and processing factors for the present invention are: Cpsf7, Cstf2, Hnrnpk, Hnrnpu, Pcbp1, Polr2a, Rbm8a, Sf3b1, Snrnp200, Srrm1, Srsf2, Srsf6, Srsf9, Srsf11, Ybx1.

**Cpsf7,** Cleavage And Polyadenylation Specific Factor 7, (Entrez Gene: 79869, UniProtKB: Q8N684, RNA Refseq: NM_172302) encodes a protein which is part of the "cleavage factor Im" (CFIm) that functions as an activator of the pre-mRNA 3'-end cleavage and polyadenylation processing required for the maturation of pre-mRNA into functional mRNAs. CFIm is composed of three different subunits of 25, 59, and 68 kDa, and it functions as a heterotetramer, with a dimer of the 25 kDa subunit binding to two of the 59 or 68 kDa subunits. The protein encoded by Cpsf7 represents the 59 kDa subunit, which can interact with the splicing factor U2 snRNP Auxiliary Factor (U2AF) 65 to link the splicing and polyadenylation complexes.

**Cstf2,** Cleavage Stimulation Factor Subunit 2, (Entrez Gene: 1478, UniProtKB: P33240, RNA Refseq: NM_133196), encodes a nuclear protein with an RRM (RNA recognition motif) domain. The protein is a member of the cleavage stimulation factor (CSTF) complex that is involved in the 3' end cleavage and polyadenylation of pre-mRNAs. Specifically, this protein binds GU-rich elements within the 3'-untranslated region of mRNAs.

**Hnrnpk**, Heterogeneous Nuclear Ribonucleoprotein K, (Entrez Gene: 3190, UniProtKB: P61978, RNA Refseq: NM_025279), belongs to the subfamily of ubiquitously expressed heterogeneous nuclear ribonucleoproteins (hnRNPs). The hnRNPs are RNA binding proteins and they complex with heterogeneous nuclear RNA (hnRNA). These proteins are associated with pre-mRNAs in the nucleus and appear to influence pre-mRNA processing and other aspects of mRNA metabolism and transport. While all of the hnRNPs are present in the nucleus, some seem to shuttle between the nucleus and the cytoplasm. The hnRNP proteins have distinct nucleic acid binding properties. The protein encoded by this gene is located in the nucleoplasm and has three repeats of KH domains that binds to RNAs. It is distinct among other hnRNP proteins in its binding preference; it binds tenaciously to poly(C). This protein is also thought to have a role during cell cycle progession. Several alternatively spliced transcript variants have been described for this gene, however, not all of them are fully characterized.

**Hnrnpu**, Heterogeneous Nuclear Ribonucleoprotein U, (Entrez Gene: 3192, UniProtKB: Q00839, RNA Refseq: NM_016805), encodes a member of a family of proteins that bind nucleic acids and function in the formation of ribonucleoprotein complexes in the nucleus with heterogeneous nuclear RNA (hnRNA). The encoded protein has affinity for both RNA and DNA, and binds scaffold-attached region (SAR) DNA. Mutations in this gene have been associated with epileptic encephalopathy, early infantile.

**Pcbp1,** Poly(RC) Binding Protein 1, (Entrez Gene: 5093, UniProtKB: Q153659, RNA Refseq: NM_011865) encodes a protein likely to be multifunctional. It along with PCBP-2 and hnRNPK corresponds to the major cellular poly(rC)-binding protein. It contains three K-homologous (KH) domains which may be involved in RNA binding. This encoded protein together with PCBP-2 also functions as translational coactivators of poliovirus RNA via a sequence-specific interaction with stem-loop IV of the IRES and promote poliovirus RNA replication by binding to its 5'-terminal cloverleaf structure. It has also been implicated in translational control of the 15-lipoxygenase mRNA, human Papillomavirus type 16 L2 mRNA, and hepatitis A virus RNA. The encoded protein is also suggested to play a part in formation of a sequence-specific alpha-globin mRNP complex which is associated with alpha-globin mRNA stability.

**Polr2a**, RNA Polymerase II Subunit A, (Entrez Gene: 5430, UniProtKB: P24928, RNA Refseq: NM_009089) is the largest subunit of RNA polymerase II, the polymerase responsible for synthesizing messenger RNA in eukaryotes. The product of this gene contains a carboxy terminal domain composed of heptapeptide repeats that are essential for polymerase activity. These repeats contain serine and threonine residues that are phosphorylated in actively transcribing RNA polymerase. In addition, this subunit, in combination with several other polymerase subunits, forms the DNA binding domain of the polymerase, a groove in which the DNA template is transcribed into RNA.

**Rbm8a**, RNA Binding Motif Protein 8A (Entrez Gene: 9939, UniProtKB: Q9Y5S9, RNA Refseq: NM_001039518). has a conserved RNA-binding motif. The protein is found predominantly in the nucleus, although it is also present in the cytoplasm. It is preferentially associated with mRNAs produced by splicing, including both nuclear mRNAs and newly exported cytoplasmic mRNAs. It is thought that the protein remains associated with spliced mRNAs as a tag to indicate where introns had been present, thus coupling pre- and post-mRNA splicing events. Previously, it was thought that two genes encode this protein, RBM8A and RBM8B; it is now thought that the RBM8B locus is a pseudogene.

**Sf3b1**, Splicing Factor 3b Subunit 1, (Entrez Gene: 23451, UniProtKB: O75533, RNA Refseq: NM_031179), is the subunit 1 of the splicing factor 3b protein complex. Splicing factor 3b, together with splicing factor 3a and a 12S RNA unit, forms the U2 small nuclear ribonucleoproteins complex (U2 snRNP). The splicing factor 3b/3a complex binds pre-mRNA upstream of the intron's branch site in a sequence independent manner and may anchor the U2 snRNP to the pre-mRNA. Splicing factor 3b is also a component of the minor U12-type spliceosome. Alternative splicing results in multiple transcript variants encoding different isoforms.

**Snrnp200**, Small Nuclear Ribonucleoprotein U5 Subunit 200, (Entrez Gene: 23020, UniProtKB: O75643, RNA Refseq: NM_177214). Pre-mRNA splicing is catalyzed by the spliceosome, a complex of specialized RNA and protein subunits that removes introns from a transcribed pre-mRNA segment. The spliceosome consists of small nuclear RNA proteins (snRNPs) U1, U2, U4, U5 and U6, together with approximately 80 conserved proteins. U5 snRNP contains nine specific proteins. This gene encodes one of the U5 snRNP-specific proteins. This protein belongs to the DEXH-box family of putative RNA helicases. It is a core component of U4/U6-U5 snRNPs and appears to catalyze an ATP-dependent unwinding of U4/U6 RNA duplices. Mutations in this gene cause autosomal-dominant retinitis pigmentosa. Alternatively spliced transcript variants encoding different isoforms have been found, but the full-length nature of these variants has not been determined.

**Srrm1**, Serine And Arginine Repetitive Matrix 1, (Entrez Gene: 10250, UniProtKB: Q8IYB3, RNA Refseq: NM_001130477), is part of pre- and post-splicing multiprotein mRNP complexes, involved in numerous pre-mRNA processing events. Promotes constitutive and exonic splicing enhancer (ESE)-dependent splicing activation by bridging together sequence-specific (SR family proteins, SFRS4, SFRS5 and TRA2B/SFRS10) and basal snRNP (SNRP70 and SNRPA1) factors of the spliceosome. Stimulates mRNA 3'-end cleavage independently of the formation of an exon junction complex. Binds both pre-mRNA and spliced mRNA 20-25 nt upstream of exon-exon junctions. Binds RNA and DNA with low sequence specificity and has similar preference for either double- or single-stranded nucleic acid substrates.

**Srsf2**, Serine And Arginine Rich Splicing Factor 2, (Entrez Gene: 6427, UniProtKB: Q01130, RNA Refseq: NM_011358), protein is a member of the serine/arginine (SR)-rich family of pre-mRNA splicing factors, which constitute part of the spliceosome. Each of these factors contains an RNA recognition motif (RRM) for binding RNA and an RS domain for binding other proteins. The RS domain is rich in serine and arginine residues and facilitates interaction between different SR splicing factors. In addition to being critical for mRNA splicing, the SR proteins have also been shown to be involved in mRNA export from the nucleus and in translation. Two transcript variants encoding the same protein and one noncoding transcript variant have been found for this gene.

**Srsf6**, Serine And Arginine Rich Splicing Factor 6, (Entrez Gene: 6431, UniProtKB: Q13247, RNA Refseq: NM_026499) is involved in mRNA splicing and may play a role in the determination of alternative splicing. The encoded nuclear protein belongs to the splicing factor serine/arginine (SR)-family and has been shown to bind with and modulate another member of the family, SFRS12. Alternative splicing results in multiple transcript variants.

**Srsf9**, Serine And Arginine Rich Splicing Factor 9, (Entrez Gene: 8683, UniProtKB: Q13242, RNA Refseq: NM_025573) protein is a member of the serine/arginine (SR)-rich family of pre-mRNA splicing factors, which constitute part of the spliceosome. Each of these factors contains an RNA recognition motif (RRM) for binding RNA and an RS domain for binding other proteins. The RS domain is rich in serine and arginine residues and facilitates interaction between different SR splicing factors. In addition to being critical for mRNA splicing, the SR proteins have also been shown to be involved in mRNA export from the nucleus and in translation.

**Srsf11**, Serine And Arginine Rich Splicing Factor 11, (Entrez Gene: 9295, UniProtKB: Q05519, RNA Refseq: NM_026989)is a 54-kD nuclear protein that contains an arginine/serine-rich region similar to segments found in pre-mRNA splicing factors. Although the function of this protein is not yet known, structure and immunolocalization data suggest that it may play a role in pre-mRNA processing. Alternative splicing results in multiple transcript variants encoding different proteins.

**Ybx1**, Y-Box Binding Protein 1, (Entrez Gene: 4904, UniProtKB: P67809, RNA Refseq: NM_011732) protein functions as both a DNA and RNA binding protein and has been implicated in numerous cellular processes including regulation of transcription and translation, pre-mRNA splicing, DNA reparation and mRNA packaging. This protein is also a component of messenger ribonucleoprotein (mRNP) complexes and may have a role in microRNA processing. This protein can be secreted through non-classical pathways and functions as an extracellular mitogen. Aberrant expression of the gene is associated with cancer proliferation in numerous tissues. This gene may be a prognostic marker for poor outcome and drug resistance in certain cancers. Alternate splicing results in multiple transcript variants.

Y-box binding protein-1 (Ybox1, YBX1, Ybx1) is a factor involved in mRNA processing and a multifunctional oncoprotein containing an evolutionarily conserved cold shock domain, dysregulating a wide range of genes involved in cell proliferation and survival, drug resistance, and chromatin destabilization by cancer. YBX1 is phosphorylated by kinases, including AKT, p70S6K, and p90RSK, and translocates into the nucleus to promote the transcription of resistance-and malignancy-related genes. Phosphorylated YBX1, therefore, plays a crucial role as a potent transcription factor in cancer. YBX1 is involved in tumor drug resistance. YBX1 dysregulates drug resistance-related genes, including ABCB1, MVP/LRP, PCNA, MYC, TOP2A, CD44, CD49f, p53, BCL2, and androgen receptor (AR), conferring resistance to cancer cells against a wide range of anticancer therapeutic agents.
Overall, YBX1 enhances the expression of genes involved in cell proliferation, cell cycle, survival, and drug resistance and facilitates malignant progression as well as acquired resistance to anticancer chemotherapeutics by cancer cells (Michihiko Kuwano, et al., Cancer Science, 2019, 110, pp.1536 -1543).

Other known mRNA splicing and processing factors are: Cpsf7, Cstf2, Hnrnpk, Hnrnpu, Pcbp1, Polr2a, Rbm8a, Sf3b1, Snrnp200, Srrm1, Srsf2, Srsf6, Srsf9, Srsf11.

As used herein, the term "**in combination**" refers to the use of more than one therapeutic agent (e.g., a JAK2 inhibitor, a mRNA splicing and processing factor inhibitor). The use of the term "**in combination**" does not restrict the order in which said therapeutic agents are administered to a subject with a disease or disorder, e.g., a myeloproliferative disorder.

As used herein, "**antagonist**" or "**inhibitor**" refers to a compound or combination of compounds that can reduce, minimize, suppress, block, or eliminate expression or function of a target molecule. Thus, an "**inhibitor of a mRNA splicing and processing factor**" refers to a compound or combination of compounds that can reduce, minimize, suppress, block, or eliminate expression or function of **a mRNA splicing and processing factor.** Preferred mRNA splicing and processing factor are Cpsf7, Cstf2, Hnrnpk, Hnrnpu, Pcbp1, Polr2a, Rbm8a, Sf3b1, Snrnp200, Srrm1, Srsf2, Srsf6, Srsf9, Srsf11, Ybx1.

An "**inhibitor of a mRNA splicing and processing factor**" is selected from a mRNA expression inhibitor, a protein expression inhibitor, a post-translational modification inhibitor, and a functional inhibitor.

mRNA expression inhibitors of **a mRNA splicing and processing factor are selected from the group comprising shRNA, antisense oligonucleotides, small interfering RNA (siRNA), and decoy oligonucleotides.**

Post-translational modification inhibitor are for example inhibitor of protein phosphorylation. The inventors found that MEK/ERK-inhibitors are able to inhibit Ybx1 phosphorylation. Thus, a post-translational modification or phosphorylation inhibitor for Ybx1 is selected from cobimetinib, CI-1040, PD0325901, Binimetinib (MEK162), selumetinib, Trametinib (GSK1120212).

Functional inhibitors are molecule able to block or antagonize an mRNA splicing and processing factor so that this is not able to exert its function. Functional inhibitors may be pharmaceutical compounds, small molecule compounds, or antibodies specifically binding to the active phosphorylated form of a mRNA splicing and processing factor. For example, functional inhibitor of Ybx1 are antibodies specifically binding Ybx1 phosphorylated at Ser-30 (pS30-Ybx1), or specifically binding Ybx1 phosphorylated at Ser-34 (pS34-Ybx1). Further **mRNA splicing and processing factor inhibitors** are E7107, blocking spliceosome assembly by preventing tight binding of U2 snRNP to pre-mRNA, and H3B-8800, acting as a modulator of the SF3b complex, compounds targeting Sf3b1such as sudemycin, spliceostatin, FR901464, pladienolide, herboxidine, and derivatives or analogs of each of these compounds.

Thus, more in particular, an "**inhibitor of a mRNA splicing and processing factor**" is selected from siRNA (small interfering RNA), shRNA (short hairpin RNA), miRNA (microRNA), antisense oligonucleotide, pharmaceutical compound, small molecule compound, antibody, polypeptide.

A preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor.

A more preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor.

A still more preferred embodiment of the invention is a combination for use in the treatment of a myeloproliferative neoplasm, wherein the combination comprises:
at least one inhibitor of a mRNA splicing and processing factor, and
at least one JAK inhibitor, and,
wherein the at least one inhibitor of a mRNA splicing and processing factor is selected from the group comprising a mRNA expression inhibitor, a protein expression inhibitor, a post-translational modification inhibitor, and a functional inhibitor.

A particularly preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor.

Myeloproliferative neoplasms include polycythemia vera, (PCV) primary myelofibrosis (PMF), idiopathic myelofibrosis (IMF), essential thrombocythemia (ET), chronic myeloid leukemia (CML), acute myeloid leukemia (AML), chronic eosinophilic leukemia (CEL), hypereosinophilic syndrome, chronic myelomonocytic leukemia (CMML), atypical chronic myelogenous leukemia, chronic neutrophilic leukemia, systemic mastocytosis (SM), juvenile myelomonocytic leukemia, myeloma, post-polycythemia vera myelofibrosis or post-essential thrombocythemia myelofibrosis.

Thus, a further embodiment of the present invention is directed to a combination for use in the treatment of a myeloproliferative neoplasm, wherein the combination comprises:
at least one inhibitor of a mRNA splicing and processing factor, and
at least one JAK inhibitor.
wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

A further preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and,
wherein the at least one inhibitor of a mRNA splicing and processing factor is selected from the group comprising a mRNA expression inhibitor, a protein expression inhibitor, a post-translational modification inhibitor, and a functional inhibitor, and
wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

A further particularly preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor,
wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

JAK2V617F is the most prevalent mutation causing or being associated with myeloproliferative neoplasms. Thus, in some embodiments of the invention the myeloproliferative neoplasm is caused by and/or associated with a JAK2V617F mutation

Thus, a further embodiment of the present invention is directed to a combination for use in the treatment of a myeloproliferative neoplasm, wherein the combination comprises:
at least one inhibitor of a mRNA splicing and processing factor, and
at least one JAK inhibitor, and
wherein the myeloproliferative neoplasm is caused by and/or associated with a JAK2V617F mutation.

A further preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the myeloproliferative neoplasm is caused by and/or associated with a JAK2V617F mutation.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the myeloproliferative neoplasm is caused by and/or associated with a JAK2V617F mutation.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and,
wherein the at least one inhibitor of a mRNA splicing and processing factor is selected from the group comprising a mRNA expression inhibitor, a protein expression inhibitor, a post-translational modification inhibitor, and a functional inhibitor, and
wherein the myeloproliferative neoplasm is caused by and/or associated with a JAK2V617F mutation.

A further particularly preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, and wherein the myeloproliferative neoplasm is caused by and/or associated with a JAK2V617F mutation.

The inventors could show that inactivation of Ybx1, a well-known mRNA splicing and processing factor, in combination with a JAK inhibitor is able to render JAK2V617F mutant cells sensistive to the pro-apoptotic action of the JAK inhibitor. Thus, the combination for use disclosed herein can be particularly useful for myeloproliferative neoplasms persistent to JAK-inhibitor.

Therefore, a further embodiment of the present invention is directed to a combination for use in the treatment of a myeloproliferative neoplasm, wherein the combination comprises:
at least one inhibitor of a mRNA splicing and processing factor, and
at least one JAK inhibitor, and
wherein the myeloproliferative neoplasm is persistent to JAK-inhibitor.

A further particular embodiment of the present invention is directed to a combination for use in the treatment of a myeloproliferative neoplasm, wherein the combination comprises:
at least one inhibitor of a mRNA splicing and processing factor, and
at least one JAK inhibitor, and
wherein the myeloproliferative neoplasm is caused by and/or associated with a JAK2V617F mutation, and
wherein the myeloproliferative neoplasm is persistent to JAK-inhibitor.

A particularly preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the myeloproliferative neoplasm is persistent to JAK-inhibitor.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the myeloproliferative neoplasm is persistent to JAK-inhibitor.

A still more preferred embodiment of the invention is a combination for use in the treatment of a myeloproliferative neoplasm, wherein the combination comprises:
at least one inhibitor of a mRNA splicing and processing factor, and
at least one JAK inhibitor, and,
wherein the at least one inhibitor of a mRNA splicing and processing factor is selected from the group comprising a mRNA expression inhibitor, a protein expression inhibitor, a post-translational modification inhibitor, and a functional inhibitor, and
wherein the myeloproliferative neoplasm is persistent to JAK-inhibitor.

A further particularly preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, and
wherein the myeloproliferative neoplasm is persistent to JAK-inhibitor.

A "**JAK inhibitor**," as used herein, includes any compound that disrupts JAK production and or the JAK/STAT signaling pathway. Examples of JAK inhibitors include, but are not limited to, ruxolitinib (NCB 18424), pacritinib, NS-018, CEP-33779, NVP-BVB808, TG101209, fedratinib (TG101348), momelotinib (CYT387), baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

Thus, for the combination for use disclosed herein, JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A particular embodiment of the present invention is directed to a combination for use in the treatment of a myeloproliferative neoplasm, wherein the combination comprises:
at least one inhibitor of a mRNA splicing and processing factor, and
at least one JAK inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A more particular embodiment of the present invention is directed to a combination for use in the treatment of a myeloproliferative neoplasm, wherein the combination comprises:
at least one inhibitor of a mRNA splicing and processing factor, and
at least one JAK inhibitor, and
wherein the myeloproliferative neoplasm is persistent to JAK-inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

Thus, a further embodiment of the present invention is directed to a combination for use in the treatment of a myeloproliferative neoplasm, wherein the combination comprises:
at least one inhibitor of a mRNA splicing and processing factor, and
at least one JAK inhibitor, and wherein the myeloproliferative neoplasm is caused by and/or associated with a JAK2V617F mutation, and
wherein the myeloproliferative neoplasm is persistent to JAK-inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further more preferred embodiment of the invention is a combination for use in the treatment of a myeloproliferative neoplasm, wherein the combination comprises:
at least one inhibitor of a mRNA splicing and processing factor, and
at least one JAK inhibitor, and,
wherein the at least one inhibitor of a mRNA splicing and processing factor is selected from the group comprising a mRNA expression inhibitor, a protein expression inhibitor, a post-translational modification inhibitor, and a functional inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further particularly preferred embodiment of the invention is a combination for use in the treatment of a myeloproliferative neoplasm, wherein the combination comprises:
at least one inhibitor of a mRNA splicing and processing factor, and
at least one JAK inhibitor, and
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

Finally, a further preferred embodiment of the invention provides a combination comprising at least one inhibitor of a mRNA splicing and processing factor, and at least one JAK inhibitor.

### PHARMACEUTICAL COMPOSITIONS

The disclosure also provides pharmaceutical compositions comprising one or more of the disclosed inhibitors in association with a pharmaceutically acceptable carrier.

An embodiment of the present invention is a pharmaceutical composition comprising at least one inhibitor of a mRNA splicing and processing factor, and at least one JAK inhibitor, together with at least one pharmaceutically acceptable vehicle, excipient and/or diluent.

A particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a mRNA expression inhibitor, a protein expression inhibitor, a post-translational modification inhibitor, and a functional inhibitor.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A further more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a mRNA expression inhibitor, a protein expression inhibitor, a post-translational modification inhibitor, and a functional inhibitor.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor.

An embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, for use in the treatment of a myeloproliferative neoplasm.

A particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, for use in the treatment of a myeloproliferative neoplasm.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, for use in the treatment of a myeloproliferative neoplasm.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, for use in the treatment of a myeloproliferative neoplasm.

A further particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, for use in the treatment of a myeloproliferative neoplasm.

A further more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, for use in the treatment of a myeloproliferative neoplasm.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, wherein the at least one inhibitor of a mRNA splicing and processing factor is a Ybx1 inhibitor, for use in the treatment of a myeloproliferative neoplasm.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm.

An embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, **for use** in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, **for use** in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A further particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A further more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent, and
wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, and
wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A still more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

A more particular embodiment of the present invention is a pharmaceutical composition comprising at least one inhibitor of a mRNA splicing and processing factor, and at least one JAK inhibitor, together with at least one pharmaceutically acceptable vehicle, excipient and/or diluent,
wherein the at least one inhibitor of a mRNA splicing and processing factor is a Ybx1 inhibitor, and
wherein the at least one inhibitor of a mRNA splicing and processing factor is selected from the group comprising a mRNA expression inhibitor, a protein expression inhibitor, a post-translational modification inhibitor, and a functional inhibitor, for use in the treatment of a myeloproliferative neoplasm, wherein the myeloproliferative neoplasm is selected from the group comprising polycythemia vera, primary myelofibrosis, essential thrombocythemia, chronic myeloid leukemia, acute myeloid leukemia, chronic eosinophilic leukemia, chronic myelomonocytic leukemia, systemic mastocytosis, idiopathic myelofibrosis, and myeloma.

"**Pharmaceutical composition**" refers to a preparation in a form that allows the biological activity of the active ingredient(s) to be effective, and which contain no additional components which are toxic to the subjects to which the formulation is administered.

As used herein, the terms "**pharmaceutically acceptable**", "**physiologically tolerable**" and grammatical variations thereof, are used interchangeably and represent that the materials are capable of administration to or upon a mammal without the production of undesirable physiological effects such as nausea, dizziness, gastric upset and the like.

"**Pharmaceutically acceptable carrier**" refers to an ingredient in a pharmaceutical composition, other than an active ingredient, which is nontoxic to the subject to whom it is administered. A pharmaceutically acceptable carrier includes, but is not limited to, a buffer, excipient, stabilizer, or preservative.

The pharmaceutical composition is designed to facilitate the administering of the inventive polypeptides comprising the single domain antibodies in an effective manner.

"**Pharmaceutically acceptable vehicle**" refers to an ingredient in a pharmaceutical formulation, other than an active ingredient, which is nontoxic to the subject to whom it is administered. A pharmaceutically acceptable vehicle includes, but is not limited to, a buffer, stabilizer, or preservative.

Examples of suitable vehicles or excipients include, without limitation, lactose, dextrose, sucrose, glucose, powdered sugar, sorbitol, mannitol, xylitol, starches, acacia gum, xanthan gum, guar gum, tara gum, mesquite gum, fenugreek gum, locust bean gum, ghatti gum, tragacanth gum, inositol, molasses, maltodextrin, extract of Irish moss, panwar gum, mucilage of isapol husks, Veegum, larch arabogalactan, calcium silicate, calcium phosphate, dicalcium phosphate, calcium sulfate, kaolin, sodium chloride, polyethylene glycol, alginates, gelatine, microcrystalline cellulose, polyvinylpyrrolidone, cellulose, water, saline, syrup, methylcellulose, ethylcellulose, hydroxypropylnethylcellulose, carboxymethylcellulose, polyacrylic acids such as Carbopols, such as Carbopol941, Carbopol980, Carbopol981,and gum bases such as Pharmagum™ (SPI Pharma Group; New Castle, Del.), and similar. Typically, the compositions of the present invention comprise from about 10% to about 90% by weight of the vehicle, the excipient or combinations thereof.

The pharmaceutical composition can be formulated into powders, granules, tablets, capsules, suspensions, emulsions, syrups, oral dosage form, external preparation, suppository or in the form of sterile injectable solutions, such as aerosolized in a usual manner, respectively. When formulated, it can be prepared using a **diluent** or **excipient** such as generally used fillers, extenders, binders, wetting agents, disintegrating agents, surface active agents.

In the pharmaceutical composition, the solid preparation for oral administration may be a tablet, pill, powder, granule, or capsule. The solid preparation may further comprise an excipient. Excipients may be, for example, starch, calcium carbonate, sucrose, lactose, or gelatine. In addition, the solid preparation may further comprise a lubricant, such as magnesium stearate, or talc. In the pharmaceutical composition, liquid preparations for oral administration may be best suspensions, solutions, emulsions, or syrups. The liquid formulation may comprise water, or liquid paraffin. The liquid formulation may, for excipients, for example, include wetting agents, sweeteners, aromatics or preservatives. For the purposes of parenteral administration, compositions containing the polypeptides of the invention are preferably dissolved in distilled water and the pH preferably adjusted to about 6 to 8.

Useful preparations of the compositions of the invention for parenteral administration also include sterile aqueous and non-aqueous solvents, suspensions and emulsions. Examples of useful non-aqueous solvents include propylene glycol, polyethylene glycol, vegetable oil, fish oil, and injectable organic esters.

For preparing solid compositions such as tablets, the principal active ingredient is mixed with a pharmaceutically acceptable carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to for a solid preformulation composition containing a homogeneous mixture for a compound of the present invention, or a pharmaceutically acceptable salt thereof. When referring to these preformulation compositions as homogeneous, it is mean that the active ingredient is dispersed evenly throughout the composition so that the composition may be easily subdivided into equally effective unit dosage forms such as tablets, pills and capsules. This solid pre-formulation composition is then subdivided into unit dosage forms of the type described above containing from 0.1 to about 500 mg of the active ingredient of the present invention. Typical unit dosage forms contain from 1 to 100 mg, for example, 1, 2, 5, 0, 25, 5 or 00 mg, of the active ingredient. The tablets or pills can be coated o otherwise compounded to provide a dosage affording the advantage of prolonged action. For example, the tablet or pill can comprise an inner dosage and an outer dosage component, the latter being in the form of an envelope over the former. The two components can be separated by an enteric layer which, serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol and cellulose acetate.
The liquid forms in which the composition of the present invention may be incorporated for administration orally or by injection include aqueous solutions, suitably flavoured syrups, aqueous or oi suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium caboxymethylcellulose, luethylcellulose, polyvinylpyrrolidone or gelatin.

### USES of the COMBINATIONS

Also described herein is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, or a pharmaceutical composition comprising said combination.

In particular, it is described a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor, or a pharmaceutical composition comprising said combination.

Also described herein is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, wherein the at least one inhibitor of a mRNA splicing and processing factor is a Ybx1 inhibitor, or a pharmaceutical composition comprising said combination.

Also described is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, wherein the at least one inhibitor of a mRNA splicing and processing factor is selected from the group comprising a mRNA expression inhibitor, a protein expression inhibitor, a post-translational modification inhibitor, and a functional inhibitor, or a pharmaceutical composition comprising said combination.

Also described herein is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, or a pharmaceutical composition comprising said combination, wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

Also described herein is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, or a pharmaceutical composition comprising said combination, wherein the myeloproliferative neoplasm is caused by and/or associated with a JAK2V617F mutation.

Also described herein is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, or a pharmaceutical composition comprising said combination, wherein the myeloproliferative neoplasm is persistent to JAK-inhibitor.

Also described herein is a **method for the treatment** of a myeloproliferative neoplasm, comprising administering to a patient a combination comprising a therapeutically effective amount of at least one inhibitor of a mRNA splicing and processing factor, and a therapeutically effective amount of at least one JAK inhibitor, wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1, or a pharmaceutical composition comprising said combination.

As used herein, the term "**administering**" refers to bringing a subject, tissue, organ or cells in contact with the combination comprising at least one inhibitor of a mRNA splicing and processing factor, and at least one JAK inhibitor, as described in this disclosure. In certain embodiments, the present invention encompasses administering the combination comprising at least one inhibitor of a mRNA splicing and processing factor, and at least one JAK inhibitor, as described in this disclosure to a patient or subject.

"**Treatment**", "**treat**", "**treating**", "**therapy**" and/or "**therapy regimen**" refers to clinical intervention in an attempt to alter the natural course of a disease or disorder in the individual being treated, and can be performed either for prophylaxis or during the course of clinical pathology. Desired results of treatment can include, but are not limited to, preventing occurrence or recurrence of the disorder, alleviation of symptoms, diminishment of any direct or indirect pathological consequences of the disorder, preventing metastasis, slowing or stopping the progression or worsening of a disease, disorder, or condition and/or the remission of the disease, disorder or condition, decreasing the rate of progression, amelioration or palliation of a disease state, and remission or improved prognosis. For example, treatment can include administration of a therapeutically effective amount of a pharmaceutical composition comprising one inhibitor of a mRNA splicing and processing factor, and at least one JAK inhibitor, to a subject to delay development or slow progression of a myeloproliferative disease.

"**Therapeutically effective amount**" refers to the amount of an active ingredient or agent (e.g., a pharmaceutical formulation) to achieve a desired therapeutic or prophylactic result, e.g., to treat or prevent a disease or disorder in a subject. In the case of a cancer, the therapeutically effective amount of the therapeutic agent is an amount that reduces the number of cancer cells ; reduces the primary tumour size ; inhibits (i.e. slows to some extent and preferably stop) cancer cell infiltration into peripheral organs; inhibits (i.e. slows to some extent and preferably stop) tumour metastasis; inhibits, to some extent, tumour growth; and/or relieves to some extent one or more of the symptoms associated with the cancer. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), the response rates (RR), duration of response, and/or quality of life.

The specific "**effective amount**" will, obviously, vary with such factors as the particular condition being treated, the physical condition of the patient, the duration of the treatment, the nature of concurrent therapy (if any), and the specific formulations employed. The optimum effective amounts can be readily determined by one of ordinary skill in the art using routine experimentation.

As used herein, the term "**subject**" and "**patient**" are used interchangeably herein and refer to both human and nonhuman animals. The term "nonhuman animals" of the disclosure includes all vertebrates, e.g., mammals and non-mammals, such as nonhuman primates, sheep, dog, cat, horse, cow, chickens, amphibians, reptiles, and the like. Preferably, the subject is a human patient that is at for, or suffering from, a cancer.

"**Cell proliferative disorder**" and "**proliferative disorder**" refer to disorders that are associated with some degree of abnormal cell proliferation, such as cancer.

"**Cancer**" and "**cancerous**" refer to, or describe a physiological condition in mammals that is typically characterized by a cell proliferative disorder. Cancer generally can include, but is not limited to, carcinoma, lymphoma (e.g., Hodgkin's and non-Hodgkin's lymphoma), blastoma, sarcoma, and leukemia. Myeloproliferative neoplasms are blood cancers that occur when the body makes too many white or red blood cells, or platelets.

An "ERK/MEK inhibitor," as used herein, includes any compound that disrupts mitogen-activated protein kinase enzymes (MEK and/or MEK2) or ERK production and or the MEK/ERK signaling pathway. MEK1 and MEK2 function by phosphorylating proteins in the Ras-Raf-MEK-ERK signaling pathway, thereby turning the pathway "on" and "off". Examples of MEK/ERK inhibitors include, but are not limited to, Trametinib (GSK1120212), Selumetinib (AZD6244), AS703026, binimetinib (MEK162, ARRY 438162), pimasertib (AST03026), refametinib (RDEA119), dacarbazine, fametinib, PD-0325901, TAK733, RO5126766, RO4987655 (CH4987655), WX-554, Cobimetinib GDC-0973 (XL518), CI-1040 (PD184352), and AZD8330, and the like.

Human MAPK interacting serine/threonine kinase 1 (MKNKs) (Gene ID: 8569) comprise a group of four proteins encoded by two genes (Gene symbols: MKNK1 and MKNK2) by alternative splicing. MKNK, a target at the junction of the Ras-Raf-MEK-ERK and PI3K-AKT-mTor pathways, modulates the function of elF4E through phosphorylation of a conserved serine residue (Ser209). In addition to elF4E, MKNKs mediate phosphorylation of several other target proteins such as Sprouty2, hnRNPA1 (heterogenous nuclear ribonucleoprotein A1), PSF (polypyrimidine tract-binding protein-associated splicing factor) and cPLA2 (cytosolic phospholipase A2) and therefore, play a key role in oncogenic progression, drug resistance, production of proinflammatory cytokines and cytokine signaling in cancer. MKNK activity is tightly regulated and is mediated by ERK (extracellular regulated kinase) and p38 MAPK binding.

A "Mknk1 inhibitor," as used herein, includes any compound that preventing Mknk1 activation and the downstream MKNK1-mediated phosphorylation and activation of eukaryotic translation initiation factor 4E (elF4E). Examples of Mknk1 inhibitor include, but are not limited to, CGP57380, Tomivosertib (eFT-508), ETC-206, SLV-2436, and Cercosporamide.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Description of the Figures

- Figure 1: shows on the left, the interplay between Ybx1, Mknk1, and ERK in JAK-inhibitor persistent cells with or without Ybx-1 inactivation. The workflow on the right depicts the combination therapy according to the present invention.

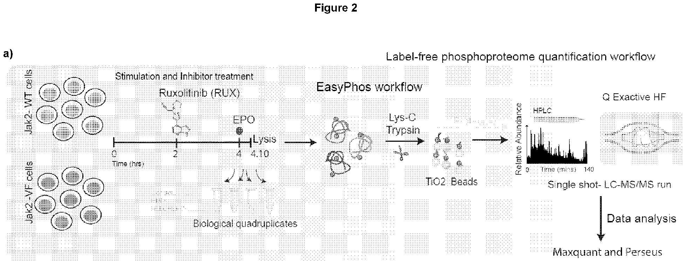

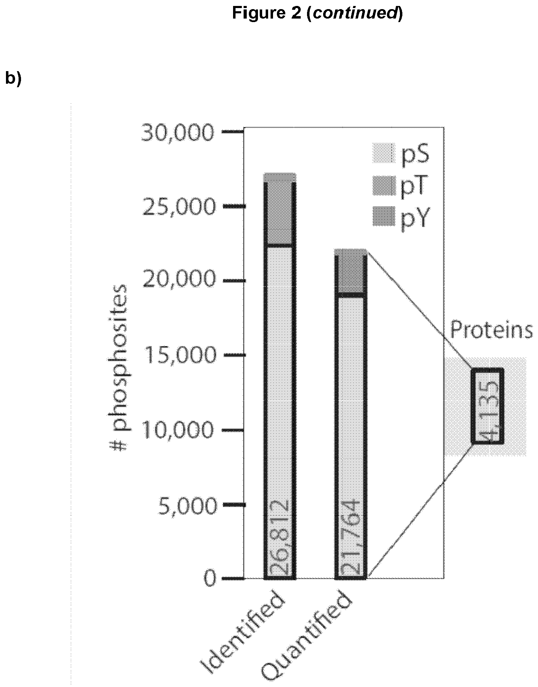

- Figure 2: shows schematic of the phosphoproteome workflow. a) Following sample collection, phosphopeptides were enriched using EasyPhos work flow (Humphrey, et al., 2015. High-throughput phosphoproteomics reveals in vivo insulin signaling dynamics. Nat Biotechnol 33). and analyzed in single-run LC-MS/MS. Data were analyzed in Maxquant and Perseus. b) summary of identified and quantified class-I phosphosites (localization probability of >0.75) corresponding to number of proteins of this experiment.

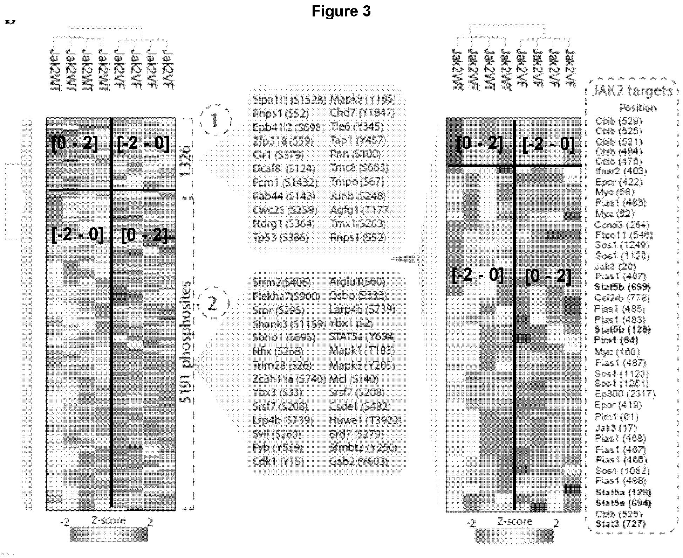

- Figure 3: shows the results of the functional phosphoproteomics screen identifying mRNA splicing and processing factors downstream of Jak2V617F. Unsupervised hierarchical clustering of significantly (t-test with permutation based FDR < 0.01) regulated phosphosites in Jak2WT and Jak2V617F cells. The numbers between square brackets indicate the range of Z-score. [0 - 2]: phosphorylation increase; [-2 - 0] phosphorylation decrease The panel highlight shows the top-upregulated phosphoproteins in WT (cluster-1, up) and VF (cluster-2, bottom) cells and the individual phosphosites with the amino acid position of significantly regulated Jak2-Stat signaling pathway members. Heatmap on the right shows enrichment of known Jak2 targets in both WT and VF cells.

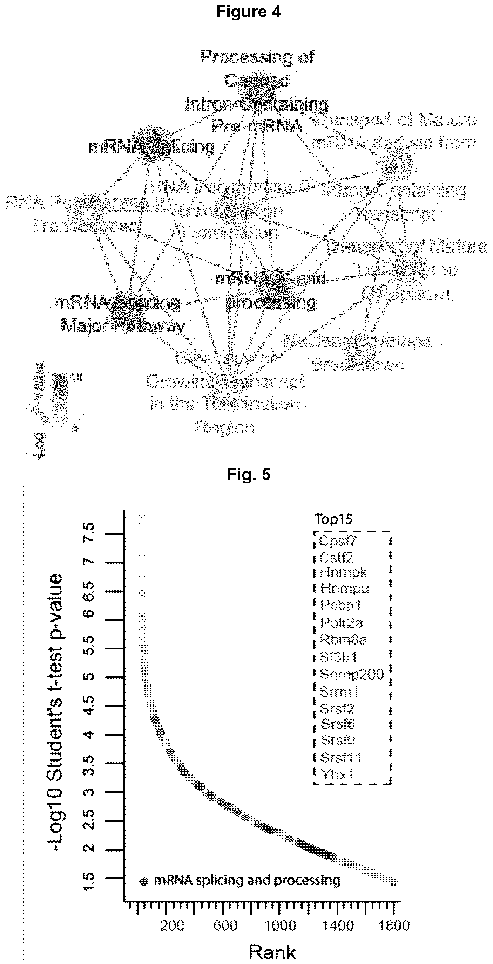

- Figure 4: shows sub-network map of significantly enriched Gene ontology (GO) terms (*p*-value < 0.01) of differentially phosphorylated proteins in Jak2V617F. The colours of the subnetwork nodes range from bright to transparency based on their p-value. Highlighted nodes indicate the core components of the sub-network based on the *p*-value.
- Figure 5: shows ranking of significantly phosphorylated proteins in Jak2V617F. Phosphorylated proteins participating in mRNA splicing and processing are highlighted in black with top 15 proteins displayed.

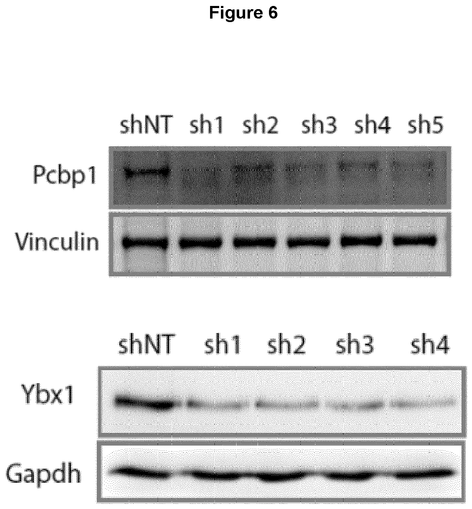

- Figure 6: shows western blot validation of shRNA library targets, Pcbp1 protein (on the top) and Ybx1 protein (on the bottom) in murine Jak2-V617F cells. The experiment was performed three times with similar results.

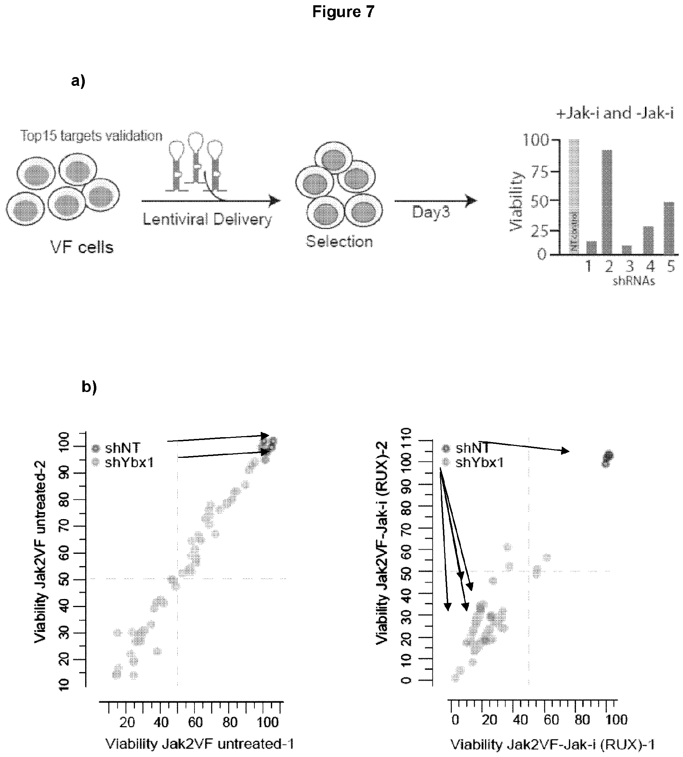

- Figure 7: a), b), c) show shRNA validation of selected top 15 targets essential for Jak2V617F cell survival and growth in the presence and absence of Jak-i (Rux, 0.5µM) measured by MTS assay. The graphs in c) show changes in cell viability depending on knockdown of respective differentially phosphorylated and splicing relevant genes (light grey dots) - with (right) or without (left) JAK-inhibitor treatment. shRNAs against Ybx1 (medium grey) and non-targeting control (dark grey) show dependency on Ybx1 only upon Jak-inhibitor treatment (right panel, dropout to the lower left quadrant of the right panel) but not per se (without JAK-I treatment) (left panel). Data obtained from 4 independent experiments each with 8 technical replicates.
- Figure 8: show *in vitro* viability of murine BaF3 Jak2V617 cells following lentiviral infection with shRNAs targeting the top 15 mRNA processing factors (selected in Figure 5) or with non-targeting control (shNT) in the presence and absence of Jak-i (Rux, 0.5µM). Data are mean±SD from 4 independent experiments each with 8 technical replicates, as measured by proliferation assay.

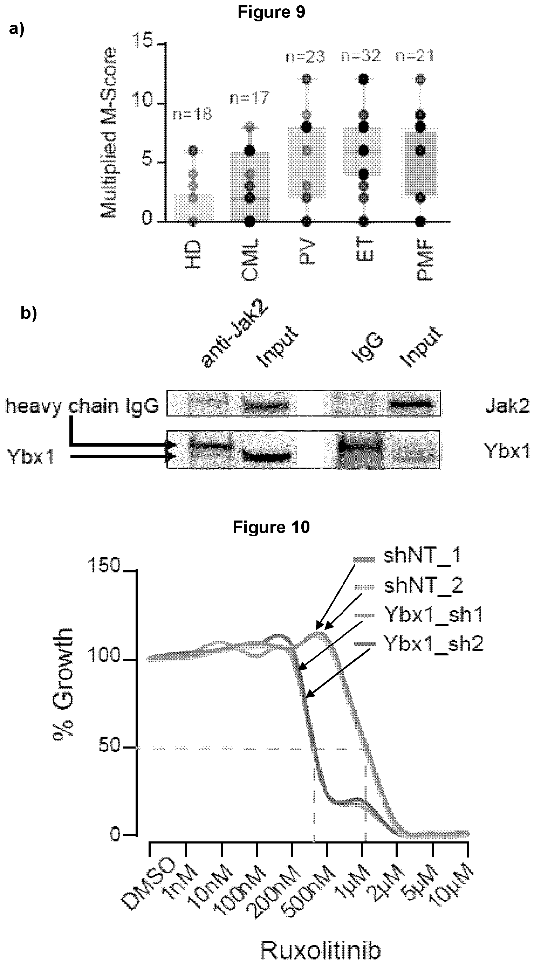

- Figure 9: a) shows quantification of Ybx1 positivity in bone marrow sections of Jak2V617F-positive patients using the multiplied M-score. Ybx1 positivity was analysed by immunohistochemistry in primary bone marrow biopsies of 93 myeloproliferative neoplasm patient samples compared to 23 healthy donor controls. Healthy donors (HD; n=18), BCR-ABL-positive chronic myelogeneous leukemia (CML; n=17). Jak2V617F positive biopsies: essential thrombocythemia (ET; n=32), polycythemia vera (PV; n=23) or primary myelofibrosis (PMF; n=21). b) Immunoprecipitation of Jak2 receptor from murine Jak2V617F cells showing binding of Ybx1 to mutated Jak2-receptor. The experiment was performed three times with similar results.
- Figure 10: shows cell growth curve of Jak2V617F cells following lentiviral infection with shRNAs targeting Ybx1 (Ybx1_sh1, Ybx1_sh2) or non-targeting control (shNT) and treatment with increasing doses of Jak-inhibitor (1nM - 10µM ruxolitinib) measured by MTS assay. Data obtained from 4 independent experiments each with 8 technical replicates.

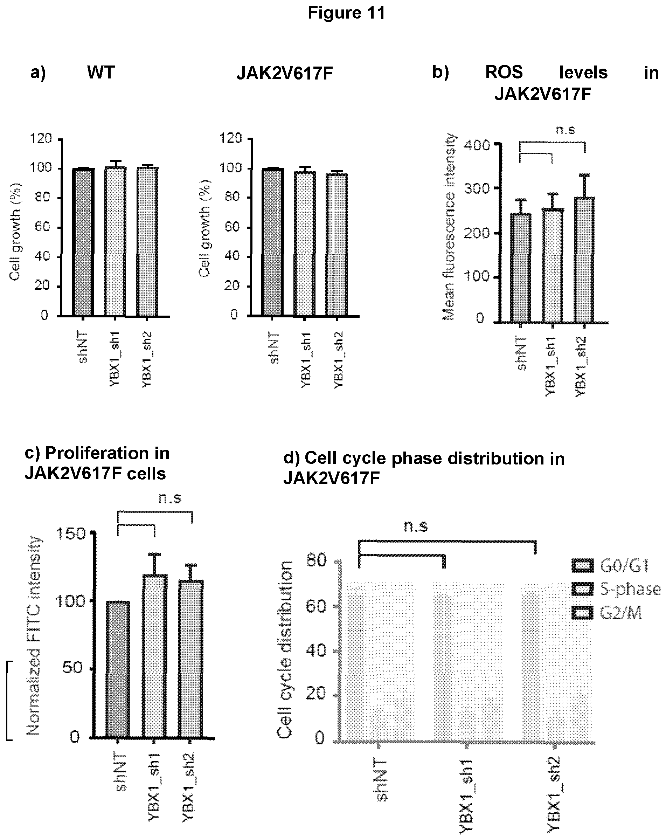

- Figure 11: shows functional consequences of Ybx1 depletion in Jak2-mutated cell lines. a) Percentage of cell growth in Jak2WT and in Jak2V617F cells following lentiviral infection with shRNAs targeting Ybx1 or non-targeting control (shNT) measured by MTS assay. Data obtained from 4 independent experiments each with 8 technical replicates. b) Representative histogram and bar plot showing ROS levels measured in Jak2V617F cells following treatment with shRNAs targeting Ybx1 or non-targeting control (shNT). Data obtained from 4 independent experiments. c) Bar plot showing proliferative marker PCNA levels measured by flow cytometry in Jak2V617F cells following treatment with shRNAs targeting of Ybx1 or non-targeting control (shNT). Data obtained from 4 independent experiments. d) Cell cycle analysis after Ybx1 inactivation in Jak2V617F cells following treatment with shRNAs targeting Ybx1 or non-targeting control (shNT).
- Figure 12: shows that inactivation of Ybx1 sensitizes Jak2V617F positive cells to Jak-inhibitor induced apoptosis. Percentage of apoptotic cells (AnnexinV⁺/Sytox⁺) of Jak2V617F positive cells following lentiviral mediated knockdown of Ybx1 (shYbx1-1 and shYbx1-2) compared to non-targeting control (scrambled shRNA, shSCR). a) murine BaF3-Jak2V617F cells, b) human Jak2V617F positive cell lines HEL and SET-2, c) primary murine lineage-negative bone marrow cells (Jak2^{+/+} and Jak2^{+/VF}).
- Figure 13: a) shows peripheral blood counts of recipient mice, week 8 and 16. White blood count (WBC), hemoglobin concentration (HGB) and platelet count (PLT) in Jak2V617F-Ybx1^{-/-} animals compared to Jak2V617F-Ybx1^{+/+} controls. b) peripheral blood chimerism of lethally irradiated (12Gy) recipient mice. Percentage of Jak2V617F-Ybx1+/+ or Jak2V617F-Ybx1-/- (CD45.2) cells / CD45.1-competitors, weeks 4 to 16. Each data point represents an individual mouse biological replicate. Statistical analysis by Mann-Whitney-U test unless otherwise specified. c) FACS plots showing percentage of apoptotic cells in Jak2+/+ and Jak2V617F/+ murine BM cells following Jak-i treatment (Rux 0.5 µM) after lentiviral knockdown of Ybx1 (sh1 and sh2) compared to non-targeting control (shNT).

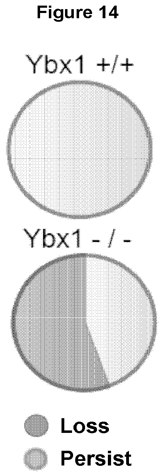

- Figure 14: shows percentage of animals with loss (dark grey) or persistence (grey) of Jak2V617F clones in Ybx1^{+/+} WT (upper panel) or Ybx1^{-/-} KO recipients, respectively.

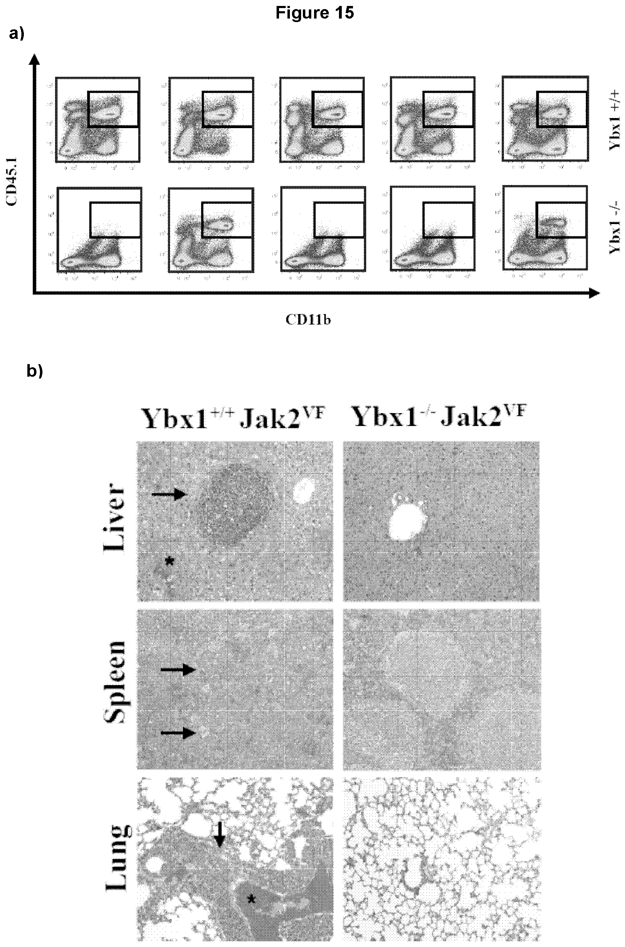

- Figure 15: a) shows peripheral blood chimerism of lethally irradiated (12Gy) recipient mice. FACS plots showing abundance of CD45.2 myeloid cells in Jak2V617F-Ybx1^{+/+} and Jak2V617F-Ybx1^{-/-} recipient mice at week 20 after bone marrow transfer (BMT). b) histology of liver, spleen and lung of Jak2V617F-Ybx1^{+/+} and Jak2V617F-Ybx1^{-/-} recipient mice at week 20 after BMT. Hematoxylin and eosin stain (H & E) at 10x magnification. Focal leukocyte infiltration (arrows) and haemorrhage (stars) of liver, spleen and lung, respectively.

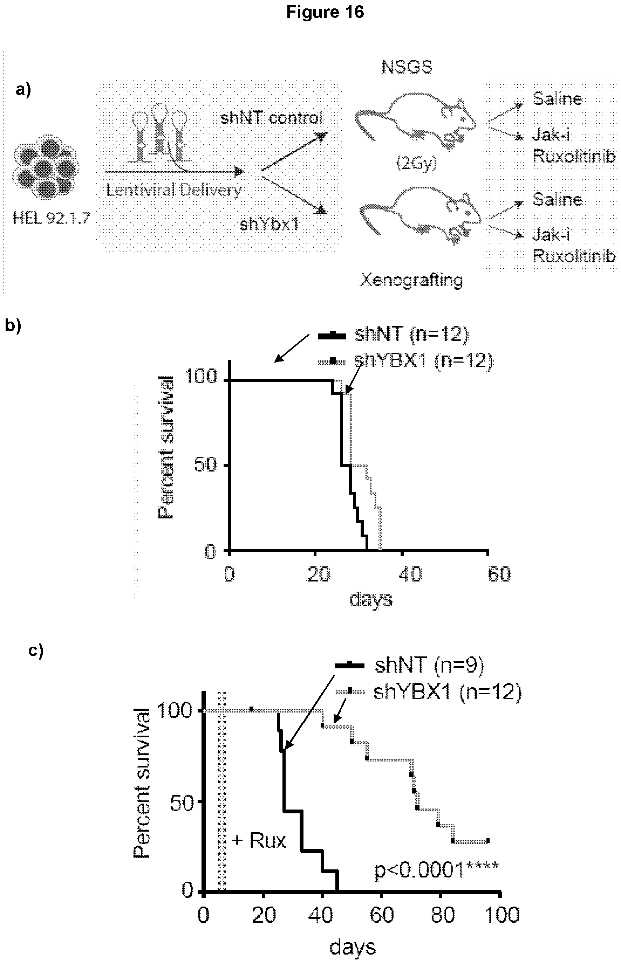

- Figure 16: a) shows a protocol for a JAK2-dependent xenograft model. b), c) Kaplan-Meier survival curves of irradiated (2Gy, single dose) recipient NSGS mice transplanted with Jak2V617F positive HEL cells treated with the indicated shRNAs (shSCR: scrambled negative control; shYBX1: targeting Ybx1).
- Figure 17: shows functional consequences of Ybx1 deletion in mouse hematopoietic stem- and progenitor cells. a) design for assessment of steady state haematopoiesis. b) white blood count (WBC), c) Gr-1 positive cells (Gr1⁺) d), hemoglobin (HGB) and e), platelets (PLT) following genetic inactivation of Ybx1 (Ybx1^{-/-}mice) compared to control mice (Ybx1^{+/+}).
- Figure 18: shows further results from the mice treated as shown in Figure 6. a) FACS plots showing comparable percentage of LSK-cells and HSCs (SLAM⁺CD34⁻L⁻S⁺K⁺ cells) following genetic inactivation of Ybx1 in conditional knockout mice (compared to wildtype littermate controls). b) total stem and progenitor cell numbers per 1 x 10⁶ whole bone marrow cells at week 16 after genetic inactivation of Ybx1. c) FACS plot showing comparable abundance of mature myeloid and erythroid cells following genetic deletion of Ybx1, d) total numbers of mature blood cells of the myeloid (Gr1⁺), erythroid (TER119⁺), B-lymphoid (CD19) and T-lymphoid (CD3) lineage at week 16 after genetic inactivation of Ybx1. LSK: Lineage⁻Sca-1⁺Kit⁺ cells. e) colony numbers of Ybx1^{+/+} versus Ybx1^{-/-} murine stem/progenitor cells. Colonies were counted at day 8 after plating. Each sample was plated in duplicate, and three repeats of the experiment were performed. Data shown as mean ± SD. f) spleen colony numbers counted on day 12 after injection of Ybx1^{+/+} or Ybx1^{-/-} LSK-cells into lethally irradiated (12Gy) recipient mice (CFU-S12). CMP: common myeloid progenitor cells, GMP: granulocyte-monocyte progenitor cells, MEP: megakaryocyte-erythrocyte progenitor cells
- Figure 19: a) shows dot plot showing the successful inhibition of respective targets of the corresponding kinase inhibitor used in this study (ANOVA test with Permutation based FDR < 0.01). The size and colour of the dots are proportional to the phosphosite intensity, Z-scored (log2 intensity). b) Dot plot showing changes in quantified Ybx1 phosphosites after various kinase inhibitor treatment (ANOVA test, permutation based FDR < 0.01). The highlighted Ybx1 pS30 phosphosite is the only site highly significantly downregulated upon MEK/ERK inhibitor treatment compared to 10 controls. The size and colour of the dots are proportional to the phosphosite intensity, Z-scored (log2 intensity).
- Figure 20: shows a network representation of Ybx1 interactome based on annotation keywords. The keywords are highlighted in colours according to the protein function. *n* = 4 biological replicates, t-test with Permutation based FDR < 0.05.

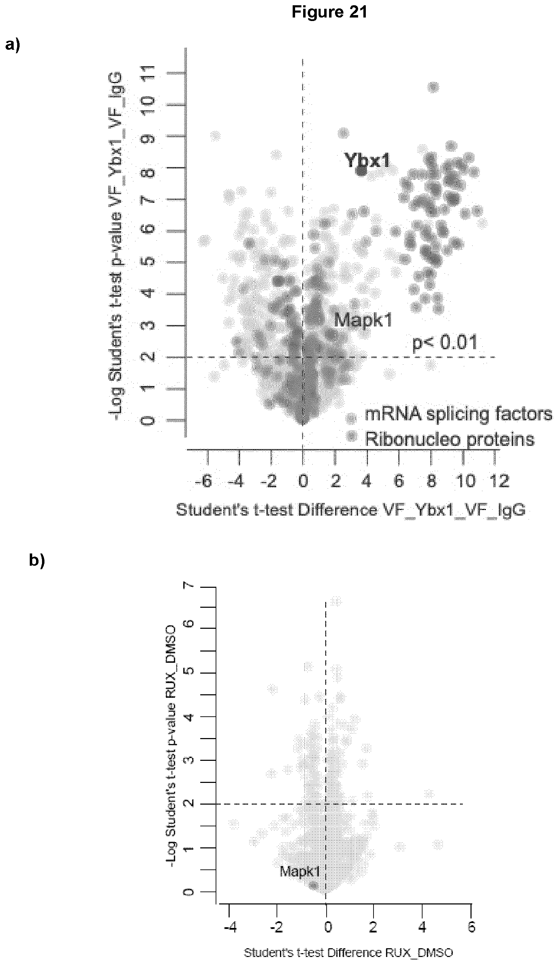

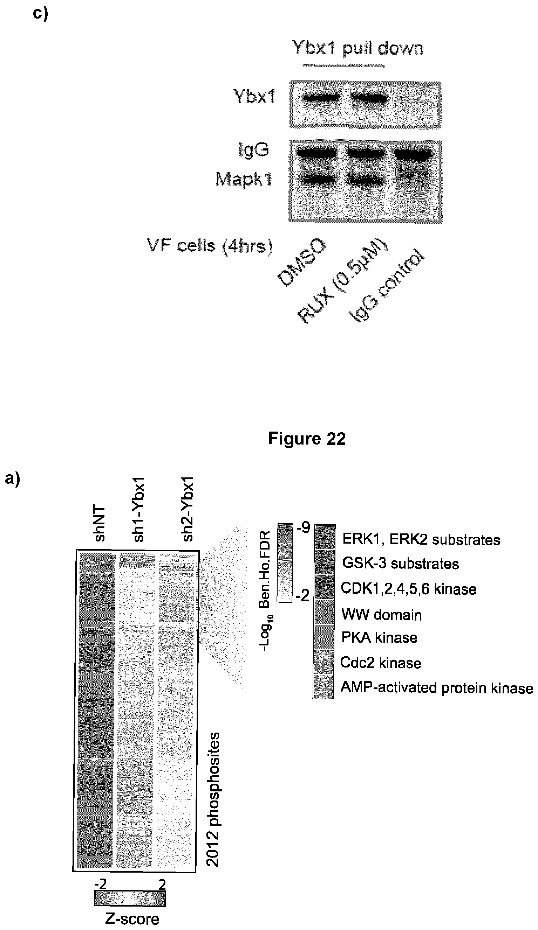

- Figure 21: a) shows scatter plot of Ybx1 interactome in Jak2V617F vs control. Ybx1 interactome is enriched for GO term mRNA splicing factors (green) and ribonucleoproteins (blue) assessed by Fisher's exact test. Fold enrichment of Ybx1 and Mapk1 in Jak2V617F cells compared to IgG control plotted against -log10. b) Scatter plot of Ybx1 interactome in DMSO vs Jak-i (Rux, 0.5µM treatment for 4 hours) treated Jak2V617F cells. Fold enrichment of Mapk1 in DMSO vs Jak-i plotted against - log10 student t-test p-value. c) Immunoprecipitation of Ybx1 from murine Jak2V617F cells with and without Jak-i treatment (Rux, 0.5µM for 4 hours) and analyzed for interaction with Mapk1 by western blot analysis using ERK1/2 antibody.
- Figure 22: shows that Jak2-mutated clones are selectively vulnerable to inhibition of ERK signaling. a) Unsupervised hierarchical clustering of significantly down regulated phosphosites (n=2012 sites) in murine Jak2-mutated cells following inactivation of Ybx1 by 2 shRNAs compared to non-targeting control. Biological replicates were averaged (n = 4), heatmap represents Z-scored log2 transformed phosphosite intensity values of sample assessed by ANOVA test with Permutation based FDR < 0.01. On the right, kinase-substrate motifs significantly down-regulated in Ybx1 targeted Jak2V617F cells are shown including Benjamin-Hochberg FDR value (-log10). b) Network map for proteins assigned as ERK substrates (significantly downregulated phosphosites, ANOVA test with permutation-based FDR <0.01).
- Figure 23: shows proteome analysis of murine Jak2V617F cells following inactivation of Ybx1 by 2 shRNAs compared to non-targeting control. Heat map representation of significantly enriched GO term biological processes is assessed by Fisher's exact test (P-value (- log₁₀) shown).

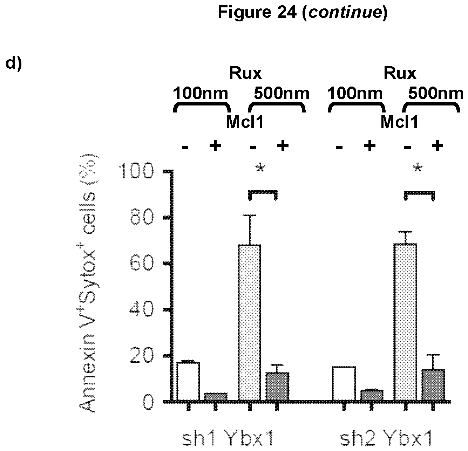

- Figure 24: a) shows quantification of Mcl-1 phosphosite pT144 (ANOVA test with Permutation based FDR < 0.01) following genetic inactivation of Ybx1 with two shRNAs (shYbx1-1 and shYbx1-2) compared to shNT control in murine Jak2V617F cells. The y-axis is the log2 intensity of the phosphopeptide. b) Scatter dot plot of Mcl-1 phosphosite pT144 after respective kinase inhibitor treatment (ANOVA test with Permutation based FDR < 0.01). The y-axis is the z-scored, log2 intensity of the phosphopeptide. c) Western blot analysis for relative protein abundance of Mcl1, Ybx1, Bim and Bcl-XL following genetic inactivation of Ybx1 with 4 different shRNA constructs (shYbx1 1-4), compared to non-targeting control (shNT). d) Measurement of apoptosis (AnnexinV / Sytox-positive cells) after genetic inactivation of Ybx1 and concomitant Jak-i treatment (Rux 100nM, 500nM), and ectopic overexpression of Mcl1. Results of 3 independent experiments. P = 0.0286 as determined by Mann-Whitney test.

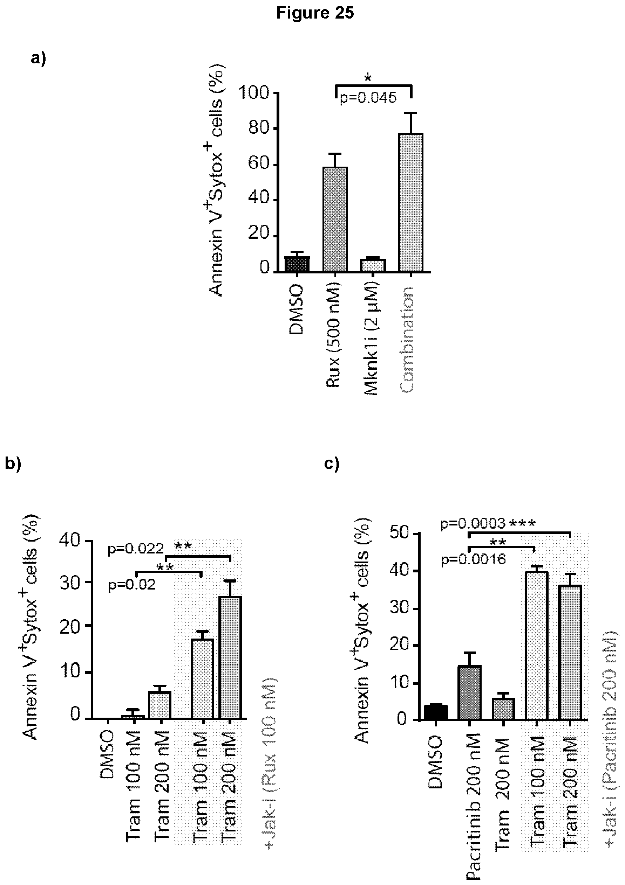

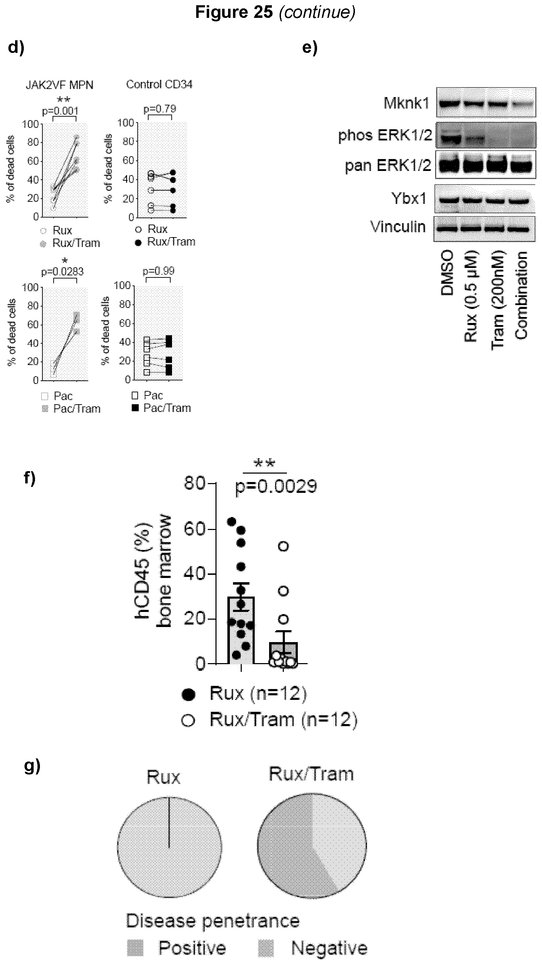

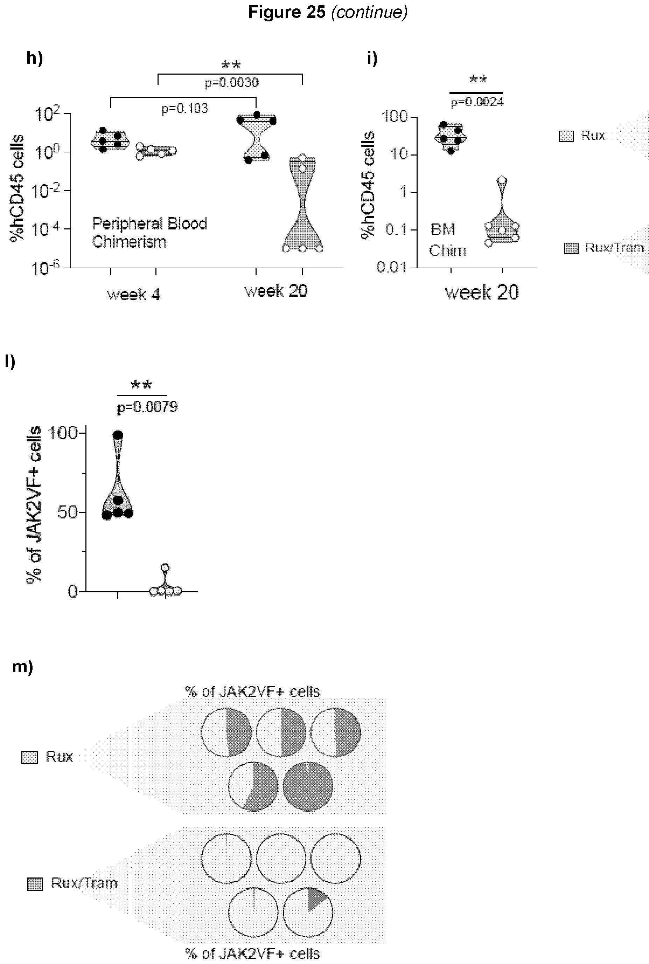

- Figure 25: shows pharmacologic inhibition of murine Jak2V617F cells using a) Jak-inhibitor (Ruxolitinib, Rux) alone or in combination with a Mknk1 inhibitor (2µM). b) Jak-i (Ruxolitinib, Rux) alone or in combination with the ERK-inhibitor trametinib (100nM, 200nM). Bar plot represents n= 4 individual experiments with statistical analysis by Mann-Whitney-U test. c) Jak2-i (Pacritinib) alone or in combination with the ERK-inhibitor trametinib (100nM, 200nM). Bar plot represents n= 4 individual experiments with statistical analysis by Mann-Whitney-U test. d) Induction of apoptosis after pharmacologic inhibition of FACS sorted CD34+ human bone marrow cells from patients with Jak2VF mutated neoplasms (n=6) and CD34+ healthy bone marrow cells (n=6) using Jak1/2 inhibitor Ruxolitinib (Rux, 500nM), Jak2 inhibitor Pacritinib (Pac, 200nM) or combinations with MEK/ERK inhibitor Trametinib (Tram, 200nM) as indicated. Aligned dot plot shows proportion of dead cells, p-values shown in the plot (Paired t-test). e) Representative Western blot showing downregulation of Mknk1 protein abundance in human HEL cells following overnight combination treatment with Ruxolitinb and Trametinib. Experiment was performed three times. f,g) Xenograft model using NSGS mice transplanted with Jak2VF positive HEL cells treated with Ruxolitinb alone (n=12) or in combination with Trametinib (n=12). f) Percentage of hCD45 positive cells in the bone marrow. Paired t-test. g) Percentage of disease penetrance in NSGS animals. hCD45 positivity (>1%, grey) or negativity (<1%, green) following RUX or RUX/Tram treatment, respectively. h) Peripheral blood and o, Bone marrow analysis of human cell chimerism in the treated mice in weeks 4 and 20. i) Molecular quantification of JAK2V617F allelic burden in sorted human cells derived from the bone marrow of recipient animals as determined by pyrosequencing. I) Pie chart of JAK2V617F allelic burden per individual mouse that were either RUX (grey) or RUX/Tram (green) treated.

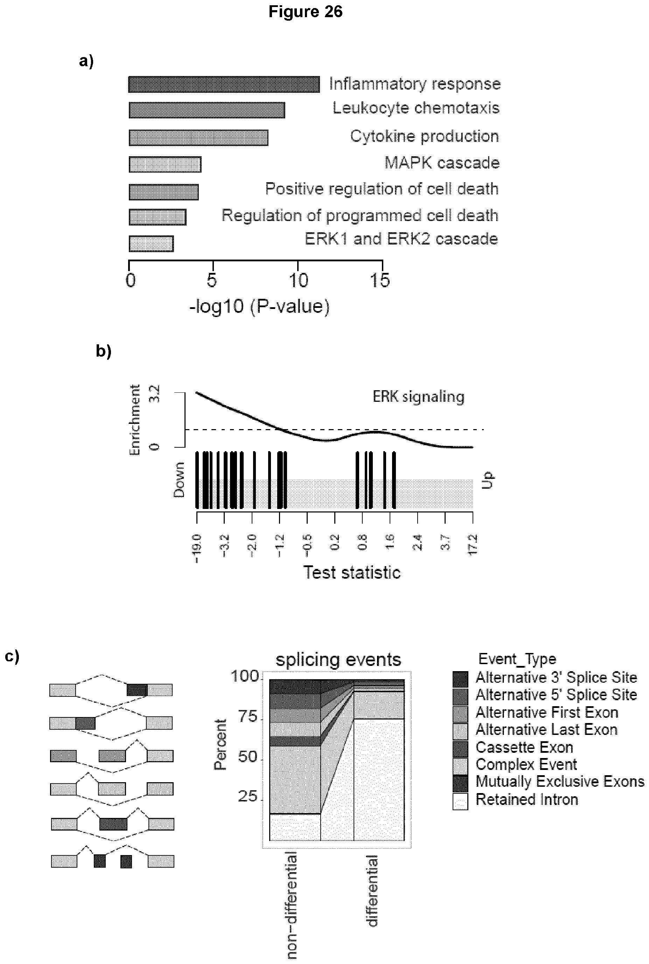

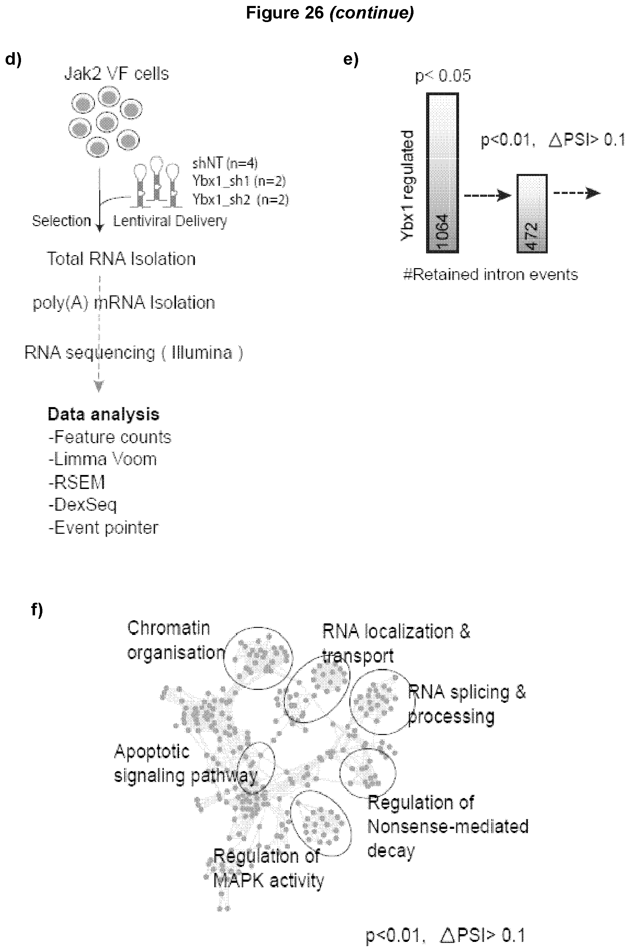

- Figure 26: shows that targeting Ybx1 in Jak2VF cells promotes retained introns. a) gene-ontology enrichment analysis of differentially expressed genes after inactivation of Ybx1 in murine Jak2VF cells. Bar plot shows significantly deregulated pathways with p-values on the x-axis. b) gene-set-enrichment analysis (GSEA) depicting negative enrichment of Erk-signaling related genes following inactivation of Ybx1. c) schematic representation of alternative splicing events types (left panel). Percentage and type of splicing events (1943 differential splicing events with p-value < 0.05) differentially regulated following inactivation of Ybx1. Retained intron (RI, colored yellow) being significantly upregulated splicing event (70% increase) following inactivation of Ybx1; a) Experimental design of RNA sequencing and data analysis performed in this study; b) Bars represent number of retained intron events significantly upregulated in Ybx1 depleted cells with p-value 0.05 (1064 RI events) then filtered for p-value 0.01 and delta-PSI 0.1 are down to 472 highly significant RI events. c) Network map displaying enrichment of gene sets in the 472 highly significant RI events. Each node represents significantly enriched gene sets. Clusters of functionally related gene sets are circled and labels are highlighted e.g, pathways involved in the regulation of MAPK- and apoptosis-signaling.
- Figure 27: shows in a) retained intron read density profile between indicated exon-intron locations of Araf, Braf and Mnkn1 genes in control and Ybx1 targeted. b) transcript (top) and protein (bottom) expression levels of Araf, Braf and Mnkn1 in control and inYbx1 targeted murine Jak2VF cells. The transcript levels in TPM were determined by RNA-seq and protein levels in Log 2-fold change were determined by proteome data (n=4, p-values using one tailed student t-test). c) Western blot validation of Mknk1 protein abundance following inactivation of Ybx1 in murine BaF3 Jak2VF and human HEL cells, experiment was performed three times with similar results.

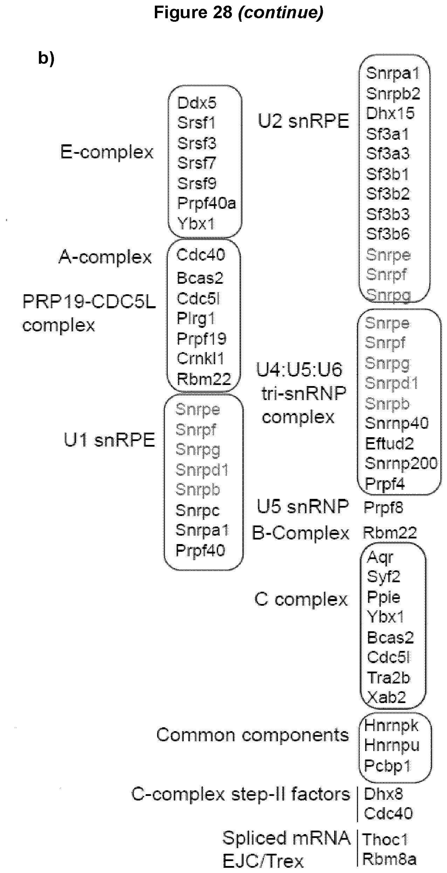

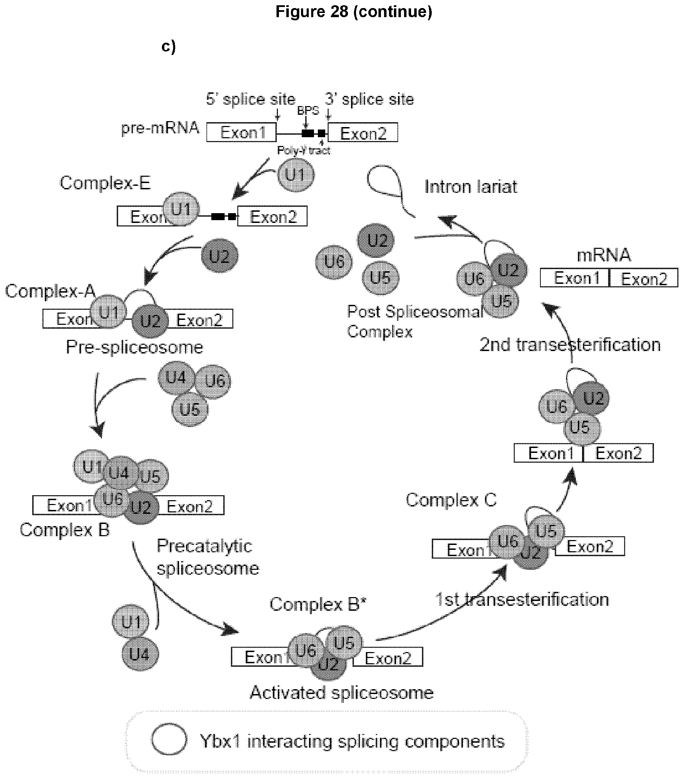

- Figure 28: shows in a) network representation of Ybx1 interacting spliceosomal proteins in Jak2VF cells. The size and color of the node indicates the abundance of the corresponding proteins (Z-scored protein intensity is used as mentioned in the figure) and the edges are connected by STRING database interactions. b) List of significant Ybx1 interacting spliceosomal proteins presented according to their spliceosome complex. c) Spliceosome proteins interacting with Ybx1 participate in spliceosome assembly reaction in a stepwise manner to excise intronic sequences from immature mRNA to form a mature mRNA. BPS
- Figure 29: shows generation of Ybx1 phospho-mimetic and phospho-null mutants. a) Western blot showing the expression of Ybx1 phosphomutants in BaF3 Jak2VF cells as indicated. b) Bar plot shows quantification of nuclear Ybx1 expression in Ybx1 phosphomutants expressing BaF3 Jak2VF cells, analysed by confocal microscopy. Data from 3 independent imaging experiments (p-values using student t-test). c) Cell growth curve of Ybx1 phosphomutants expressing BaF3 Jak2VF cells following treatment with increase doses of Jak-inhibitor (1nM-10µM RUX) measured by MTS assay. Data obtained from 4 independent experiments each with 8 technical replicates. d) Results of FACS analysis of apoptosis induction in Ybx1 phosphomutants expressing BaF3 Jak2VF cells following Jak-i treatment (RUX 0.5µM) compared to untreated Ybx1wt expressing BaF3 Jak2VF cells.

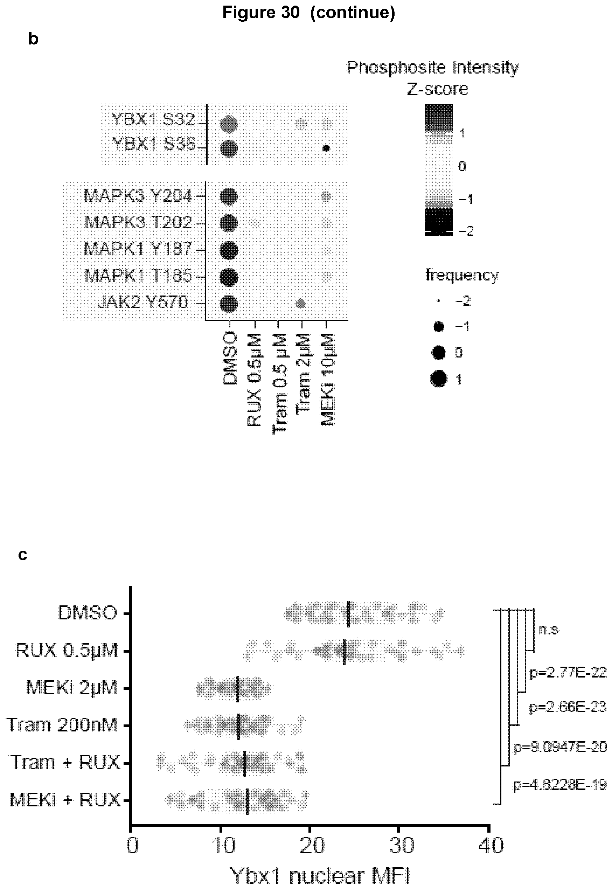

- Figure 30: shows that MEK-inhibition prevents Ybx1 nuclear localization in Jak2-mutated cells. a) Bar plot shows quantification of nuclear Ybx1 expression, analysed by confocal microscopy, in BaF3 Jak2VF cells after treatment with Ruxolitinib (0.5µM), MEKi (2µM), Trametinib (100nM and 200nM), Ruxolitinib in combination with Trametinib or DMSO control for 2 hours. Data from 3-4 independent imaging experiments (p-values using student t-test). b) Bubble plot showing the regulation of human Ybx1 pS32 and pS36 phosphorylation (representative of the murine Ybx1 pS30 and pS34) in HEL cells following treatment with Ruxolitinib (0.5µM), MEKi (10µM), Trametinib (500nM and 2µM) for 4 hours in vitro. Phosphorylation status of Mapk and Jak2 is shown as successful inhibition of respective targets of the corresponding kinase inhibitors. Following biological quadruplicates sample collection (n=4 per group), phosphopeptides were enriched using EasyPhos workflow and analyzed in single-run LC-MS/MS. Each data point is the averaged median of biological quadruplicate. Size and color of the bubbles are proportional to the Z-scored (log2 phosphosite intensity) and significance was tested using multiple sample test. c) Bar plot shows quantification of nuclear Ybx1 expression, analyzed by confocal microscopy, in HEL cells following treatment with respective inhibitors as shown for 2 hours. Data from 3 independent imaging experiments (p-values using student t-test).
- Figure 31: shows in a) Western blot showing the regulation of Mknk1 protein abundance in Ybx1 phosphomutants expressing Ba/F3 Jak2VF cells as indicated. Representative images from n=3 biological independent experiments.

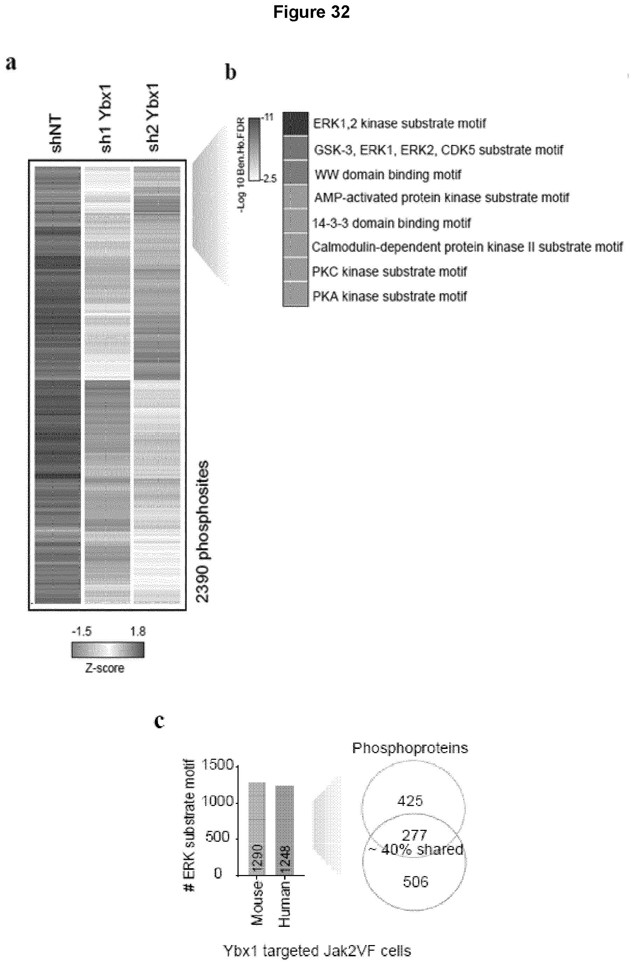

- Figure 32: a) Unsupervised hierarchical clustering of significantly down regulated phosphosites (n=2390 sites) in human HEL Jak2-mutated cells following inactivation of Ybx1 by 2 independent shRNAs compared to non-targeting control. Biological replicates were averaged (n=4), heatmap represents Z-scored log2 transformed phosphosite intensity values of sample assessed by ANOVA test with Permutation based FDR < 0.01. b) Kinase-substrate motifs significantly down-regulated in Ybx1 targeted HEL cells are shown including Benjamin-Hochberg FDR value (-log10). c) ERK substrate motifs significantly downregulated and shared between Ybx1 targeted mouse and human Jak2VF cells. d) Western Blot analysis of total protein abundance and phosphorylation of Jak2-downstream targets upon Jak-i treatment and/or genetic inactivation of Ybx1 by RNAi. Levels of GAPDH were used as loading control for each individual blot. Blots are representative of at least 3 independently performed experiments with similar results. e-f) Bar plots show the mean fluorescence intensity of pERK levels measured e) in human HEL and f) patient Jak2 mutated cells following genetic inactivation by RNAi with or without drug treatment as indicated. Representative FACS plots shown in Fig. 22c. Data shown as mean ± SD and p-value determined by two-tailed student t-test. g) Western blot validation of Mknk1 targeting shRNAs in murine BaF3 Jak2VF cells. h) Representative western blot analysis of pERK upon genetic inactivation of Mknk1 in BaF3 Jak2VF cells. The experiment was performed four times with comparable results. i) Growth curve of Jak2VF cells following lentiviral infection with shRNAs targeting Mknk1 or non-targeting control (shNT) and treatment with increase doses of Jak-inhibitor (1nM-10µM RUX) measured by MTS assay. Data obtained from 4 independent experiments each with 8 technical replicates. j) Percentage of apoptotic Jak2VF cells following lentiviral knockdown of Mknk1 (sh2 and sh3 with or without Ruxolitinib 0.5µM) compared to non-targeting control (shSCR). Data obtained from 4 independent experiments and p-value determined by two-tailed student t-test.

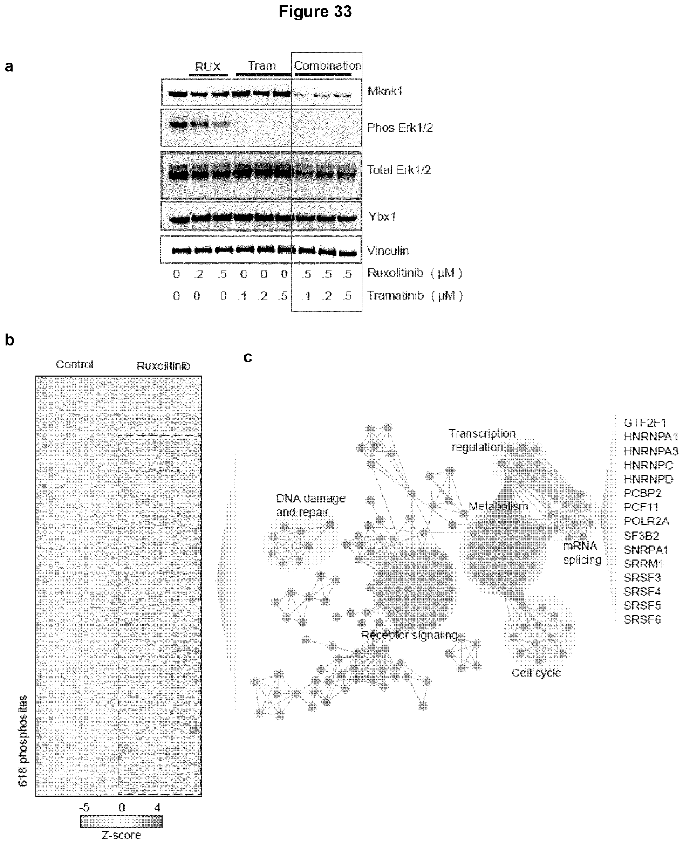

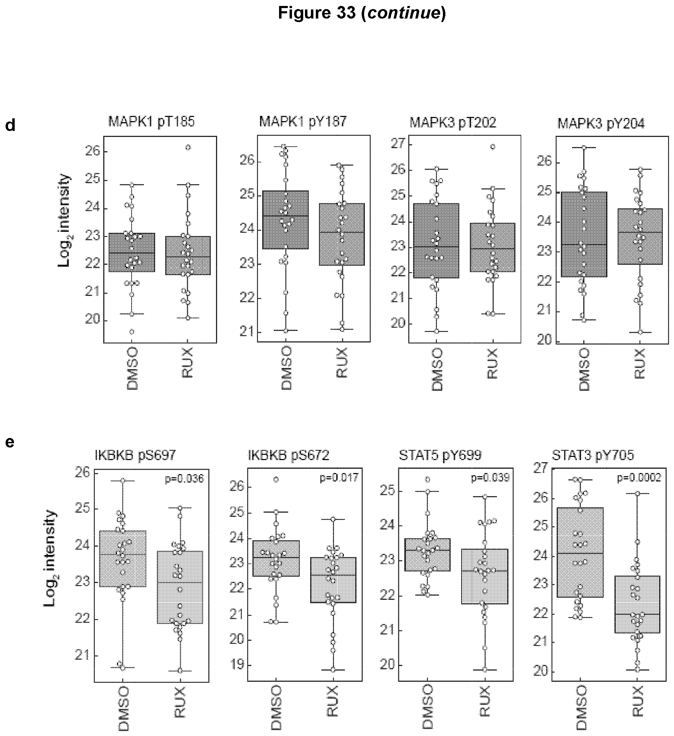

- Figure 33: a) Measurement of apoptosis (AnnexinV/Sytox-positive cells) after genetic inactivation of Ybx1 and concomitant Jak-i treatment (RUX 100nM, 500nM). Rescue by ectopic overexpression of Mcl-1. Results of 3 independent experiments. P=0.0286 as determined by Mann-Whitney test. b) Heatmap shows unsupervised hierarchical clustering of significantly regulated (t-test with permutation-based FDR < 0.01) phosphosites with (n=24) and without (n=24) Jak-i treatment in Jak2 mutated primary patient samples. Phosphoproteome analysis of Jak2 mutated primary patient samples (total n= 48) samples following in vitro (n=18, Jak-i treatment for 2 hours) or in vivo (n=6, 2 hours post dosing of Rux samples were collected) exposure to Ruxolitinib. c) Network map of significantly enriched GO terms (p-value < 0.01) of dephosphorylated proteins upon Jak-i treatment. Proteins involved in mRNA splicing are enriched and listed. d) Box plot shows no significant changes in the Mapk1 and Mapk3 phosphorylation in control vs Jak-i treated patient samples. e) Box plot shows significant changes in Ikbkb, Stat3 and Stat5 phosphorylation in control vs Jak-i treated patient samples. p-values as determined by Mann-Whitney test.

### Examples

### Methods

### Cell culture

Murine Ba/F3 cells stably expressing Jak2WT and Jak2V617F, human SET-2 and HEL 92.1.7 cells (purchased from DSMZ, Braunschweig, Germany) were cultured in RPMI 1640 medium (Life Technologies, Carlsbad, CA, USA) supplemented with 10% FBS (Life Technologies) in a humid atmosphere of 5% CO₂ at 37 °C. Cell lines were tested and maintained mycoplasma free throughout the study. Primary murine cells were cultured in StemSpan SFEM medium (Stemcell Technologies, Vancouver, Canada) supplemented with cytokines (100 ng/ml SCF, 10 ng/ml TPO, 6 ng/ml IL-3 and 10 ng/ml IL-6; Pepro Tech, Rocky Hill, NJ, USA).

### Primary patient samples

Patient samples and healthy donor controls were obtained after informed consent and according to the Helsinki declaration from the Tumor Banks in Jena and Magdeburg, approved by the respective local ethics committee (*Ethics Committe*, *University Hospital Jena* #4753/04-16 and *Ethics Committee, Medical Faculty, OvGU Magdeburg* #115/08).

### Focused lentiviral shRNA library screen

In brief, the Mission TRC lentiviral pLKO.1 shRNA vectors (Sigma Aldrich) targeting the top 15 hits of mRNA processing and splicing factors enriched in Jak2V617F were selected) and lentiviruses were individually produced per shRNA by co-transfecting with 3rd generation packaging plasmids pMDL, pRSV and pVSVG in HEK293T cells seeded in a 10 cm culture dishes. In total were used 74 shRNAs, 4 - 5 different shRNAs per each target (SEQ ID NO. 1 - 144), and 4 non targeting controls (Sigma Aldrich, catalog number: SHC016, SHC016V). Sequences for Cpsf7 were bought from Sigma Aldrich, catalog number SHCLNG-NM_172302, SEQ ID NOs 1-10. The viruses were collected at 48 hours and 72 hours after transfection, syringe filtered through a 0.45 µm syringe filter, concentrated using 30K Amicon Ultra-15 centrifugal filters (Merck) and the target murine BaF3 Jak2V617 cells were infected with polybrene (8mg / ml, Sigma). For screening purpose 200,000 murine BaF3 Jak2V617 cells in 2ml RPMI media with 10% heat-inactivated FBS (Invitrogen) were seeded in each well of a 6 well plates. Concentrated virus was added per well (with polybrene 8mg / ml), centrifuged at 500 x g for 1 hour at 35 °C, subsequently 48hrs after transduction cells were 1µg/ml puromycin selected for 2days. On day 3, cells were washed, viable cells were counted and plated in 96 well plates (8 technical replicates per sample condition per experiment. In addition, each experiment was performed in technical duplicates for growth assay with and without Jak2 inhibitor Ruxolitinib (0.5µM). The plates were incubated at 37°C and 5% CO₂ for 72 hours and subjected to Cell Titre 96 aqueous one solution (Promega) according to the manufacture's protocol. Further viable cells were counted after 72 hours with the countess automated cell counter (ThermoFischer Scientific) using trypan blue. Western blot was carried out to assess knock down efficiency on protein level for Pcbp1 and Ybx1. For data analysis, the 8 technical duplicates were averaged and the values were normalized against non-target controls included in each plates. The analysis of the normalized data between biological replicates showed correlation coefficient between r = 0.963 to 0.988 indicating high reproducibility of the procedure. Targets were considered potential candidates if 2 or more shRNA responded only in the Jak2 inhibitor treated group but not in the untreated controls of all the biological replicate experiments performed. Using these criteria, we choose our candidates for further characterization.

### Inactivation of murine and human Ybx1

In order to inactivate Ybx1, 4 shRNAs targeting mouse Ybx-1 (SEQ ID NO. 137-144) and 4 shRNAs targeting human Ybx-1 (SEQ ID NO. 159-162, Sigma-Aldrich, St. Louis, MO, USA) were validated and the two selected shRNAs (mouse Ybx1: SEQ ID NO: 141, 143; human Ybx1: SEQ ID NO: 159, 160) were used thereafter using respective non-targeting controls. In brief, Ba/F3, SET-2, and HEL cells were lentiviral transduced with lentiviral particles by centrifuging the cells at 872 x g for 1.5 hours at 33 °C. The cells were cultured for 2 days, puromycin selected for 48 hours and seeded (5x10⁶ cells) followed by inhibitor treatment or addition of diluent control as indicated below. Cells were harvested and Ybx-1 knock-down was checked by qPCR and western blotting.

### Jak-i dose dependent cell growth and viability in Ybx1-inactivated cells.

To analyse Jak inhibitor dose dependent cell growth and viability, murine BaF3 Jak2V617F cells treated with a non-targeting shNT control or shYbx1 (two different shRNA targeting Ybx1, sh1 and sh2) were counted with the Countess™ automated cell counter (Thermo Fischer Scientific) using trypan blue in 96 well plates after 2 days of 1µg / ml puromycin selection. 3x10⁴ viable cells per well (8 technical replicates per sample condition) in 96 well plate were seeded in RPMI medium with 10% heat-inactivated FBS and exposed to different concentrations of the Jak2 inhibitor Ruxolitinib ranging between 1 nM - 10 µM. The plates were incubated at 37 °C and 5%CO₂ for 72 hours and subjected to Cell Titer 96 Aqueous One Solution (Promega) according to the manufacture's protocol. Viable cells were counted after 72 hours by trypan blue. Determination of IC₅₀ inhibitory concentration of the Jak-i was calculated using GraphPad Prism.

### Drug combination treatments

Viable BaF3 Jak2V617F cells were seeded at a density of 30,000 cells per well in RPMI medium with 10% heat inactivated FBS and exposed to Ruxolitinib (0.5 µM) alone or in combination with different concentration of the Mknk1 inhibitor CGP57380 (Sigma), MEK/ERK inhibitor Trametinib (Novartis), PI3K inhibitor LY294002, and p38 MAP kinase inhibitor SB203580 (Merck) and incubated for 72 hours. Cell Titer 96 Aqueous One Solution was added to the plates according to the manufacture's protocol and measurements were performed after 4 hours. The plates were read at 490 nm in Tecan Infinite M200 and the responses were analyzed using GraphPad Prism.

### Apoptosis assays

Cells stably infected with either non-targeting or Ybx1 specific shRNA were seeded in six-well plates and selected for 24 hours with puromycin. Primary murine lineage-depleted cells or FACS-sorted human CD34⁺ cells were incubated in 48 well plates. Inhibitor treatment was performed at concentrations as indicated for 48 hours unless otherwise stated. Apoptosis was measured by flow cytometry on a BD FACS Canto™ cytometer using Annexin V in combination with SYTOX™ Blue or SYTOX™ Green as dead cell stains.

### Proliferation assay with PCNA

After puromycin selection of murine BaF3 Jak2V617F treated with non-targeting shNT or shYbx1, cells were washed twice with ice cold 1X PBS, fixed in 70% ethanol and permeabilized with 0.1% Tween-20. The cells were stained with PCNA-Alexa fluro488 conjugate (Biozol) on ice for 20 min.

### Cell cycle analysis

For cell cycle measurements, 2x 10⁶ murine BaF3 Jak2V617F cells stably expressing shNT or shYbx1 were washed in ice cold 1x PBS twice, fixed in ice cold 70% ethanol for 30 minutes on ice and stored at 4°C. After collection of biological replicates, samples were Ribonuclease A treated and stained with Propidium Iodide (PI). The PI stained cells were measured using BD Canto flow cytometer and data analyzed in FlowJo.

### ROS measurements using Carboxy-H₂DFFDA

In brief, 1x 10⁶ murine BaF3 Jak2V617F cells stably expressing shNT or shYbx1 were washed twice with 1X PBS and resuspended in 20µM carboxy-_{H2DFFDA} for 30 mins in dark at room temperature. Thereafter, the cells were washed thrice in 1X PBS and measured using BD FACSCanto™ cytometer. Data were analyzed in FlowJo.

### DNA damage analysis using yH2AX pS139

1x 10⁶ murine BaF3 Jak2V617F cells stably expressing shNT or shYbx1 cells were seeded on Poly-L-Lysin coated dishes for 2-4 hours, washed in PBS, fixed with 4% Paraformaldehyde, blocked in blocking buffer (0.2% Triton-X,1%BSA and 5%Normal rabbit serum) and incubated over night with rabbit yH2AX pS139 antibody (#CST 2577) overnight. After overnight incubation, samples were washed and incubated with secondary antibody (anti-rabbit-Alexa 568). DAPI was used for nuclear staining (NucBlue, ThermoScientific). Positive control samples were prepared by exposing the BaF3 Jak2VF cells stably expressing shNT to 20 mins UV under the cell culture hood. Imaging was performed using Zeiss LSM 780 microscope and processed in Zen Black software tool.

### Label free phospho-proteome sample preparation

Samples were collected as quadruplicate biological replicates for each condition, lysed in Guanidinium chloride (Gmdcl) buffer (6M Gdmcl, 100mM Tris pH8.5, 10mM TCEP and 40mM CAA), heated for 5 mins at 95°C and cooled on ice for 15 min. Lysed samples were then sonicated (Branson probe sonifier output 3 - 4, 50% duty cycle, 10 x 30 sec) and heated again. Proteins were precipitated with acetone, and quantified by bicinchoninic acid BCA assay. A protein sample of 2mg was digested with LysC and Trypsin overnight at room temperature and phosphopeptides enriched by TiO₂ beads. The enriched peptides were desalted, washed and eluted on StageTips with 2 layers of SDB-RPS material with elution buffer (80% Acetonitrile and 5% NH₄OH). The eluted peptides were vacuum centrifuged until dryness and reconstituted in 2% ACN /0.1% TFA. All the samples were stored in - 20°C until measurement.

### Phosphoproteome of primary patient samples

In brief, peripheral blood samples from patients with Jak2 mutated myeloproliferative neoplasms were collected, granulocytes isolated, and treated with DMSO or Ruxolitinb 0.5µM for 2hours (either in vitro or in vivo). Cells were lysed and processed in 4% SDC buffer (4%SDC,100mM Tris pH8.5, 10mM TCEP and 40mM CAA), heated for 5 mins at 95°C and cooled on ice for 15min. Lysed samples were sonicated, heated again for 5 mins and BCA quantified. Approximately 350µg of proteins were digested with LysC and Trypsin overnight at room temperature and phosphopeptides were enriched by TiO₂ beads as described elsewhere.

### Deep-proteome quantification.

Proteome samples of phosphoproteome analysis were collected after TiO₂ enrichment. In brief, cells were lysed in 1% SDC buffer (1%SDC, 100mM TrispH8.0, 40mM CAA and 10mM TCEP), heated for 5 mins at 95°C, cooled on ice for 15 mins and sonicated (Branson probe sonifier output 3 - 4, 50% duty cycle, 10 x 30 sec). 25 µg were digested with LysC and Trypsin overnight and peptides were eluted on Stage Tips with 3 layers of SDB-RPS material with elution buffer. The eluted peptides were vacuum centrifuged until dryness and reconstituted in 2% acetonitril (ACN) / 0.1% trifluoro acetic acid (TFA). All the samples were stored in - 20°C until measurement.

### Drug-perturbed phosphoproteome profiling

In order to profile the kinase inhibitor action on Jak2V617F cells, murine Jak2V617F BaF3 were treated with 0.5 µM Jak2 inhibitor Ruxolitinib (Selleckchem, S1378) for 2 hours and 10 µM MEK inhibitor PD0325901 (Sigma), 10 µM p38 inhibitor SB203580 (Merck), 20 µM JNK inhibitor SP600125 (Sigma), 50 µM PI3K inhibitor LY294002 (Merck), 10 µM AKT inhibitor MK2206 (Enzo Life) and 100 nM mTOR inhibitor Torin-1 (Millipore) for 1 hour. The cells were lysed in Gmdcl buffer and processed as mentioned in the phospho-proteome sample preparation protocol.

### Liquid chromatography (LC) -MS/MS measurement

For the LC-MS/MS analysis, Q-Exactive mass spectrometer with a nanospray ion source connected online to an Easy-nLC 1000 HPLC system was used. Peptides were separated on an in-house prepared 50cm C18 columns (75µM inner diameter with 1.9µM C18 ReproSil particle, Dr.Maisch GmbH) in a 140 minute gradient between 5% - 65% in buffer B (0.5% formic acid, 80% acetonitrile). The column temperature was maintained at 50°C using column oven (in-house made). Peptides were analyzed with a full scan (300-1600 m/z, R=60,000 at 200 m/z) at a target of 3e6 ions, followed by high energy collisional disassociation-based fragmentation (HCD) of top10 most abundant isotope patterns with a charge ≥2 MS/MS scan, detected in the orbitrap detector (R=15,000 at 200 m/z). Dynamic exclusion of sequenced peptides was set to 40s and apex trigger (4 to 7s) were on. All data were acquired using X-caliber software (Thermoscientific).

### Data processing with Maxquant

Mass spectrometric raw files were processed using the Andromeda search engine integrated into Maxquant 15 environment (1.5.5.2 version). The MS/MS spectra were matched against the mouse (UniProt FASTA 2015_08) database with an FDR < 0.01 at the level of proteins, peptides and modifications. The search included fixed modification for carbamidomethyl and in the variable modifications table phosphoSTY was added additionally for the phosphorylated peptide search to the default settings. Peptides with at least seven amino acids were considered for identification. Maximum two missed cleavages were allowed for protease digestion. Match between run was enabled with the matching window of 1 min to transfer peptide identification to across runs based on normalized retention time and high mass accuracy.

### Phosphoproteome data analysis

Perseus16 software (1.5.2.11 version) environment was used for all Maxquant output table analysis. For phosphoproteome analysis, sample for class -I phosphosites (localization probability >0.75) and required a minimum of 3 or 4 valid values in each of the biological quadruplicates Statistical analysis of was performed on the logarithmized intensities values. Significance was assessed by Student's t-test using permutation-based FDR, to identify the significantly regulated phosphosites. In group comparisons two sample t-test or for multiple samples comparison ANOVA test was performed with permutation-based FDR cut-off 0.01 or 0.05. The significantly regulated phosphosites were filtered, Z-scored and represented as either unsupervised hierarchical clustered heat maps or profile plot. Annotations were extracted from UniprotKB, Gene ontology (GO), Kyoto Encyclopedia of Genes and Genomes (KEGG) and Reactome. Kinase-substrates relationships were extracted from phosphosite plus database (phosphosite.org). Fischer exact test was performed to discover motifs and annotations that are significantly regulated in the sample groups. For phosphosite occupancy calculation, the proteome and phosphoproteome of corresponding samples were matched in maxquant to estimate occupancy, occupancy ratio and occupancy error scale using the extracted signal difference of modified peptide, unmodified peptide and the corresponding protein ratios (described earlier, 8). Phosphoprotein network architecture were obtained using String database and further networks and sub-networks were analyzed and visualized in Cytoscape.

### Ybx1 interactome preparation

For Ybx1 affinity purification, cells were lysed in 150mM NaCl, 50mM Tris (pH7.5), 5% glycerol, 1% IGPAL-CA-630 (Sigma), protease inhibitors (EDTA free, Roche), 1% Benzonase, and 1mM MgCl2 for 30 min on ice. 1mg of total lysate was incubated with validated Ybx1 antibody (Abcam# 76149) overnight and 30µl of rec-protein A sepharose 4B conjugates (Invitrogen) for 2 hours. Non-specific binders were removed by three washes with wash buffer I (150mM NaCl, 50mM Tris (pH7.5), 5% glycerol, 0.05% IGPAL-CA-630) and three washes with wash buffer II (150mM NaCl, 50mM Tris (pH7.5)).
The bound proteins were on-beads digested with Trypsin and LysC overnight. The peptides were desalted on C18 Stage tips and analyzed by mass spectrometry.

### RNA sequencing

Transcriptome profiling of BaF3 Jak2VF cells stably expressing shNT or shYbx1 cells was performed using a strand-specific RNA sequencing protocol described previously. In brief, total RNA was isolated using 2x10⁶ cells using NucleoSpin RNA Kit according to the Manufacturer's protocol (Macherey Nagel). RNA library for sequencing was prepared using NEBNext Poly(A) mRNA Magnetic Isolation Module. The quality was analyzed on a Bioanalyzer (Agilent 2100 Bioanalyzer) high sensitivity DNA assay. Samples were sequenced on Illumina Nexseq500 and multiplexed reads were demultiplexed on the basis of their barcodes. Sequencing reads were filtered, trimmed and then mapped to the Ensembl gene annotation and the mouse genome assembly GRCm38 using STAR aligner with ENCODE settings in two-pass mode considering splice junctions across all samples in the second mapping step. Gene counts were quantified using featureCounts 28 and differential expression calculated with the limma-voom pipeline. Gene and transcript expression levels were quantified using RSEM. Event level differential splicing was calculated with the EventPointer package in R.

### Ybx1 conditional knock out genotyping and gene expression by qPCR

Genotyping of tails and fetal livers was performed using the following primers: Ybx1_cond_for (GCCTAAGGATAGTGAAGTTTCTGG SEQ ID NO 145), Ybx1_con_rev (CCTAGCACACCTTAATCTACAGCC, SEQ ID NO 146), Cre_for (CGTATAGCCGAAATTGCCAG, SEQ ID NO 147), Cre_rev (CAAAACAGGTAGTTATTCGG, SEQ ID NO 148). Genotyping PCR was performed using the Dream Taq Green PCR Master Mix (2X) (Thermo Fisher Scientific, Waltham, MA, USA) following the manufacturers' protocol.

### Histology staining and immunohistochemistry

Formalin-fixed and paraffin-embedded bone marrow biopsies with proven myeloproliferative neoplasia or primary samples without histopathologic abnormalities were retrieved from the archival files of the Institute of Pathology, Otto-von-Guericke University Medical Center, Magdeburg, Germany. All MPNs were diagnosed and classified according to the World Health Organization (WHO) 2008 classification in synopsis with clinical data and presentation. The study comprises of biopsies derived from 76 MPN patients (PV (n=23), ET (n=32) and MF (n=21)) compared to healthy donor controls (n=18) or BCRABL positive CML (n=17). Immunohistochemistry was performed using a monoclonal Rabbit-antihuman Ybx1 antibody (Abcam; ab76149) in a dilution of 1:100.

### Hematopoietic progenitor cell assays

Colony formation assay: For investigation of colony formation in methylcellulose, LSK (Lin-Sca1+KIT+) cells were sorted from bone marrow of the respective donor mice as previously described. 1x10³ cells were seeded in MethoCult M3434 (Stem Cell Technologies), respectively. Colony numbers were counted on day 10 after plating using standard methods. Spleen colony formation assays (CFU-S12): bone marrow cells were collected from donor mice and 1x 10² LSK cells were FACS sorted and injected via tail vein into lethally irradiated (12Gy TBI) C57BL/6 recipient mice. At day 12 post-injection, spleens from recipient mice were harvested and stained with Bouin's fixative solution (Sigma-Aldrich), and colonies were counted using standard methods.

### Experimental animals

All mice were housed under pathogen-free conditions in the accredited Animal Research facility at the Animal Research Facility of the Otto-von-Guericke University - Medical Faculty, Magdeburg. All experiments were approved by the Landesverwaltungsamt Saxony-Anhalt, Halle, Germany. Conventional Ybx1 knockout mice have been generated as previously described 23. Mice harboring a 'floxed' (flanked with IoxP sites) allele of Ybx1 have been generated at Taconic-Artemis in a pure C57BL/6 background.

### Transplantation assays and in vivo treatment

For competitive repopulation assays 2x 10⁶ BM cells (for Jak2WT) or 5x 10⁴ sorted LSK cells (for Jak2V617F) of 6-8 week old Ybx1^{-/-} or Ybx1^{+/+} (CD45.2) littermates and 2x 10⁶ (CD45.1/2) competitor cells (derived from intercrossing CD45.1 animals with CD45.2 animals purchased from Charles River) were transplanted via lateral tail vein injection into lethally irradiated (12Gy, single dose) 6 - 8 week old Ly45.1 mice (Jackson Laboratories, Bar Harbor, ME). For serial transplantation experiments whole BM of primary recipient mice was harvested and 2x 10⁶ whole BMC were injected into lethally irradiated secondary recipients. Ruxolitinib was purchased at Selleckchem (Selleckchem, S1378) and formulated for administration by oral gavage as previously described. Mice received the Jak1/2 inhibitor ruxolitinib at a dose of 90 mg/kg or vehicle control by oral gavage BID. For xenografting of Jak2-mutated human cells, HEL cells were either infected with lentiviral particles for transduction of the respective shRNAs (shNT or shYbx1) or incubated for 24 hours with inhibitors as indicated. 1x 10⁶ viable cells were injected in each irradiated (2Gy) recipient NSGS mouse via lateral tail vain injection. Engraftment and expansion of human cells was monitored weekly by the presence of hCD45-positive cells in the peripheral blood. For patient derived xenograft experiments (PDX) we used an improved model for human HSC transplantation and analysis, that has been developed by her group from immune-deficient mouse strains containing Kit mutations (NSGW41). These mice can be engrafted without prior conditioning and therefore maintain an intact niche and microenvironment. Primary bone marrow samples were acquired during routine biopsies and cells were isolated by Ficoll gradient centrifugation followed by depletion of CD3 positive cells. 1.8-2x 10⁶ stem- and progenitor cells (HSPCs) were engrafted (pairwise) per animal. Mice were followed for 4 weeks and peripheral blood chimerism of human CD45 positive cells was measured by flow cytometry. Between weeks 4 and 20 all animals were treated for 5 days every 4 weeks with either Ruxolitinib (90mg/kg BID per gavage) or the combination of Ruxolitinib with the MEK/ERK-inhibitor Trametinib (1mg/kg QD per gavage).

### Quantification of JAK2VF mutant cells by pyrosequencing

In order to assess for the relative abundance of JAK2-mutated cells within the patient derived xenograft (PDX) model, we sorted human CD45 positive cells from the bone marrow at week 20 and performed pyrosequencing for the JAK2V671F mutation. DNA isolation and whole-genome amplification were carried out on FACS-sorted hCD45 positive cells using the REPLI-g Single Cell Kit (Qiagen, Hilden, Germany) according to the manufacturer's instructions. Amplicons were generated using AmpliTaq Gold DNA Polymerase (Thermo Fisher Scientific, Waltham, MA, USA; biotinylated forward primer: GAAGCAGCAAGTATGATGAGCA (SEQ ID NO 149); reverse primer: TGCTCTGAGAAAGGCATTAGAA (SEQ ID NO 150)) according to standard protocols. Samples were then analyzed by pyrosequencing (PyroMark Q96 ID, Qiagen, Hilden, Germany; sequencing primer: TCTCGTCTCCACAGA (SEQ ID NO 151)) to assess for the mutational status of the JAK2V617F variant of the individual subpopulations.

### Rescue of shRNA-inactivated endogenous Ybx1 with exogenous enforced expression of Mcl-1

Ba/F3 cells expressing EpoR (MSCV-EpoR-Neo) and Jak2V617F-GFP (MSCV-Jak2V617F-GFP) were infected with retrovirus expressing empty vector (MSCV-Puro) or Mcl-1 (MSCV-Mcl-1-Puro). Knockdown of Ybx1 was performed as indicated.

### Immunoprecipitation and Immunoblotting

Ba/F3 EpoR and wild-type Jak2 or Jak2V617F, respectively, were washed twice with PBS and starved for 4 h in serum reduced (0.5%) medium at a density of 1x10⁶/ml. For immunoprecipitation, the TrueBlot Anti-Rabbit Ig IP Beads Kit (Rockland Immunochemicals, Gilbertsville, PA, USA) was used following the manufacturer's instruction. The following antibodies were purchased from Cell Signaling (Danvers, MA, USA) and used at a 1: 1000 dilution: p-Akt (9271), Akt (9272), p-p44/42 MAPK (9106), p44/42 MAPK (9102), p-cRaf (9427), cRaf (9422) and p-Ybx1 (Ser102) (2900). GAPDH antibody (H86504M, 1: 5000) was purchased from Meridian Life Sciences (Memphis, TN, USA), p-Stat5 antibody (05-495, 1: 1000) was purchased from Millipore (Darmstadt, Germany) and Stat5 (sc-1081, 1: 100) antibody was purchased from Santa Cruz Biotechnologies (Dallas, TX, USA). Mcl-1 antibody (600-401-394, 1:1000) was delivered by Rockland (Limerick, PA, USA) and Ybx1 antibody (ab76149, 1:1000) was delivered by Abcam (Cambridge, UK).

### Flow Cytometry

For immunophenotype analysis, peripheral blood cells, bone marrow or spleen cells were resuspended in PBS/1% FBS after erythrocyte lysis (PharmLyseTM, BD Pharmingen). Unless otherwise stated, the following antibodies were used: Sorting and analysis of LSK-cells or Sca-1+cells were performed as previously described 25,26. Biotinylated antibodies against Gr-1 (RB6-8C5), B220 (RA3-6B2), CD19 (6D5), CD3 (145-2C11), CD4 (GK1.5), CD8 (53-6.7), TER119 and IL7Ra (A7R34) (all Biolegend, San Diego, CA, USA) were used for lineage staining. An APC-Cy7-or BV421-labeled streptavidin-antibody (Biolegend) was used for secondary staining together with an APC-anti-KIT (clone 2B8) and a FITC- or PE-anti-Sca-1 antibody (clone E13-161.7). Cells were analyzed using a FACSCantoIITM (Becton-Dickinson) cytometer. Analysis was performed using FlowJoTM software (Treestar, Ashland, OR). Fix&Perm Kit (Life Technologies) was used for intracellular staining according to the manufacturer's protocol.

### Immunofluorescence analysis (Jak2-Ybx1 co-localization)

2x 10⁴ cells were washed and seeded onto adhesion slides (Marienfeld). Attached cells were fixed in PBS/2 % PFA/0.01 % glutaraldehyde for 15 min on ice followed by permeabilization for 10 min with PBS/0.02% Triton X100 at room temperature. After blocking for 30 min with PBS/1%BSA/0.1% Tween 20, Ybx-1 (Abcam ab76149, 1:50) or pYbx-1 labeled with Alexa488 (Ser102) (BIOSSUSA bs-3477R-A488, 1:50) was incubated in blocking solution 1h. The samples were washed 5 times 5 min with PBS followed by incubation of Alexa Fluor 488 donkey anti-rabbit antibody (life technologies A21206, 1:200) for 1h. After additional washing, Jak2 labeled with Cy3 (BIOSSUSA bs-0908R-Cy3, 1:50) was incubated in blocking solution for 1h. In the following, the samples were washed, incubated with DAPI for 10 min and mounted using ProTaqs® Mount Fluor (quartett, 401603095). Samples were analyzed using confocal microscope Leipa SP8.

### Generation of Ybx1 phosphorylation mutants

Phophorylation mutants mimicking hyperphosphorylation or de-phosphorylation of Ybx1 were generated by site-directed mutagenesis at amino acid residues that were (i) highly conserved and (ii) differentially phosphorylated in the absence or presence of mutated JAK2 kinase. These aspects applied to the murine serine residues S30, S34, S172 and S174. In detail the following mutants were generated by site directed mutagenesis using a retroviral MSCV-IRES-GFP backbone (Addgene, plasmid #20672): (1) MIG-mYbx1-S30A/S34A (SEQ ID NO 152); (2) MIG-mYbx1-S30A (SEQ ID NO 153); (3) MIG-mYbx1-S34A (SEQ ID NO 154); (4) MIG-mYbx1-S30D/S34D (SEQ ID NO 155); (5) MIG-mYbx1-S30D (SEQ ID NO 156); (6) MIG-mYbx1-S34D (SEQ ID NO 157) and (7) MIGmYbx1-S172A/S174A (SEQ ID NO 158). Constructs were expressed in murine Jak2-mutated (Ba/F3-JAK2VF) cell lines. In brief, cells were infected by co-localization of virus supernatant (containing the respective constructs as indicated above) with Ba/F3-Jak2-V617F(VF) cells on retronectin-coated plates. Infection has been repeated after 24 hours and GFP-positive cells were sorted to ensure expression of the mutants in a homogeneous population.

### Statistical Analysis

For survival analysis, Kaplan-Meier curves were plotted using GraphPad Prism™ version 6.0h (GraphPad Software, SanDiego, CA). Differences between survival distributions were analyzed using the logrank test. Statistical analyses were performed using Student t test (normal distribution) or Mann-Whitney U test (when normal distribution was not given). P less than 0.05 was considered statistically significant (p<0.05 indicated as *, p<0.01 indicated as **, and p<0.001 indicated as ***).

### Example 1. Phosphoproteomic analysis of JAK2V617F mutants

To identify downstream effectors of the mutant Jak2 kinase that drive the evolution of persistent clones, we performed in-depth mass spectrometry (MS)-based phosphoproteomics in murine hematopoietic cells expressing erythropoietin-receptor and either Jak2-wildtype (Jak2WT) or mutated Jak2-V617F (Jak2V617F) kinase (**Fig. 2a**). Cells were exposed to either erythropoietin (EPO) alone or combined with a Jak-inhibitor (Jak-i; Ruxolitinib, Rux) to investigate EPO-independent and ruxolitinib -responsive signaling in Jak2V617F compared to Jak2WT. In total, we quantified 21,764 distinct class-I phosphorylated sites on 4135 different proteins (**Fig. 2b**). Of 6517 phosphosites significantly regulated in Jak2V617F and Jak2WT at a False Discovery Rate of 5%, 5191 were distinctly regulated in Jak2V617F cells on 1758 proteins, including bona fide Jak2 targets such as STAT5, STAT3, and PIM (**Fig. 3**). Kinase motifs of glycogen synthase kinase-3 (GSK3), ERK, and cyclin-dependent kinases (CDKs) were also enriched. Notably, the most highly enriched cellular processes in Jak2V617F cells were those of mRNA splicing and processing (**Fig. 4**).

### Example 2. Inactivation of mRNA splicing and processing factors sensitizes Jak2V617F mutant cells to treatment with Jak inhibitors.

To assess the potential functional role of protein members (n = 47) of the mRNA splicing and processing pathway, we chose 15 members that were significantly phosphorylated in Jak2V617F mutant on the basis of their statistical significance, i.e. p-value (**Fig. 5**), and subjected them to an RNA interfering (RNAi) based screening. Each candidate gene was targeted with 4 - 5 shRNAs, so that 70 shRNAs targeting 15 candidates and 4 non-targeting controls were used (western blotting, **Fig. 6**). shRNA- treated cells were analysed for knock-down efficiencies, and for growth with and without the Jak-inhibitor (Jak-i). Targeting Hnrnpk, Polr2a Srsf2, Srsf9, and Snrnp200 resulted in dropouts (**Fig. 8**), with at least 3 of 4 shRNAs displaying significant growth impairment. Interestingly, some of the targeted proteins identified in this screen appeared dispensable for proliferation and survival of Jak2V617F cells but their inactivation sensitized persistent cells to Jak-i treatment (**Fig. 7a**).

The most prominent candidate (4 shRNAs out of 4 targeted) that sensitized Jak2V617F cells to Jak-i treatment was the pleiotropic Y-box binding protein 1 (Ybx1) (**Fig. 7b****,** **8**), a member of core spliceosomal proteins and regulates mRNA splicing in various cellular contexts.

Ybx1 was highly expressed in 76 primary bone marrow (BM) biopsies of patients diagnosed with Jak2-mutated MPN (**Fig. 9a**) compared to normal bone marrow (n=18) or Jak2-negative CML (n=17). Moreover, Ybx1 colocalized with and bound to Jak2 in Jak2V617F-positive cells (**Fig. 9b**).

### Example 3. Genetic inactivation of Ybx1 in vitro

Genetic inactivation of Ybx1 in BaF3 Jak2V617F cells, resulted in a dose-dependent reduction of *in vitro* proliferation after exposure to Jak-i Ruxolitinib (IC50 reduction: 1000nM to 275nM; **Fig.10**). Surprisingly, Ybx1-depletion alone did not affect viability, proliferation, cell cycle activity, ROS production and DNA damage in Jak2WT and Jak2V617F cells (**Figs. 11a** - **11d**).
Reduction of Jak2V617F cell growth exposed to Jak-i in combination with Ybx1 inactivation could be attributed to induction of apoptosis. These findings were confirmed in Jak2V617F mutated murine (**Fig. 12a**) and human (**Fig. 12b**) cell lines, as well as primary lineage depleted Jak2V617F (Jak2^{+/VF}) murine bone marrow cells (**Fig. 12c**).

### Example 4. Genetic inactivation of Ybx1 in vivo

To assess Ybx1 as a potential therapeutic target in Jak2-mutated neoplasms, it was investigated if Ybx1 genetic inactivation would lead to a reduction of Jak-i persistent clones *in vivo.*

A conditional knockout mouse model was generated with Exon 3 of Ybx1 flanked with IoxP sites crossed with conditional Jak2V617F knock-in mice harboring an inducible Mx1-Cre recombinase. Bone marrows from Ybx1F/F Jak2V617F Mx+ and Ybx1^{+/+} or Ybx1^{+/-} Jak2V617F Mx⁺ littermate controls (CD45.2) was transplanted in a competitive manner along with 45.1 competitor cells. Following engraftment of transplanted cells, recipient animals received pIpC injections to activate Mx1-Cre and genetically delete Ybx1 with concomitant Jak-i medication by gavage.

Recipients of Jak2V617F Ybx1^{+/+} bone marrow showed hyperleukocytosis, thrombocytosis, and onset of symptomatic myeloproliferation (splenomegaly). In contrast, Ybx1-deficient Jak2V617F clones did not lead to symptomatic disease within 16 weeks after transplantation (**Fig. 13a**).

Peripheral blood (PB) chimerism revealed an increasing percentage of PB CD45.2/Jak2V617F positive cells, while genetic inactivation of Ybx1 resulted in suppression or loss of the Jak2-mutated clone (**Fig. 13b** and **Fig. 13c**). When followed for a total of 20 weeks, 5 of 9 recipients lost the Jak2V617F clone (<1% CD45.2 cells in PB and bone marrow, BM) while all controls notably increased PB chimerism (**Fig. 14**). 4 of 9 recipients of Ybx1F/F Jak2V617F Mx⁺ bone marrow showed counter-selection of clones with incomplete excision of Ybx1, indicating selective pressure. Consistently, development of myeloid hyperplasia in the BM (**Fig. 15a**) and organ infiltration (**Fig. 15b**) was blunted in recipients of Ybx1-depleted cells.

In a xenograft model of Jak2V617F mutated human cells, shRNA mediated inactivation of Ybx1 followed by saline injection did not result in significant delay of disease progression (**Fig. 16a, 16b**). However, concurrent Jak-i treatment with Ybx1 inactivation resulted in significantly prolonged survival and loss of disease penetrance in 4/12 (30%) recipients (**Fig. 16c**). Notably, Jak-i alone delayed disease progression only to a minor extent (Not treated (NT) + diluent: Overall Survival (OS) = 27 vs 30 days; NT + Jak-i: OS = 27 vs 72 days).

**These data suggest that the evolution of persistent Jak2-clones under pharmacologic Jak inhibition can be disrupted in human and murine cells by eliminating Ybx1.**

### Example 5. Safety of Ybx1 inactivation

To be clinically relevant, a treatment based on Ybx1 inactivation must have a suitable therapeutic index between hematopoietic stem- and progenitor cells (HSPCs) and their malignant counterpart. Fortunately, genetic inactivation of Ybx1 did not perturb steady-state haematopoiesis (**Fig. 17a****-e**) and Ybx1-deficient HSPCs (Lineage-Sca-1⁺Kit⁺ cells; LSK) did not reveal any disadvantage in colony forming potential or lineage commitment compared to Ybx1+/+ controls (**Fig. 18a-****f**). Thus, inactivation of Ybx1 does not impair HSPC function *in vitro* and *in vivo*, demonstrating to be clinically relevant.

### Example 6 Analysis of Ybx1-MAPK interaction

To further gain insight into the regulation of Ybx1 by mutant Jak2, the phosphoprofile of Ybx1 was investigated in Jak2V617F cells. Following treatment with EPO and Jak-i, Ybx1 was specifically phosphorylated at several phosphosites in Jak2V617F compared to Jak2WT cells (phosphosites: pS2, pS3, pS27, pS30, pS34, pS172, and pS174). To further characterize post-translational modulators of Ybx1, phosphoproteome analysis was performed following pharmacologic short-term inhibition of several bona fide Jak2 downstream effectors AKT, JNK, MEK, mTOR, p38, PI3K, and Jak (**Fig. 19a**). This identified significant changes in Ybx1 phosphorylation, and specifically the MEK/ERKi (PD0325901) responsive pS30 and pS34 residues (**Fig. 19b**).

To corroborate the Ybx1-MAPK interaction, we performed Ybx1 affinity purification combined with quantitative interaction proteomics, which revealed 260 Jak2-VF specific interactors. Among these, ribonucleoproteins, mRNA splicing factors and ribosomal proteins were significantly enriched, which also explains the large number of interactors (p<0.05). Most notably, several bona fide members of mRNA splicing complexes were identified as Ybx-1 interactors in Jak-2 mutated cells (**Fig.20**), that are known to interact during spliceosome formation and activation (44/134 core splicing factors that participate in several steps of dynamic spliceosome assembly, (**Fig. 28a****-c**)). Notably, it was also discovered that Jak2 and Mapk1 interact (**Fig. 20****,** **21a**) indicating that Ybx1 may be recruited and regulated by the Jak2-Mapk1 axis. Furthermore, the interactome of Jak-i treated Jak2-VF cells confirmed that Ybx1-Mapk1 interaction is Jak-i independent (**Fig. 21b****, c**). Collectively these data demonstrate that Ybx1 undergoes Jak2V617F-Mapk1 dependent phosphorylation and that Ybx1-Mapk1 interaction persists upon Jak-i treatment.

### Example 7 Ybx1 phosphomutants

In order to delineate the relevance of regulation of Ybx1 phosphorylation sites, we expressed Ybx1 phospho-mutants mimicking hyper- or hypo-phosphorylated states at conserved and relevant serine residues in Jak2-mutated cells (**Fig. 29a**). Expression of pS30A, pS34A and pS30A/34A double phosphomutants (but not pS30D, pS34D, pS30D/34D or pS170A/S172A mutants) resulted in reduction of nuclear Ybx1 translocation (**Fig. 29b****,c**). Moreover, these modifications recapitulated the phenotype of genetic inactivation and sensitized Jak-mutated cells to Jak-i mediated cell death (**Fig. 29d****,e**).

Consistently, MEKi treatment alone or in combination with ruxolitinib significantly prevented Ybx1 nuclear import, an effect that was not detectable on ruxolitinib treatment alone, in murine and human Jak-mutated cells (**Fig. 30a****-c**).
These data suggest that MAPK signaling stabilizes nuclear Ybx1, and this notion was further supported by impaired binding of Ybx1 pS30A and pS34A-containing phosphomutants to Mapk1 (**Fig. 30d**).
Collectively our data demonstrate that Ybx1 undergoes Jak2V617F-Mapk1 dependent phosphorylation and that Ybx1-Mapk1 interaction is crucial for Ybx1 nuclear translocation and persists despite Jak-i treatment.

### Moreover, these data show that inhibitors of Ybx1 phosphorylation could be used as therapeutic agent to sensitize Jak-mutated cells to Jak-i mediated cell death.

### Example 8 Transcription regulation by YBX1

In order to identify the transcriptional pathways controlled by Ybx1, RNA sequencing (RNAseq) analysis of murine and human Jak2-mutated cells was performed following inactivation of Ybx1 by RNA interference. Gene-ontology (GO) analysis of the differentially expressed coding genes revealed strong signatures of inflammation, chemotaxis and cytokine production but also of MAPK and ERK signaling and programmed cell death (**Fig. 26a****, b**).
Given the established role of Ybx1 in mRNA splicing, altered splicing events were analysed. Results showed an increase in intron retention (IR; 70% increase, 1064 events, p< 0.05 & ΔPSI>0.1) compared to other alternative splicing events (**Fig. 26c****,d**). Gene-ontology term analysis of intron retention highlighted that the involved genes (472 events, p< 0.01 & ΔPSI>0.1) are enriched for RNA splicing, nonsense-mediated decay, apoptosis and MAPK signaling (**Fig. 26 c****,****d,e**). Depending on their localization, intron retention results in initiation of translation, nuclear degradation or mRNA stabilization.
Thus, it was analysed whether ERK-signaling molecules that had increased intron retention such as Araf, Braf and Mknk1 were regulated in Jak-mutated cells (**Fig. 27a**). Comparison of global mRNA and protein abundance of ERK signaling proteins, showed a significant decrease of Braf (mRNA) and Mknk1 in Jak2-mutated cells (mRNA and protein) in Ybx1 targeted Jak-mutated cells (**Fig. 27b**). Western blot further confirms loss of Mknk1 expression in Ybx1 depleted Jak2V617F murine and human cells (**Fig. 27c**). Of note, Ybx1 ChIP-seq revealed no significant binding to Mknk1 in Jak2V617F cells (**Fig.31a,b**). Consistently, expression of Ybx1 phosphomutants (S30A, S34A, and S30A/S34A) resulted in reduction or abrogation of Mknk1 (**Fig.31c**), confirming that pS30/pS34 phosphorylation of Ybx1 in Jak2V617F cells is a critical requirement for efficient mRNA splicing and transcriptional regulation of Mknk1 transcripts and a mechanistic link to the Ybx1 dependent disruption of ERK-signaling.

### Example 9 Role of Ybx-1 in ERK-signaling and regulation of apoptotic factors McI1, Bim

Phosphoproteome profiling of Ybx1-inactivated Jak2V617F cells revealed significant downregulation of phosphosites (downregulated phosphosites, 2012 in murine and 2390 in human, (**Fig. 22a** **and** **Fig. 32a**) with key phosphorylation changes in substrates enriched for motifs of ERK1/2, GSK3 and CDK shared between the two shRNAs (**Fig. 22a** **and** **Fig. 32a**). Of these, Mknk1 and Mcl-1 were identified as relevant ERK targets (**Fig. 22b**).
To confirm these effects on cell signaling we investigated Jak2-dependent pathways in the presence or absence of pharmacologic Jak-i treatment. As observed on a global scale, inactivation of Ybx1 led to a considerable reduction of ERK phosphorylation in murine and human cells (**Fig. 22c** and **Fig. 32d****-f**) while leaving STAT signaling largely unaffected. Concomitant pharmacologic Jak inhibition resulted in abrogation of ERK-signaling, while decreasing STAT3/5 signaling irrespective of Ybx1 inactivation. These findings indicate that Ybx1 is required for maintenance of ERK-signaling downstream of Jak2V617F.

Likewise, genetic inactivation of Mknk1 by RNAi abrogated ERK signaling and sensitized the cells to Jak-i induced cell death (**Fig.32g****-j**), which links Ybx1 dependent Mknk1 expression to maintenance of ERK signaling in Jak2V617F cells.

In the proteome of Ybx1 depleted Jak2V617F cells, affected cellular functions besides RNA splicing and processing included positive regulation of programmed cell death and apoptosis (**Fig. 23**). Supporting this, inactivation of Ybx1 in Jak2V617F cells by RNAi was mirrored by reduction of pro-apoptotic Mcl-1 phosphorylation, quantified on the basis of pT144 phosphorylation required for Mcl-1 stability (**Fig. 24a****,b**). Likewise, Ybx1 loss resulted in concomitant decrease of Mcl-1 protein abundance and induction of Bim in a gene-dose dependent manner (**Fig. 24c**). Consistently, induction of apoptosis after Ybx1-kd and RUXOLITINIB treatment could be rescued by forced expression of Mcl1 (**Fig. 24d**).

### Example 10 Pharmacological modulation of Mknk1- or ERK-signaling in combination with Jak2 inhibitors.

Finally, pharmacological modulation of Mknk1- or ERK-signaling in combination with two different Jak-i resulted in induction of apoptosis in murine (**Fig. 25a****-c**) and primary human VF-mutated CD34+ BM cells but not in CD34+ healthy donor controls (**Fig. 25d**). This therapeutic combination of Jak2 and ERK inhibition dramatically downregulated Mknk1 protein levels in both murine and human cells indicating that Mknk1 is the primary target of this combination (**Fig. 25e** and **Fig. 33a**). Likewise, treatment of human Jak2V617F-mutated cell-lines with a combination of RUXOLITINIB and the ERK-inhibitor Trametinib (Tram) in a xenograft model reduced persistent cells (**Fig. 25f**) and disease penetrance (**Fig. 25g**) in vivo. These findings could be recapitulated using primary human Jak2V617F-mutated BM cells in a patient-derived xenograft (PDX) model of MPN. Here, in vivo treatment with a combination of RUXOLITINIB and Tram following engraftment of human cells resulted in significant decrease of hCD45 PB and BM chimerism (**Fig. 25h****,i**) and abrogated the Jak2V617F clone in 2/5 recipients as confirmed by pyrosequencing of FACS-isolated hCD45 cells (**Fig. 25l****, m**). To further consolidate ERK as a relevant target in Jak2 mutant cells, we performed in-depth phosphoproteome analysis following in vitro and in vivo exposure to Jak-i (ruxolitinib) in primary human Jak2-mutated cells. In primary cells, Jak-i treatment significantly altered 618 phosphosites including relevant mRNA splicing factors (**Fig. 33b****, c**). Consistent with our previous findings, Jak-i treatment did not affect MAPK phosphorylation to a major extent (**Fig. 33d**), unlike NfKB and STAT signaling (**Fig. 33e**).

## Claims

1. A combination for use in the treatment of a myeloproliferative neoplasm, wherein the combination comprises:
at least one inhibitor of a mRNA splicing and processing factor, and
at least one JAK inhibitor.

2. The combination for use according to claim 1, wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor.

3. The combination for use according to claim 1 or claim 2, wherein the at least one inhibitor of a mRNA splicing and processing factor is a Ybx1 inhibitor.

4. The combination for use according to any of the claims 1 - 3, wherein the at least one inhibitor of a mRNA splicing and processing factor is selected from the group comprising a mRNA expression inhibitor, a protein expression inhibitor, a post-translational modification inhibitor, and a functional inhibitor.

5. The combination for use according to any of the claims 1 - 4, wherein the myeloproliferative neoplasm is selected from the group comprising polycythemia vera, primary myelofibrosis, idiopathic myelofibrosis, essential thrombocythemia, chronic myeloid leukemia, acute myeloid leukemia, chronic eosinophilic leukemia, hypereosinophilic syndrome, chronic myelomonocytic leukemia, atypical chronic myelogenous leukemia, chronic neutrophilic leukemia systemic mastocytosis, juvenile myelomonocytic leukemia, and myeloma, post-polycythemia vera myelofibrosis or post-essential thrombocythemia myelofibrosis.

6. The combination for use according to any of the claims 1 - 5, wherein the myeloproliferative neoplasm is caused by and/or associated with a JAK2V617F mutation.

7. The combination for use according to any of the claims 1 - 6, wherein the myeloproliferative neoplasm is persistent to JAK-inhibitor.

8. The combination for use according to any of the claims 1 - 7, wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

9. A combination comprising:
at least one inhibitor of a mRNA splicing and processing factor, and
at least one JAK inhibitor.

10. A pharmaceutical composition comprising at least one inhibitor of a mRNA splicing and processing factor, and at least one JAK inhibitor, together with at least one pharmaceutically acceptable vehicle, excipient and/or diluent.

11. The pharmaceutical composition according to claim 10, wherein the at least one inhibitor of a mRNA splicing and processing factor is selected from the group comprising a Cpsf7 inhibitor, a Cstf2 inhibitor, a Hnrnpk inhibitor, a Hnrnpu inhibitor, a Pcbp1 inhibitor, a Polr2a inhibitor, a Rbm8a inhibitor, a Sf3b1 inhibitor, a Snrnp200 inhibitor, a Srrm1 inhibitor, a Srsf2 inhibitor, a Srsf6 inhibitor, a Srsf9 inhibitor, a Srsf11 inhibitor, and a Ybx1 inhibitor.

12. The pharmaceutical composition according to claim 10 or 11, wherein the at least one inhibitor of a mRNA splicing and processing factor is a Ybx1 inhibitor.

13. The pharmaceutical composition according to any of the claims 10 - 12, wherein the at least one inhibitor of a mRNA splicing and processing factor is selected from the group comprising a mRNA expression inhibitor, a protein expression inhibitor, a post-translational modification inhibitor, and a functional inhibitor.

14. The pharmaceutical composition according to any one of the claims 10 - 13, wherein the JAK inhibitor is selected from the group consisting of ruxolitinib, pacritinib, NS-018, CEP-33779, NVP-BVB808, TG101209, fedratinib, momelotinib, baricitinib, AZD960, AZD1480, tofacitinib, gandotinib, XL019, NVP-BSK805, peficitinib, pyridone 6, filgotinib, itacitinib, decernotinib, janex1, and JAK3-IN-1.

15. The pharmaceutical composition according to any one of the claims 10 - 14, for use in the treatment of a myeloproliferative neoplasm.


SEQUENCE LISTING

<110>  Universitätsklinikum Jena
<110>  Max-Planck-Gesellschaft zur Foerderung der Wissenschaften e.V.

<120>  Pharmaceutical combination for the treatment of
       myeloproliferative neoplasms

<130>  UNJ-P04211EP

<160>  162

<170>  PatentIn version 3.5

<210>  1
<211>  21
<212>  DNA
<213>  Artificial Sequence

<220>
<223>  Cpsf7 shRNA sense sequence 1

<400>  1
atccaggtta tccgctctat a                                                21


<210>  2
<211>  21
<212>  DNA
<213>  Artificial Sequence

<220>
<223>  Cpsf7 shRNA sense sequence 2

<400>  2
ataaccagct cttaggtatt t                                                21


<210>  3
<211>  21
<212>  DNA
<213>  Artificial Sequence

<220>
<223>  Cpsf7 shRNA sense sequence 3

<400>  3
ccagcgtgtt gccctacttt a                                                21


<210>  4
<211>  21
<212>  DNA
<213>  Artificial Sequence

<220>
<223>  Cpsf7 shRNA sense sequence 4

<400>  4
acagatcaga ttgacctata t                                                21


<210>  5

<211>  21
<212>  DNA
<213>  Artificial Sequence

<220>
<223>  Cpsf7 shRNA sense sequence 5

<400>  5
agaccggcat gatgattatt t                                                21


<210>  6
<211>  58
<212>  DNA
<213>  Artificial Sequence

<220>
<223>  Cpsf7 shRNA full hairpin sequence 1

<400>  6
ccggatccag gttatccgct ctatactcga gtatagagcg gataacctgg atttttg         58


<210>  7
<211>  58
<212>  DNA
<213>  Artificial Sequence

<220>
<223>  Cpsf7 shRNA full hairpin sequence 2

<400>  7
ccggataacc agctcttagg tatttctcga gaaataccta agagctggtt atttttg         58


<210>  8
<211>  58
<212>  DNA
<213>  Artificial Sequence

<220>
<223>  Cpsf7 shRNA full hairpin sequence 3

<400>  8
ccggccagcg tgttgcccta ctttactcga gtaaagtagg gcaacacgct ggtttttg        58


<210>  9
<211>  58
<212>  DNA
<213>  Artificial Sequence

<220>
<223>  Cpsf7 shRNA full hairpin sequence 4

<400>  9
ccggacagat cagattgacc tatatctcga gatataggtc aatctgatct gtttttg         58


<210>  10
<211>  58
<212>  DNA
<213>  Artificial Sequence

<220>
<223>  Cpsf7 shRNA full hairpin sequence 5

<400>  10
ccggagaccg gcatgatgat tatttctcga gaaataatca tcatgccggt ctttttg          58


<210>  11
<211>  21
<212>  DNA
<213>  Artificial Sequence

<220>
<223>  Cstf2 shRNA sense sequence 1

<400>  11
gctggcaaca aatctggaaa t                                                 21


<210>  12
<211>  21
<212>  DNA
<213>  Artificial Sequence

<220>
<223>  Cstf2 shRNA sense sequence 2

<400>  12
cctgtcatag agtcacctta t                                                 21


<210>  13
<211>  21
<212>  DNA
<213>  Artificial Sequence

<220>
<223>  Cstf2 shRNA sense sequence 3

<400>  13
gcacaggtag tgatgagaat t                                                 21


<210>  14
<211>  21
<212>  DNA
<213>  Artificial Sequence

<220>
<223>  Cstf2 shRNA sense sequence 4

<400>  14
gcaaagacag agtatcctaa t                                                 21


<210>  15
<211>  21
<212>  DNA
<213>  Artificial Sequence

<220>
<223>  Cstf2 shRNA sense sequence 5

<400>  15
gctttgatta tgcaggtcct t                                                21


<210>  16
<211>  58
<212>  DNA
<213>  Artificial Sequence

<220>
<223>  Cstf2 shRNA full hairpin sequence 1

<400>  16
ccgggctggc aacaaatctg gaaatctcga gatttccaga tttgttgcca gctttttg        58


<210>  17
<211>  58
<212>  DNA
<213>  Artificial Sequence

<220>
<223>  Cstf2 shRNA full hairpin sequence 2

<400>  17
ccggcctgtc atagagtcac cttatctcga gataaggtga ctctatgaca ggtttttg        58


<210>  18
<211>  58
<212>  DNA
<213>  Artificial Sequence

<220>
<223>  Cstf2 shRNA full hairpin sequence 3

<400>  18
ccgggcacag gtagtgatga gaattctcga gaattctcat cactacctgt gctttttg        58


<210>  19
<211>  58
<212>  DNA
<213>  Artificial Sequence

<220>
<223>  Cstf2 shRNA full hairpin sequence 4

<400>  19
ccgggcaaag acagagtatc ctaatctcga gattaggata ctctgtcttt gctttttg        58


<210>  20
<211>  58
<212>  DNA
<213>  Artificial Sequence

<220>
<223>  Cstf2 shRNA full hairpin sequence 5

<400>  20
ccgggctttg attatgcagg tccttctcga gaaggacctg cataatcaaa gctttttg        58

<210>  21
<211>  21
<212>  DNA
<213>  Artificial Sequence

<220>
<223>  Hnrnpk shRNA sense sequence 1

<400>  21
ccaaagattt ggctggatct a                                                21


<210>  22
<211>  21
<212>  DNA
<213>  Artificial Sequence

<220>
<223>  Hnrnpk shRNA sense sequence 2

<400>  22
aggaggaata attggtgtta a                                                21


<210>  23
<211>  21
<212>  DNA
<213>  Artificial Sequence

<220>
<223>  Hnrnpk shRNA sense sequence 3

<400>  23
ccttactttg tggtcactgt a                                                21


<210>  24
<211>  21
<212>  DNA
<213>  Artificial Sequence

<220>
<223>  Hnrnpk shRNA sense sequence 4

<400>  24
cggattaaac aaattcgtca t                                                21


<210>  25
<211>  21
<212>  DNA
<213>  Artificial Sequence

<220>
<223>  Hnrnpk shRNA sense sequence 5

<400>  25
cagtgctgat attgagacga t                                                21


<210>  26

<211>  58
<212>  DNA
<213>  Artificial Sequence

<220>
<223>  Hnrnpk shRNA full hairpin sequence 1

<400>  26
ccggccaaag atttggctgg atctactcga gtagatccag ccaaatcttt ggtttttg          58


<210>  27
<211>  58
<212>  DNA
<213>  Artificial Sequence

<220>
<223>  Hnrnpk shRNA full hairpin sequence 2

<400>  27
ccggaggagg aataattggt gttaactcga gttaacacca attattcctc ctttttg          58


<210>  28
<211>  58
<212>  DNA
<213>  Artificial Sequence

<220>
<223>  Hnrnpk shRNA full hairpin sequence 3

<400>  28
ccggccttac tttgtggtca ctgtactcga gtacagtgac cacaaagtaa ggtttttg          58


<210>  29
<211>  58
<212>  DNA
<213>  Artificial Sequence

<220>
<223>  Hnrnpk shRNA full hairpin sequence 4

<400>  29
ccggcggatt aaacaaattc gtcatctcga gatgacgaat ttgtttaatc cgtttttg          58


<210>  30
<211>  58
<212>  DNA
<213>  Artificial Sequence

<220>
<223>  Hnrnpk shRNA full hairpin sequence 5

<400>  30
ccggcagtgc tgatattgag acgatctcga gatcgtctca atatcagcac tgtttttg          58


<210>  31
<211>  21
<212>  DNA
<213>  Artificial Sequence

<220>
<223>  Hnrnpu shRNA sense sequence 1

<400>  31
cctgggaaat acaacattct t                                                21


<210>  32
<211>  21
<212>  DNA
<213>  Artificial Sequence

<220>
<223>  Hnrnpu shRNA sense sequence 2

<400>  32
gcgaggaaat aatcgtggct a                                                21


<210>  33
<211>  21
<212>  DNA
<213>  Artificial Sequence

<220>
<223>  Hnrnpu shRNA sense sequence 3

<400>  33
ccataactgt gcagttgaat t                                                21


<210>  34
<211>  21
<212>  DNA
<213>  Artificial Sequence

<220>
<223>  Hnrnpu shRNA sense sequence 4

<400>  34
cagtggtttg tcttgatact t                                                21


<210>  35
<211>  21
<212>  DNA
<213>  Artificial Sequence

<220>
<223>  Hnrnpu shRNA sense sequence 5

<400>  35
gcaataagaa taagagtggc a                                                21


<210>  36
<211>  58
<212>  DNA
<213>  Artificial Sequence

<220>
<223>  Hnrnpu shRNA full hairpin sequence 1

<400>  36
ccggcctggg aaatacaaca ttcttctcga gaagaatgtt gtatttccca ggtttttg          58


<210>  37
<211>  58
<212>  DNA
<213>  Artificial Sequence

<220>
<223>  Hnrnpu shRNA full hairpin sequence 2

<400>  37
ccgggcgagg aaataatcgt ggctactcga gtagccacga ttatttcctc gctttttg          58


<210>  38
<211>  58
<212>  DNA
<213>  Artificial Sequence

<220>
<223>  Hnrnpu shRNA full hairpin sequence 3

<400>  38
ccggccataa ctgtgcagtt gaattctcga gaattcaact gcacagttat ggtttttg          58


<210>  39
<211>  58
<212>  DNA
<213>  Artificial Sequence

<220>
<223>  Hnrnpu shRNA full hairpin sequence 4

<400>  39
ccggcagtgg tttgtcttga tacttctcga gaagtatcaa gacaaaccac tgtttttg          58


<210>  40
<211>  58
<212>  DNA
<213>  Artificial Sequence

<220>
<223>  Hnrnpu shRNA full hairpin sequence 5

<400>  40
ccgggcaata agaataagag tggcactcga gtgccactct tattcttatt gctttttg          58


<210>  41
<211>  21
<212>  DNA
<213>  Artificial Sequence

<220>
<223>  Pcbp1 shRNA sense sequence 1

<400>  41
ccatgatcca actgtgtaat t                                                  21

<210>  42
<211>  21
<212>  DNA
<213>  Artificial Sequence

<220>
<223>  Pcbp1 shRNA sense sequence 2

<400>  42
gcctaccaat gccatcttta a                                                21


<210>  43
<211>  21
<212>  DNA
<213>  Artificial Sequence

<220>
<223>  Pcbp1 shRNA sense sequence 3

<400>  43
gcaagacaac agtctcactt t                                                21


<210>  44
<211>  21
<212>  DNA
<213>  Artificial Sequence

<220>
<223>  Pcbp1 shRNA sense sequence 4

<400>  44
ccatgaactc accattccaa a                                                21


<210>  45
<211>  21
<212>  DNA
<213>  Artificial Sequence

<220>
<223>  Pcbp1 shRNA sense sequence 5

<400>  45
cctggcccag tatctaatca a                                                21


<210>  46
<211>  58
<212>  DNA
<213>  Artificial Sequence

<220>
<223>  Pcbp1 shRNA full hairpin sequence 1

<400>  46
ccggccatga tccaactgtg taattctcga gaattacaca gttggatcat ggtttttg        58


<210>  47

<211>  58
<212>  DNA
<213>  Artificial Sequence

<220>
<223>  Pcbp1 shRNA full hairpin sequence 2

<400>  47
ccgggcctac caatgccatc tttaactcga gttaaagatg gcattggtag gctttttg          58


<210>  48
<211>  58
<212>  DNA
<213>  Artificial Sequence

<220>
<223>  Pcbp1 shRNA full hairpin sequence 3

<400>  48
ccgggcaaga caacagtctc actttctcga gaaagtgaga ctgttgtctt gctttttg          58


<210>  49
<211>  58
<212>  DNA
<213>  Artificial Sequence

<220>
<223>  Pcbp1 shRNA full hairpin sequence 4

<400>  49
ccggccatga actcaccatt ccaaactcga gtttggaatg gtgagttcat ggtttttg          58


<210>  50
<211>  58
<212>  DNA
<213>  Artificial Sequence

<220>
<223>  Pcbp1 shRNA full hairpin sequence 5

<400>  50
ccggcctggc ccagtatcta atcaactcga gttgattaga tactgggcca ggtttttg          58


<210>  51
<211>  21
<212>  DNA
<213>  Artificial Sequence

<220>
<223>  Polr2a shRNA sense sequence 1

<400>  51
gccctaacta ttctccaact a                                                  21


<210>  52
<211>  21
<212>  DNA
<213>  Artificial Sequence

<220>
<223>  Polr2a shRNA sense sequence 2

<400>  52
gctcataaca atgagctaga a                                                21


<210>  53
<211>  21
<212>  DNA
<213>  Artificial Sequence

<220>
<223>  Polr2a shRNA sense sequence 3

<400>  53
cgagtgcatg aaatcttcaa a                                                21


<210>  54
<211>  21
<212>  DNA
<213>  Artificial Sequence

<220>
<223>  Polr2a shRNA sense sequence 4

<400>  54
gctacactta aaccttctaa t                                                21


<210>  55
<211>  21
<212>  DNA
<213>  Artificial Sequence

<220>
<223>  Polr2a shRNA sense sequence 5

<400>  55
ccgctcatga aatgctcctt t                                                21


<210>  56
<211>  58
<212>  DNA
<213>  Artificial Sequence

<220>
<223>  Polr2a shRNA full hairpin sequence 1

<400>  56
ccgggcccta actattctcc aactactcga gtagttggag aatagttagg gctttttg        58


<210>  57
<211>  58
<212>  DNA
<213>  Artificial Sequence

<220>
<223>  Polr2a shRNA full hairpin sequence 2

<400>  57
ccgggctcat aacaatgagc tagaactcga gttctagctc attgttatga gctttttg          58


<210>  58
<211>  58
<212>  DNA
<213>  Artificial Sequence

<220>
<223>  Polr2a shRNA full hairpin sequence 3

<400>  58
ccggcgagtg catgaaatct tcaaactcga gtttgaagat ttcatgcact cgtttttg          58


<210>  59
<211>  58
<212>  DNA
<213>  Artificial Sequence

<220>
<223>  Polr2a shRNA full hairpin sequence 4

<400>  59
ccgggctaca cttaaacctt ctaatctcga gattagaagg tttaagtgta gctttttg          58


<210>  60
<211>  58
<212>  DNA
<213>  Artificial Sequence

<220>
<223>  Polr2a shRNA full hairpin sequence 5

<400>  60
ccggccgctc atgaaatgct cctttctcga gaaaggagca tttcatgagc ggtttttg          58


<210>  61
<211>  21
<212>  DNA
<213>  Artificial Sequence

<220>
<223>  Rbm8a shRNA sense sequence 1

<400>  61
gcggaccttg tgtttatatt t                                                  21


<210>  62
<211>  21
<212>  DNA
<213>  Artificial Sequence

<220>
<223>  Rbm8a shRNA sense sequence 2

<400>  62
ccatgacaaa ttcgctgaat a                                                  21

<210>  63
<211>  21
<212>  DNA
<213>  Artificial Sequence

<220>
<223>  Rbm8a shRNA sense sequence 3

<400>  63
gctgccatgg aaggactaaa t                                                21


<210>  64
<211>  21
<212>  DNA
<213>  Artificial Sequence

<220>
<223>  Rbm8a shRNA sense sequence 4

<400>  64
cgctctgttg aaggttggat t                                                21


<210>  65
<211>  21
<212>  DNA
<213>  Artificial Sequence

<220>
<223>  Rbm8a shRNA sense sequence 5

<400>  65
gaaggactaa atggtcaaga t                                                21


<210>  66
<211>  58
<212>  DNA
<213>  Artificial Sequence

<220>
<223>  Rbm8a shRNA full hairpin sequence 1

<400>  66
ccgggcggac cttgtgttta tatttctcga gaaatataaa cacaaggtcc gctttttg        58


<210>  67
<211>  58
<212>  DNA
<213>  Artificial Sequence

<220>
<223>  Rbm8a shRNA full hairpin sequence 2

<400>  67
ccggccatga caaattcgct gaatactcga gtattcagcg aatttgtcat ggtttttg        58


<210>  68

<211>  58
<212>  DNA
<213>  Artificial Sequence

<220>
<223>  Rbm8a shRNA full hairpin sequence 3

<400>  68
ccgggctgcc atggaaggac taaatctcga gatttagtcc ttccatggca gctttttg          58


<210>  69
<211>  58
<212>  DNA
<213>  Artificial Sequence

<220>
<223>  Rbm8a shRNA full hairpin sequence 4

<400>  69
ccggcgctct gttgaaggtt ggattctcga gaatccaacc ttcaacagag cgtttttg          58


<210>  70
<211>  58
<212>  DNA
<213>  Artificial Sequence

<220>
<223>  Rbm8a shRNA full hairpin sequence 5

<400>  70
ccgggaagga ctaaatggtc aagatctcga gatcttgacc atttagtcct tctttttg          58


<210>  71
<211>  21
<212>  DNA
<213>  Artificial Sequence

<220>
<223>  Sf3b1 shRNA sense sequence 1

<400>  71
cctgcttcaa tctgatgata t                                                  21


<210>  72
<211>  21
<212>  DNA
<213>  Artificial Sequence

<220>
<223>  Sf3b1 shRNA sense sequence 2

<400>  72
gcccactttc tgatgaagaa t                                                  21


<210>  73
<211>  21
<212>  DNA
<213>  Artificial Sequence

<220>
<223>  Sf3b1 shRNA sense sequence 3

<400>  73
cgcagcagat attgaccata a                                                21


<210>  74
<211>  21
<212>  DNA
<213>  Artificial Sequence

<220>
<223>  Sf3b1 shRNA sense sequence 4

<400>  74
gccgagagat tatttctaat t                                                21


<210>  75
<211>  21
<212>  DNA
<213>  Artificial Sequence

<220>
<223>  Sf3b1 shRNA sense sequence 5

<400>  75
gcctgcctta atgaatgaat a                                                21


<210>  76
<211>  58
<212>  DNA
<213>  Artificial Sequence

<220>
<223>  Sf3b1 shRNA full hairpin sequence 1

<400>  76
ccggcctgct tcaatctgat gatatctcga gatatcatca gattgaagca ggtttttg        58


<210>  77
<211>  58
<212>  DNA
<213>  Artificial Sequence

<220>
<223>  Sf3b1 shRNA full hairpin sequence 2

<400>  77
ccgggcccac tttctgatga agaatctcga gattcttcat cagaaagtgg gctttttg        58


<210>  78
<211>  58
<212>  DNA
<213>  Artificial Sequence

<220>
<223>  Sf3b1 shRNA full hairpin sequence 3

<400>  78
ccggcgcagc agatattgac cataactcga gttatggtca atatctgctg cgtttttg        58


<210>  79
<211>  58
<212>  DNA
<213>  Artificial Sequence

<220>
<223>  Sf3b1 shRNA full hairpin sequence 4

<400>  79
ccgggccgag agattatttc taattctcga gaattagaaa taatctctcg gctttttg        58


<210>  80
<211>  58
<212>  DNA
<213>  Artificial Sequence

<220>
<223>  Sf3b1 shRNA full hairpin sequence 5

<400>  80
ccgggcctgc cttaatgaat gaatactcga gtattcattc attaaggcag gctttttg        58


<210>  81
<211>  21
<212>  DNA
<213>  Artificial Sequence

<220>
<223>  Sfrs11 shRNA sense sequence 1

<400>  81
gcacagtgat tccatgttat a                                                21


<210>  82
<211>  21
<212>  DNA
<213>  Artificial Sequence

<220>
<223>  Sfrs11 shRNA sense sequence 2

<400>  82
gtggtgtatc agggagacat t                                                21


<210>  83
<211>  21
<212>  DNA
<213>  Artificial Sequence

<220>
<223>  Sfrs11 shRNA sense sequence 3

<400>  83
gcagagatga aagggaacga t                                                21

<210>  84
<211>  21
<212>  DNA
<213>  Artificial Sequence

<220>
<223>  Sfrs11 shRNA sense sequence 4

<400>  84
gagttattcc tgatgagact a                                                21


<210>  85
<211>  21
<212>  DNA
<213>  Artificial Sequence

<220>
<223>  Sfrs11 shRNA sense sequence 5

<400>  85
ccttcgagag tccaaagtga a                                                21


<210>  86
<211>  58
<212>  DNA
<213>  Artificial Sequence

<220>
<223>  Sfrs11 shRNA full hairpin sequence 1

<400>  86
ccgggcacag tgattccatg ttatactcga gtataacatg gaatcactgt gctttttg        58


<210>  87
<211>  58
<212>  DNA
<213>  Artificial Sequence

<220>
<223>  Sfrs11 shRNA full hairpin sequence 2

<400>  87
ccgggtggtg tatcagggag acattctcga gaatgtctcc ctgatacacc actttttg        58


<210>  88
<211>  58
<212>  DNA
<213>  Artificial Sequence

<220>
<223>  Sfrs11 shRNA full hairpin sequence 3

<400>  88
ccgggcagag atgaaaggga acgatctcga gatcgttccc tttcatctct gctttttg        58


<210>  89

<211>  58
<212>  DNA
<213>  Artificial Sequence

<220>
<223>  Sfrs11 shRNA full hairpin sequence 4

<400>  89
ccgggagtta ttcctgatga gactactcga gtagtctcat caggaataac tctttttg          58


<210>  90
<211>  58
<212>  DNA
<213>  Artificial Sequence

<220>
<223>  Sfrs11 shRNA full hairpin sequence 5

<400>  90
ccggccttcg agagtccaaa gtgaactcga gttcactttg gactctcgaa ggtttttg          58


<210>  91
<211>  21
<212>  DNA
<213>  Artificial Sequence

<220>
<223>  Sfrs2 shRNA sense sequence 1

<400>  91
cgacaccctg aggcgcgtct t                                                  21


<210>  92
<211>  21
<212>  DNA
<213>  Artificial Sequence

<220>
<223>  Sfrs2 shRNA sense sequence 2

<400>  92
cgaagcgaga gtccaagtct a                                                  21


<210>  93
<211>  21
<212>  DNA
<213>  Artificial Sequence

<220>
<223>  Sfrs2 shRNA sense sequence 3

<400>  93
ccaagtccag atctgcccga a                                                  21


<210>  94
<211>  21
<212>  DNA
<213>  Artificial Sequence

<220>
<223>  Sfrs2 shRNA sense sequence 4

<400>  94
cgcgtcggcg acgtgtacat t                                                21


<210>  95
<211>  58
<212>  DNA
<213>  Artificial Sequence

<220>
<223>  Sfrs2 shRNA full hairpin sequence 1

<400>  95
ccggcgacac cctgaggcgc gtcttctcga gaagacgcgc ctcagggtgt cgtttttg        58


<210>  96
<211>  58
<212>  DNA
<213>  Artificial Sequence

<220>
<223>  Sfrs2 shRNA full hairpin sequence 2

<400>  96
ccggcgaagc gagagtccaa gtctactcga gtagacttgg actctcgctt cgtttttg        58


<210>  97
<211>  58
<212>  DNA
<213>  Artificial Sequence

<220>
<223>  Sfrs2 shRNA full hairpin sequence 3

<400>  97
ccggccaagt ccagatctgc ccgaactcga gttcgggcag atctggactt ggtttttg        58


<210>  98
<211>  58
<212>  DNA
<213>  Artificial Sequence

<220>
<223>  Sfrs2 shRNA full hairpin sequence 4

<400>  98
ccggcgcgtc ggcgacgtgt acattctcga gaatgtacac gtcgccgacg cgtttttg        58


<210>  99
<211>  21
<212>  DNA
<213>  Artificial Sequence

<220>
<223>  Sfrs6 shRNA sense sequence 1

<400>  99
cgcctcttct ttaagaggat t                                                21


<210>  100
<211>  21
<212>  DNA
<213>  Artificial Sequence

<220>
<223>  Sfrs6 shRNA sense sequence 2

<400>  100
gctcacaaag aacgaacaaa t                                                21


<210>  101
<211>  21
<212>  DNA
<213>  Artificial Sequence

<220>
<223>  Sfrs6 shRNA sense sequence 3

<400>  101
ccagatcaag ttccagagat t                                                21


<210>  102
<211>  21
<212>  DNA
<213>  Artificial Sequence

<220>
<223>  Sfrs6 shRNA sense sequence 4

<400>  102
cgtacagagt acaggcttat t                                                21


<210>  103
<211>  21
<212>  DNA
<213>  Artificial Sequence

<220>
<223>  Sfrs6 shRNA sense sequence 5

<400>  103
cgaacaaatg agggtgtgat t                                                21


<210>  104
<211>  58
<212>  DNA
<213>  Artificial Sequence

<220>
<223>  Sfrs6 shRNA full hairpin sequence 1

<400>  104
ccggcgcctc ttctttaaga ggattctcga gaatcctctt aaagaagagg cgtttttg        58

<210>  105
<211>  58
<212>  DNA
<213>  Artificial Sequence

<220>
<223>  Sfrs6 shRNA full hairpin sequence 2

<400>  105
ccgggctcac aaagaacgaa caaatctcga gatttgttcg ttctttgtga gctttttg          58


<210>  106
<211>  58
<212>  DNA
<213>  Artificial Sequence

<220>
<223>  Sfrs6 shRNA full hairpin sequence 3

<400>  106
ccggccagat caagttccag agattctcga gaatctctgg aacttgatct ggtttttg          58


<210>  107
<211>  58
<212>  DNA
<213>  Artificial Sequence

<220>
<223>  Sfrs6 shRNA full hairpin sequence 4

<400>  107
ccggcgtaca gagtacaggc ttattctcga gaataagcct gtactctgta cgtttttg          58


<210>  108
<211>  58
<212>  DNA
<213>  Artificial Sequence

<220>
<223>  Sfrs6 shRNA full hairpin sequence 5

<400>  108
ccggcgaaca aatgagggtg tgattctcga gaatcacacc ctcatttgtt cgtttttg          58


<210>  109
<211>  21
<212>  DNA
<213>  Artificial Sequence

<220>
<223>  Sfrs9 shRNA sense sequence 1

<400>  109
cgaggacttg ttctacaagt a                                                  21


<210>  110

<211>  21
<212>  DNA
<213>  Artificial Sequence

<220>
<223>  Sfrs9 shRNA sense sequence 2

<400>  110
gcaggacctg aaagatcaca t                                                21


<210>  111
<211>  21
<212>  DNA
<213>  Artificial Sequence

<220>
<223>  Sfrs9 shRNA sense sequence 3

<400>  111
cgtaaactgg atgacaccaa a                                                21


<210>  112
<211>  21
<212>  DNA
<213>  Artificial Sequence

<220>
<223>  Sfrs9 shRNA sense sequence 4

<400>  112
tggaagaaac ggttacgatt a                                                21


<210>  113
<211>  58
<212>  DNA
<213>  Artificial Sequence

<220>
<223>  Sfrs9 shRNA full hairpin sequence 1

<400>  113
ccggcgagga cttgttctac aagtactcga gtacttgtag aacaagtcct cgtttttg        58


<210>  114
<211>  58
<212>  DNA
<213>  Artificial Sequence

<220>
<223>  Sfrs9 shRNA full hairpin sequence 2

<400>  114
ccgggcagga cctgaaagat cacatctcga gatgtgatct ttcaggtcct gctttttg        58


<210>  115
<211>  58
<212>  DNA
<213>  Artificial Sequence

<220>
<223>  Sfrs9 shRNA full hairpin sequence 3

<400>  115
ccggcgtaaa ctggatgaca ccaaactcga gtttggtgtc atccagttta cgtttttg        58


<210>  116
<211>  58
<212>  DNA
<213>  Artificial Sequence

<220>
<223>  Sfrs9 shRNA full hairpin sequence 4

<400>  116
ccggtggaag aaacggttac gattactcga gtaatcgtaa ccgtttcttc catttttg        58


<210>  117
<211>  21
<212>  DNA
<213>  Artificial Sequence

<220>
<223>  Snrnp200 shRNA sense sequence 1

<400>  117
catccagtta ctgtcccact t                                                21


<210>  118
<211>  21
<212>  DNA
<213>  Artificial Sequence

<220>
<223>  Snrnp200 shRNA sense sequence 2

<400>  118
gctgactatc acaccagatt t                                                21


<210>  119
<211>  21
<212>  DNA
<213>  Artificial Sequence

<220>
<223>  Snrnp200 shRNA sense sequence 3

<400>  119
cccaagttca atgatccaca t                                                21


<210>  120
<211>  21
<212>  DNA
<213>  Artificial Sequence

<220>
<223>  Snrnp200 shRNA sense sequence 4

<400>  120
gccaaggtga agctagactt t                                                21


<210>  121
<211>  58
<212>  DNA
<213>  Artificial Sequence

<220>
<223>  Snrnp200 shRNA full hairpin sequence 1

<400>  121
ccggcatcca gttactgtcc cacttctcga gaagtgggac agtaactgga tgtttttg        58


<210>  122
<211>  58
<212>  DNA
<213>  Artificial Sequence

<220>
<223>  Snrnp200 shRNA full hairpin sequence 2

<400>  122
ccgggctgac tatcacacca gatttctcga gaaatctggt gtgatagtca gctttttg        58


<210>  123
<211>  58
<212>  DNA
<213>  Artificial Sequence

<220>
<223>  Snrnp200 shRNA full hairpin sequence 3

<400>  123
ccggcccaag ttcaatgatc cacatctcga gatgtggatc attgaacttg ggtttttg        58


<210>  124
<211>  58
<212>  DNA
<213>  Artificial Sequence

<220>
<223>  Snrnp200 shRNA full hairpin sequence 4

<400>  124
ccgggccaag gtgaagctag actttctcga gaaagtctag cttcaccttg gctttttg        58


<210>  125
<211>  21
<212>  DNA
<213>  Artificial Sequence

<220>
<223>  Srrm1 shRNA sense sequence 1

<400>  125
gctgaagatg acaaccttga t                                                21

<210>  126
<211>  21
<212>  DNA
<213>  Artificial Sequence

<220>
<223>  Srrm1 shRNA sense sequence 2

<400>  126
gccagaatca agagtacaaa t                                                21


<210>  127
<211>  21
<212>  DNA
<213>  Artificial Sequence

<220>
<223>  Srrm1 shRNA sense sequence 3

<400>  127
gcccaagaga tcccatgtaa a                                                21


<210>  128
<211>  21
<212>  DNA
<213>  Artificial Sequence

<220>
<223>  Srrm1 shRNA sense sequence 4

<400>  128
cgctcaccac aaccaaacaa a                                                21


<210>  129
<211>  21
<212>  DNA
<213>  Artificial Sequence

<220>
<223>  Srrm1 shRNA sense sequence 5

<400>  129
cacctctaca agtgacatct t                                                21


<210>  130
<211>  21
<212>  DNA
<213>  Artificial Sequence

<220>
<223>  Srrm1 shRNA sense sequence 6

<400>  130
ccacctctac aagtgacatc t                                                21


<210>  131

<211>  58
<212>  DNA
<213>  Artificial Sequence

<220>
<223>  Srrm1 shRNA full hairpin sequence 1

<400>  131
ccgggctgaa gatgacaacc ttgatctcga gatcaaggtt gtcatcttca gctttttg          58


<210>  132
<211>  58
<212>  DNA
<213>  Artificial Sequence

<220>
<223>  Srrm1 shRNA full hairpin sequence 2

<400>  132
ccgggccaga atcaagagta caaatctcga gatttgtact cttgattctg gctttttg          58


<210>  133
<211>  58
<212>  DNA
<213>  Artificial Sequence

<220>
<223>  Srrm1 shRNA full hairpin sequence 3

<400>  133
ccgggcccaa gagatcccat gtaaactcga gtttacatgg gatctcttgg gctttttg          58


<210>  134
<211>  58
<212>  DNA
<213>  Artificial Sequence

<220>
<223>  Srrm1 shRNA full hairpin sequence 4

<400>  134
ccggcgctca ccacaaccaa acaaactcga gtttgtttgg ttgtggtgag cgtttttg          58


<210>  135
<211>  58
<212>  DNA
<213>  Artificial Sequence

<220>
<223>  Srrm1 shRNA full hairpin sequence 5

<400>  135
ccggcacctc tacaagtgac atcttctcga gaagatgtca cttgtagagg tgtttttg          58


<210>  136
<211>  58
<212>  DNA
<213>  Artificial Sequence

<220>
<223>  Srrm1 shRNA full hairpin sequence 6

<400>  136
ccggccacct ctacaagtga catctctcga gagatgtcac ttgtagaggt ggtttttg        58


<210>  137
<211>  21
<212>  DNA
<213>  Artificial Sequence

<220>
<223>  mouse Ybx1 shRNA sense sequence 1

<400>  137
gagaacccta aaccacaaga t                                                 21


<210>  138
<211>  21
<212>  DNA
<213>  Artificial Sequence

<220>
<223>  mouse Ybx1 shRNA sense sequence 2

<400>  138
gcgaaggttc ccaccttact a                                                 21


<210>  139
<211>  21
<212>  DNA
<213>  Artificial Sequence

<220>
<223>  mouse Ybx1 shRNA sense sequence 3

<400>  139
catctctacc atcatccggt t                                                 21


<210>  140
<211>  21
<212>  DNA
<213>  Artificial Sequence

<220>
<223>  mouse Ybx1 shRNA sense sequence 4

<400>  140
gtatcgccga aacttcaatt a                                                 21


<210>  141
<211>  58
<212>  DNA
<213>  Artificial Sequence

<220>
<223>  mouse Ybx1 shRNA full hairpin sequence 1

<400>  141
ccgggagaac cctaaaccac aagatctcga gatcttgtgg tttagggttc tctttttg        58


<210>  142
<211>  58
<212>  DNA
<213>  Artificial Sequence

<220>
<223>  mouse Ybx1 shRNA full hairpin sequence 2

<400>  142
ccgggcgaag gttcccacct tactactcga gtagtaaggt gggaaccttc gctttttg        58


<210>  143
<211>  58
<212>  DNA
<213>  Artificial Sequence

<220>
<223>  mouse Ybx1 shRNA full hairpin sequence 3

<400>  143
ccggcatctc taccatcatc cggttctcga gaaccggatg atggtagaga tgtttttg        58


<210>  144
<211>  58
<212>  DNA
<213>  Artificial Sequence

<220>
<223>  mouse Ybx1 shRNA full hairpin sequence 4

<400>  144
ccgggtatcg ccgaaacttc aattactcga gtaattgaag tttcggcgat actttttg        58


<210>  145
<211>  24
<212>  DNA
<213>  Artificial Sequence

<220>
<223>  Ybx1_cond_for primer

<400>  145
gcctaaggat agtgaagttt ctgg                                              24


<210>  146
<211>  24
<212>  DNA
<213>  Artificial Sequence

<220>
<223>  Ybx1_con_rev primer

<400>  146
cctagcacac cttaatctac agcc                                              24

<210>  147
<211>  20
<212>  DNA
<213>  Artificial Sequence

<220>
<223>  Cre_for primer

<400>  147
cgtatagccg aaattgccag                                                    20


<210>  148
<211>  20
<212>  DNA
<213>  Artificial Sequence

<220>
<223>  Cre_rev primer

<400>  148
caaaacaggt agttattcgg                                                    20


<210>  149
<211>  22
<212>  DNA
<213>  Artificial Sequence

<220>
<223>  pyrosequencing for primer

<400>  149
gaagcagcaa gtatgatgag ca                                                 22


<210>  150
<211>  22
<212>  DNA
<213>  Artificial Sequence

<220>
<223>  pyrosequencing rev primer

<400>  150
tgctctgaga aaggcattag aa                                                 22


<210>  151
<211>  15
<212>  DNA
<213>  Artificial Sequence

<220>
<223>  sequencing primer

<400>  151
tctcgtctcc acaga                                                         15


<210>  152

<211>  983
<212>  DNA
<213>  Artificial Sequence

<220>
<223>  phosphomutant mYbx1-S30A/S34A

<400>  152
gcggccgcat gagcagcgag gccgagaccc agcagccgcc cgccgccccc gccgccgccc      60

tcagcgccgc cgacaccaag cccggctcca cgggcgccgg cgcgggtgct ggcggcccgg     120

gtggcctcac atcggcggcg cccgccggcg gggacaagaa ggtcatcgca acgaaggttt     180

tgggaacagt caaatggttc aatgtaagga acggatacgg tttcatcaac aggaatgaca     240

ccaaggaaga cgtatttgta caccagactg ccataaagaa gaataacccc aggaagtacc     300

ttcgcagtgt aggcgatgga gagactgtgg agtttgatgt tgttgaagga gaaaagggtg     360

cggaggcagc aaatgttaca ggccctggtg gagttccagt tcaaggcagt aaatacgcag     420

cagaccgtaa ccattataga cgctatccac gtcgtagggg tcctccacgc aattaccagc     480

aaaattacca gaatagtgag agtggggaaa agaacgaggg atcggaaagc gctcctgaag     540

gccaggccca acaacgccgg ccctatcgca ggcgaaggtt cccaccttac tacatgcgga     600

gaccctatgc gcgtcgacca cagtattcca acccccctgt gcaaggagaa gtgatggagg     660

gtgctgacaa ccagggtgca ggagagcaag gtagaccagt gagacagaat atgtatcggg     720

gttacagacc acgattccga aggggccctc ctcgccaaag acagcctaga gaggatggca     780

atgaagagga caaagaaaat caaggagatg agacccaagg tcagcagcca cctcaacgtc     840

ggtatcgccg aaacttcaat taccgacgca gacgcccaga gaaccctaaa ccacaagatg     900

gcaaagagac aaaagcagcc gatccaccag ctgagaattc gtccgctccc gaggctgagc     960

agggcggggc tgagtaatga tca                                             983

<210>  153
<211>  983
<212>  DNA
<213>  Artificial Sequence

<220>
<223>  mYbx1-S30A

<400>  153
gcggccgcat gagcagcgag gccgagaccc agcagccgcc cgccgccccc gccgccgccc      60

tcagcgccgc cgacaccaag cccggctcca cgggcgccgg cgcgggtagt ggcggcccgg     120

gtggcctcac atcggcggcg cccgccggcg gggacaagaa ggtcatcgca acgaaggttt     180

tgggaacagt caaatggttc aatgtaagga acggatacgg tttcatcaac aggaatgaca     240

ccaaggaaga cgtatttgta caccagactg ccataaagaa gaataacccc aggaagtacc     300

ttcgcagtgt aggcgatgga gagactgtgg agtttgatgt tgttgaagga gaaaagggtg     360

cggaggcagc aaatgttaca ggccctggtg gagttccagt tcaaggcagt aaatacgcag 420

cagaccgtaa ccattataga cgctatccac gtcgtagggg tcctccacgc aattaccagc 480

aaaattacca gaatagtgag agtggggaaa agaacgaggg atcggaaagc gctcctgaag 540

gccaggccca acaacgccgg ccctatcgca ggcgaaggtt cccaccttac tacatgcgga 600

gaccctatgc gcgtcgacca cagtattcca acccccctgt gcaaggagaa gtgatggagg 660

gtgctgacaa ccagggtgca ggagagcaag gtagaccagt gagacagaat atgtatcggg 720

gttacagacc acgattccga aggggccctc ctcgccaaag acagcctaga gaggatggca 780

atgaagagga caaagaaaat caaggagatg agacccaagg tcagcagcca cctcaacgtc 840

ggtatcgccg aaacttcaat taccgacgca gacgcccaga gaaccctaaa ccacaagatg 900

gcaaagagac aaaagcagcc gatccaccag ctgagaattc gtccgctccc gaggctgagc 960

agggcggggc tgagtaatga tca 983

<210> 154
<211> 983
<212> DNA
<213> Artificial Sequence

<220>
<223> mYbx1-S34A

<400> 154
gcggccgcat gagcagcgag gccgagaccc agcagccgcc cgccgccccc gccgccgccc 60

tcagcgccgc cgacaccaag cccggctcca cgggcagcgg cgcgggtgct ggcggcccgg 120

gtggcctcac atcggcggcg cccgccggcg gggacaagaa ggtcatcgca acgaaggttt 180

tgggaacagt caaatggttc aatgtaagga acggatacgg tttcatcaac aggaatgaca 240

ccaaggaaga cgtatttgta caccagactg ccataaagaa gaataacccc aggaagtacc 300

ttcgcagtgt aggcgatgga gagactgtgg agtttgatgt tgttgaagga gaaaagggtg 360

cggaggcagc aaatgttaca ggccctggtg gagttccagt tcaaggcagt aaatacgcag 420

cagaccgtaa ccattataga cgctatccac gtcgtagggg tcctccacgc aattaccagc 480

aaaattacca gaatagtgag agtggggaaa agaacgaggg atcggaaagc gctcctgaag 540

gccaggccca acaacgccgg ccctatcgca ggcgaaggtt cccaccttac tacatgcgga 600

gaccctatgc gcgtcgacca cagtattcca acccccctgt gcaaggagaa gtgatggagg 660

gtgctgacaa ccagggtgca ggagagcaag gtagaccagt gagacagaat atgtatcggg 720

gttacagacc acgattccga aggggccctc ctcgccaaag acagcctaga gaggatggca 780

atgaagagga caaagaaaat caaggagatg agacccaagg tcagcagcca cctcaacgtc 840

ggtatcgccg aaacttcaat taccgacgca gacgcccaga gaaccctaaa ccacaagatg 900

gcaaagagac aaaagcagcc gatccaccag ctgagaattc gtccgctccc gaggctgagc 960

agggcggggc tgagtaatga tca 983

<210> 155
<211> 983
<212> DNA
<213> Artificial Sequence

<220>
<223> mYbx1-S30D/S34D

<400> 155
gcggccgcat gagcagcgag gccgagaccc agcagccgcc cgccgccccc gccgccgccc 60

tcagcgccgc cgacaccaag cccggctcca cgggcgacgg cgcgggtgat ggcggcccgg 120

gtggcctcac atcggcggcg cccgccggcg gggacaagaa ggtcatcgca acgaaggttt 180

tgggaacagt caaatggttc aatgtaagga acggatacgg tttcatcaac aggaatgaca 240

ccaaggaaga cgtatttgta caccagactg ccataaagaa gaataacccc aggaagtacc 300

ttcgcagtgt aggcgatgga gagactgtgg agtttgatgt tgttgaagga gaaaagggtg 360

cggaggcagc aaatgttaca ggccctggtg gagttccagt tcaaggcagt aaatacgcag 420

cagaccgtaa ccattataga cgctatccac gtcgtagggg tcctccacgc aattaccagc 480

aaaattacca gaatagtgag agtggggaaa agaacgaggg atcggaaagc gctcctgaag 540

gccaggccca acaacgccgg ccctatcgca ggcgaaggtt cccaccttac tacatgcgga 600

gaccctatgc gcgtcgacca cagtattcca acccccctgt gcaaggagaa gtgatggagg 660

gtgctgacaa ccagggtgca ggagagcaag gtagaccagt gagacagaat atgtatcggg 720

gttacagacc acgattccga aggggccctc ctcgccaaag acagcctaga gaggatggca 780

atgaagagga caaagaaaat caaggagatg agacccaagg tcagcagcca cctcaacgtc 840

ggtatcgccg aaacttcaat taccgacgca gacgcccaga gaaccctaaa ccacaagatg 900

gcaaagagac aaaagcagcc gatccaccag ctgagaattc gtccgctccc gaggctgagc 960

agggcggggc tgagtaatga tca 983

<210> 156
<211> 983
<212> DNA
<213> Artificial Sequence

<220>
<223> mYbx1-S30D

<400> 156
gcggccgcat gagcagcgag gccgagaccc agcagccgcc cgccgccccc gccgccgccc 60

tcagcgccgc cgacaccaag cccggctcca cgggcgacgg cgcgggtagt ggcggcccgg 120

gtggcctcac atcggcggcg cccgccggcg gggacaagaa ggtcatcgca acgaaggttt 180

tgggaacagt caaatggttc aatgtaagga acggatacgg tttcatcaac aggaatgaca 240

ccaaggaaga cgtatttgta caccagactg ccataaagaa gaataacccc aggaagtacc 300

ttcgcagtgt aggcgatgga gagactgtgg agtttgatgt tgttgaagga gaaaagggtg 360

cggaggcagc aaatgttaca ggccctggtg gagttccagt tcaaggcagt aaatacgcag 420

cagaccgtaa ccattataga cgctatccac gtcgtagggg tcctccacgc aattaccagc 480

aaaattacca gaatagtgag agtggggaaa agaacgaggg atcggaaagc gctcctgaag 540

gccaggccca acaacgccgg ccctatcgca ggcgaaggtt cccaccttac tacatgcgga 600

gaccctatgc gcgtcgacca cagtattcca acccccctgt gcaaggagaa gtgatggagg 660

gtgctgacaa ccagggtgca ggagagcaag gtagaccagt gagacagaat atgtatcggg 720

gttacagacc acgattccga aggggccctc ctcgccaaag acagcctaga gaggatggca 780

atgaagagga caaagaaaat caaggagatg agacccaagg tcagcagcca cctcaacgtc 840

ggtatcgccg aaacttcaat taccgacgca gacgcccaga gaaccctaaa ccacaagatg 900

gcaaagagac aaaagcagcc gatccaccag ctgagaattc gtccgctccc gaggctgagc 960

agggcggggc tgagtaatga tca 983

<210> 157
<211> 983
<212> DNA
<213> Artificial Sequence

<220>
<223> mYbx1-S34D

<400> 157
gcggccgcat gagcagcgag gccgagaccc agcagccgcc cgccgccccc gccgccgccc 60

tcagcgccgc cgacaccaag cccggctcca cgggcagcgg cgcgggtgat ggcggcccgg 120

gtggcctcac atcggcggcg cccgccggcg gggacaagaa ggtcatcgca acgaaggttt 180

tgggaacagt caaatggttc aatgtaagga acggatacgg tttcatcaac aggaatgaca 240

ccaaggaaga cgtatttgta caccagactg ccataaagaa gaataacccc aggaagtacc 300

ttcgcagtgt aggcgatgga gagactgtgg agtttgatgt tgttgaagga gaaaagggtg 360

cggaggcagc aaatgttaca ggccctggtg gagttccagt tcaaggcagt aaatacgcag 420

cagaccgtaa ccattataga cgctatccac gtcgtagggg tcctccacgc aattaccagc 480

aaaattacca gaatagtgag agtggggaaa agaacgaggg atcggaaagc gctcctgaag 540

gccaggccca acaacgccgg ccctatcgca ggcgaaggtt cccaccttac tacatgcgga 600

gaccctatgc gcgtcgacca cagtattcca acccccctgt gcaaggagaa gtgatggagg 660

gtgctgacaa ccagggtgca ggagagcaag gtagaccagt gagacagaat atgtatcggg 720

gttacagacc acgattccga aggggccctc ctcgccaaag acagcctaga gaggatggca 780

atgaagagga caaagaaaat caaggagatg agacccaagg tcagcagcca cctcaacgtc 840

ggtatcgccg aaacttcaat taccgacgca gacgcccaga gaaccctaaa ccacaagatg 900

gcaaagagac aaaagcagcc gatccaccag ctgagaattc gtccgctccc gaggctgagc 960

agggcggggc tgagtaatga tca 983

<210> 158
<211> 983
<212> DNA
<213> Artificial Sequence

<220>
<223> mYbx1-S172A/S174A

<400> 158
gcggccgcat gagcagcgag gccgagaccc agcagccgcc cgccgccccc gccgccgccc 60

tcagcgccgc cgacaccaag cccggctcca cgggcagcgg cgcgggtagt ggcggcccgg 120

gtggcctcac atcggcggcg cccgccggcg gggacaagaa ggtcatcgca acgaaggttt 180

tgggaacagt caaatggttc aatgtaagga acggatacgg tttcatcaac aggaatgaca 240

ccaaggaaga cgtatttgta caccagactg ccataaagaa gaataacccc aggaagtacc 300

ttcgcagtgt aggcgatgga gagactgtgg agtttgatgt tgttgaagga gaaaagggtg 360

cggaggcagc aaatgttaca ggccctggtg gagttccagt tcaaggcagt aaatacgcag 420

cagaccgtaa ccattataga cgctatccac gtcgtagggg tcctccacgc aattaccagc 480

aaaattacca gaatagtgag agtggggaaa agaacgaggg agcggaagcc gctcctgaag 540

gccaggccca acaacgccgg ccctatcgca ggcgaaggtt cccaccttac tacatgcgga 600

gaccctatgc gcgtcgacca cagtattcca acccccctgt gcaaggagaa gtgatggagg 660

gtgctgacaa ccagggtgca ggagagcaag gtagaccagt gagacagaat atgtatcggg 720

gttacagacc acgattccga aggggccctc ctcgccaaag acagcctaga gaggatggca 780

atgaagagga caaagaaaat caaggagatg agacccaagg tcagcagcca cctcaacgtc 840

ggtatcgccg aaacttcaat taccgacgca gacgcccaga gaaccctaaa ccacaagatg 900

gcaaagagac aaaagcagcc gatccaccag ctgagaattc gtccgctccc gaggctgagc 960

agggcggggc tgagtaatga tca 983

<210> 159
<211> 58
<212> DNA
<213> Artificial Sequence

<220>
<223> human Ybx1 shRNA full hairpin sequence 1

<400>  159
ccggccagtt caaggcagta aatatctcga gatatttact gccttgaact ggtttttg        58

<210>  160
<211>  58
<212>  DNA
<213>  Artificial Sequence

<220>
<223>  human Ybx1 shRNA full hairpin sequence 2

<400>  160
ccggagcaga ccgtaaccat tatagctcga gctataatgg ttacggtctg ctttttg        58

<210>  161
<211>  57
<212>  DNA
<213>  Artificial Sequence

<220>
<223>  human Ybx1 shRNA full hairpin sequence 3

<400>  161
ccgggacggc aatgaagaag ataaactcga gtttatcttc ttcattgccg tcttttt        57

<210>  162
<211>  57
<212>  DNA
<213>  Artificial Sequence

<220>
<223>  human Ybx1 shRNA full hairpin sequence 4

<400>  162
ccggcgtaac cattatagac gctatctcga gatagcgtct ataatggtta cgttttt        57